# EUROPEAN PATENT SPECIFICATION

(11) **EP 2 732 029 B1**
(45) Date of publication and mention of the grant of the patent: **16.01.2019**
(21) Application number: 12736042.8
(22) Date of filing: 11.07.2012
(51) Int. Cl.: C12N 5/074, C12N 15/90

(54) **METHODS FOR CELL REPROGRAMMING AND GENOME ENGINEERING**
VERFAHREN ZUR ZELLPROGRAMMIERUNG UND GENOMMANIPULATION
PROCÉDÉS DE REPROGRAMMATION CELLULAIRE ET D'INGÉNIERIE GÉNOMIQUE

(30) Priority: 11.07.2011 US 201161506314 P
(43) Date of publication of application: 21.05.2014
(73) Proprietor: FUJIFILM Cellular Dynamics, Inc., Madison, WI 53711 (US)
(72) Inventor: BURKE, Thomas, Madison, WI 53711 (US); MILLER, Michael, Madison, WI 53711 (US); MCLACHLAN, Michael, Madison, WI 53711 (US); DICKERSON, Sarah, Madison, WI 53711 (US); STROUSE, Anne, Madison, WI 53711 (US)
(74) Representative: Boult Wade Tennant LLP
(86) International application number: PCT/US2012/046194
(87) International publication number: WO 2013/009825

(56) References cited:
- WO-A1-2010/042490
- WOLTJEN KNUT ET AL: "piggyBac transposition reprograms fibroblasts to induced pluripotent stem cells", NATURE: INTERNATIONAL WEEKLY JOURNAL OF SCIENCE, NATURE PUBLISHING GROUP, UNITED KINGDOM, vol. 458, no. 7239, 9 April 2009 (2009-04-09), pages 766-770, XP009139776, ISSN: 0028-0836
- AKITSU HOTTA ET AL: "Isolation of human iPS cells using EOS lentiviral vectors to select for pluripotency", NATURE METHODS, vol. 6, no. 5, 1 May 2009 (2009-05-01), pages 370-376, XP55040636, ISSN: 1548-7091, DOI: 10.1038/nmeth.1325
- OKITA KEISUKE ET AL: "Generation of Mouse Induced Pluripotent Stem Cells Without Viral Vectors", SCIENCE, AMERICAN ASSOCIATION FOR THE ADVANCEMENT OF SCIENCE, WASHINGTON, DC; US, vol. 322, no. 5903, 7 November 2008 (2008-11-07), pages 949-953, XP002531346, ISSN: 0036-8075, DOI: 10.1126/SCIENCE.1164270
- STADTFELD MATTHIAS ET AL: "Induced Pluripotent Stem Cells Generated Without Viral Integration", SCIENCE, AMERICAN ASSOCIATION FOR THE ADVANCEMENT OF SCIENCE, WASHINGTON, DC; US, vol. 322, no. 5903, 7 November 2008 (2008-11-07), pages 945-949, XP002531345, ISSN: 0036-8075, DOI: 10.1126/SCIENCE.1162494
- J. ZOU ET AL: "Oxidase-deficient neutrophils from X-linked chronic granulomatous disease iPS cells: functional correction by zinc finger nuclease-mediated safe harbor targeting", BLOOD, vol. 117, no. 21, 26 May 2011 (2011-05-26) , pages 5561-5572, XP55040721, ISSN: 0006-4971, DOI: 10.1182/blood-2010-12-328161
- YU JUNYING ET AL: "Human induced pluripotent stem cells free of vector and transgene sequences", SCIENCE, AMERICAN ASSOCIATION FOR THE ADVANCEMENT OF SCIENCE, US, vol. 324, no. 5928, 8 May 2009 (2009-05-08), pages 797-801, XP002596377, ISSN: 1095-9203, DOI: 10.1126/SCIENCE.1172482 [retrieved on 2009-03-26]
- HOCKEMEYER DIRK ET AL: "Efficient targeting of expressed and silent genes in human ESCs and iPSCs using zinc-finger nucleases", NATURE BIOTECHNOLOGY, NATURE PUBLISHING GROUP, NEW YORK, NY, US, vol. 27, no. 9, 1 September 2009 (2009-09-01), pages 851-857, XP009143038, ISSN: 1087-0156, DOI: 10.1038/NBT.1562

## Description

### BACKGROUND OF THE INVENTION

### 1. Field of the Invention

The present invention relates generally to the field of stem cell development. More particularly, it concerns the generation of engineered pluripotent stem cells.

### 2. Description of Related Art

The unlimited proliferation capability and pluripotent potential of human embryonic stem (ES) cells have offered unprecedented access to all cell types of the human body. Human induced pluripotent stem (iPS) cells derived directly from patient somatic cells with desired genetic background share these two key properties of human ES cells, which made these cells excellent candidates for disease models, drug screening, toxicity testing and transplantation therapies. However, genetic reprogramming of human somatic cells to induced pluripotent stem cells (iPSCs) remains a time consuming, expensive and relatively inefficient process. Moreover, even when desired iPSCs have been produced many applications require further genetic modification of the cells in addition to extensive analysis and characterization of cell properties.

Therefore, there remains a need to address the inefficiency, high cost and other problems in preparing genetically engineered induced pluripotent stem cells.

WO2010/042490 describes tail tip fibroblasts obtained from sox2-GFP rosa26-M2rtTA mice transfected with a lox P multicistronic vector expressing different reprogramming factors and cultured to iPS cells to obtain transgene free iPS cells.

Knut et al. (Nature, 2009, vol 458; 7239; 766-770) describe the use of the piggypac transposition system to generate iPS cells. Once the iPS cells are generated the transgenes are removed through the use of a transposase.

In Hotta et al. (Nature Methods, 2009, vol 6; 5; 370-376), MEFs are transfected with EOS lentiviral vectors and then reprogrammed by infection with MoMLV based retroviral vectors expressing the four reprogramming factors.

In Keisuke et al. (Science, 2008, vol 322; 5903; 949-953), hepatocytes from mice expressing a reporter gene under the control of the nanog promoter are transfected with plasmid vectors expressing the different reprogramming factors.

Matthias et al. (Science, 2008, vol 322, 5903, 945-949) discloses the generation of iPS cells by employing a combination of adenoviruses expressing sox2, klf4 and c-myc.

Zou et al. (Blood, 2011, vol 117; 21; 5561-5572) discloses iPS cells generated from a patient with X linked chronic granulomatous disease and transfected with a therapeutic minigene using a zinc-finger nucleated gene targeting method.

### SUMMARY OF THE INVENTION

The present invention provides an *in vitro* method for producing a population of induced pluripotent stem (iPS) cells, comprising: a) introducing into somatic cells a first nucleic acid molecule for integration into the genome of the cells and at least a second nucleic acid molecule comprising an extra-chromosomal genetic element that expresses one or more reprogramming factors sufficient when expressed in the somatic cell to convert the somatic cell to a pluripotent stem cell; b) culturing said cells under reprogramming conditions; and c) obtaining a population of iPS cells comprising said first nucleic acid integrated in their genome, wherein said first nucleic acid is expressible and wherein the second nucleic acid molecule is not present in the iPS cells.

The present invention overcomes a major deficiency in the art by providing efficient methods for producing genetically engineered induced pluripotent stem (iPS) cells. In a first embodiment, a method for producing a population of engineered iPS cells is provided comprising (a) obtaining somatic cells; (b) introducing into said cells a first nucleic acid molecule for integration into the genome of the cells and at least a second nucleic acid molecule comprising a genetic element that expresses one or more reprogramming factors; (c) culturing said cells under reprogramming conditions; and (d) obtaining a population of iPS cells comprising said first nucleic acid integrated in their genome. Thus, as used here, the one or more reprogramming factor(s) are sufficient, when expressed in the somatic cell under appropriate cell culture conditions, to convert the somatic cell to a pluripotent stem cell. In a further aspect of the embodiment the method comprises producing at least a second population of iPS cells that do not comprise the first nucleic acid integrated in their genome. In certain aspects, the iPS cells and/or engineered iPS cells obtained by a method of the embodiment do not express reprogramming factors (*i.e.,* the factor(s) encoded by the genetic element). In still further aspects, the iPS cells (and/or engineered iPS cells) obtained by such a method do not comprise the genetic element of the second nucleic acid molecule integrated into their genome. Thus, in accordance with the present claims, the genetic element that expresses one or more reprogramming factor(s) is an extra-chromosomal genetic element.

Provided is a an in vitro method for producing a population of engineered iPS cells comprising (a) obtaining somatic cells; (b) introducing into the cells a first nucleic acid molecule for integration into the genome of the cells; (c) introducing into said cells an extra-chromosomal genetic element that expresses one or more reprogramming factor(s); and (d) culturing said cells under reprogramming conditions to produce a population of iPS cells comprising said first nucleic acid integrated in their genome. The iPS cells and/or engineered iPS cells produced by a method of the embodiment do not comprise the extra-chromosomal genetic element integrated into their genome. In a further embodiment there is provided a method for producing a population of induced pluripotent stem (iPS) cells and a population of engineered iPS cells, comprising (a) obtaining somatic cells; (b) introducing into said cells a first nucleic acid molecule for integration into the genome of the cells; (c) introducing into said cells an extra-chromosomal genetic element that expresses one or more reprogramming factor(s); and (d) culturing said cells under reprogramming conditions to produce a population of iPS cells and recovering both first iPS cells which comprise said first nucleic acid integrated in their genome and second iPS cells which do not comprise the first nucleic acid integrated in their genome, neither the first nor second iPS cells having the extra-chromosomal genetic element integrated into their genomes.

In a further aspect, iPS cells produced by a method of the embodiments comprise a first nucleic acid molecule integrated into their genome wherein the first nucleic acid is expressible. For example, the first nucleic acid can comprise at least one genetic element (*e.g.,* an RNA or polypeptide coding sequence) that can be expressed in the iPS cells or in cells differentiated from the iPS cells under appropriate conditions. In some aspects, the first nucleic acid molecule includes a genetic element under the control of an inducible or tissue or cell specific promoter. Accordingly, the genetic element would be expressible under conditions wherein the promoter is active (*e.g.,* in the presence of an inducing agent or in a particular differentiated cell type). In still a further aspect, a method of the embodiments comprises a step for screening or selecting for the presence of an expressible first nucleic acid molecule in the iPS cells.

The first nucleic acid molecule and the second nucleic acid comprising the genetic element (that expresses one or more reprogramming factors) may be introduced into the somatic cells no more than about one week apart, such as within 2, 3, 4, 5 or 6 days of each other. The first and second nucleic acid molecules may be introduced into the cells during the same day, such as within 2, 3, 4, 5, or 6 hours of each other. The first and second nucleic acid molecules may be introduced into the cells essentially concomitantly. For example, described herein is a method further defined as forming a composition comprising the somatic cells, the first nucleic acid molecule and the second nucleic acid molecule (comprising the genetic element) and culturing said composition.

Certain aspects of the disclosure concern a first nucleic acid molecule for integration into the genome of the cells. Such a nucleic acid molecule may integrate into the cell genome at a selected genomic site or in a specific region or may integrate into the genome essentially randomly. In some cases, the first nucleic acid integrates into the genome in only one copy, at one site in the genome. In other cases, 2, 3, 4, 5, 6, 7, 8 or more copies of the nucleic acid integrate into the genome at either a single site (*e.g.,* in an array of copies) or at multiple sites. A variety of mechanisms can be employed for introducing the first nucleic acid molecule into the genome of somatic cells. For example, in the case where integration is at an essentially random site(s) in the genome, the first nucleic acid can be introduced in a retroviral vector (*e.g.,* a lentiviral vector), an adeno-associated virus vector (without a functional Rep gene) or as part of a transposon system, such as a piggyBac vector. In other aspects, the first nucleic acid is integrated into a selected genomic site, for example, the nucleic acid can be integrated at the AAVS1 integration site (*e.g.,* by use of an adeno-associated virus vector in the presence of a functional Rep gene). Likewise, in certain aspects, integration at a selected genomic site can be by homologous recombination. For example, a meganuclease, a zinc-finger nuclease or a transcription activator-like effector endonuclease (TALEN) that cleaves genomic DNA at the selected site can be used to mediate integration at the selected site. As used herein, integration at a selected genomic site can comprise insertion of the nucleic acid molecules (or a portion thereof) between two contiguous nucleotide positions in the genome or between two nucleotide positions that are not contiguous (*e.g.,* resulting in a replacement of intervening genomic sequences). For example, integration of the nucleic acid at selected genomic sites can comprise replacement of a gene exon, intron, promoter, coding sequence or an entire gene.

In further aspects of the disclosure the first nucleic acid molecule comprises a coding sequence of a screenable or selectable marker. Alternatively or additionally, the first nucleic acid molecule comprises a nucleic acid sequence selected from the group consisting of a sequence that corrects a genetic defect in the cells; a sequence that provides resistance to a pathogen infection; a sequence that provides resistance to a drug; a sequence that provides sensitivity to a drug; a sequence that alters immunogencity of the cells; and a sequence that provides a genetic tag in the cells. Described herein is a method comprising introducing in the somatic cells a second, third, fourth or fifth nucleic acid molecule for integration into the genome, wherein the nucleic acid molecules are different from one another and integrate into the genome independently of one another.

As used herein the genetic element that expresses one or more reprogramming factor(s) may be any genetic material or nucleic acids, such as DNA or RNA. The genetic element may be integrated into the genome of a cell (*i.e.,* either randomly or at a specific site as described in detail above). However, in accordance with the present claims, the genetic element is an element that remains extra-chromosomal upon introduction into the cells such as an episomal vector or RNA. For example, the episomal vector may comprise a replication origin and one or more expression cassettes for expression of reprogramming factors. Such one or more of the expression cassettes may further comprise a nucleotide sequence encoding a trans-acting factor that binds to the replication origin to replicate an extra-chromosomal template. Alternatively or additionally, the somatic cell may express such a trans-acting factor.

In certain aspects, episomal vectors for use according to the invention can be essentially free of bacterial elements. Such bacterial elements may be components of the vector backbone that is required for plasmid propagation in bacteria, such as bacterial origin of replication, *e.g.,* the pUC replication origin, and bacterial selection cassette, *e.g.,* an ampicillin selection cassette.

The replication origin may be a replication origin of a lymphotrophic herpes virus or a gamma herpesvirus, an adenovirus, SV40, a bovine papilloma virus, or a yeast, such as a replication origin of a lymphotrophic herpes virus or a gamma herpesvirus corresponding to oriP of EBV. The lymphotrophic herpes virus may be Epstein Barr virus (EBV), Kaposi's sarcroma herpes virus (KSHV), Herpes virus saimiri (HS), or Marek's disease virus (MDV).

For replication and transient maintenance of extra-chromosomal genetic elements, the trans-acting factor may be a polypeptide corresponding to, or a derivative of, a wild-type protein of EBNA-1 (EBV nuclear antigen 1) of EBV, preferably in the presence of a replication origin corresponding to OriP of EBV. The derivative may have a reduced ability to activate transcription from an integrated template as compared to wild-type EBNA-1 and thus reduced chances to ectopically activate chromosome genes to cause oncogenic transformation. Meanwhile, the derivative may activate transcription at least 5% that of the corresponding wild-type protein from an extra-chromosomal template after the derivative binds the replication origin.

For reprogramming of somatic cells, certain aspects of the present methods may involve using the reprogramming factors sufficient, when expressed in the somatic cell under appropriate cell culture conditions, to convert the somatic cell to a pluripotent stem cell. For example, the reprogramming factor(s) can comprise one or more selected from the group consisting of Sox, Oct, Nanog, Lin-28, Klf4, C-myc, L-myc and SV40LT, for example, a set of Sox, Oct, Nanog, and optionally Lin-28, a set of Sox, Oct, Klf4, and optionally C-myc, or a combination of these factors. To reduce the potential toxic effect of C-myc expression, the SV40 large T gene (SV40LT) may be included with c-Myc. In certain aspects to further improve reprogramming efficiency, Myc mutants, variants or homologs that are deficient in transformation may be used. Non-limiting examples include a Myc proto-oncogene family member such as LMYC (NM_001033081), MYC with 41 amino acid deleted at the N-terminus (dN2MYC), or MYC with mutation at amino acid 136 (W136E).

In certain aspects, the somatic cells for use according to the embodiments are primary human cells, which are cells directly obtained from a living human subject, and may exclude the use of an established or immortalized cell line. Some aspects can comprise the use of terminally differentiated human cells. Non-limiting examples of the primary human cell include a fibroblast, a keratinocyte, a hematopoietic cell, a mesenchymal cell, an adipose cell, an endothelial cell, a neural cell, a muscle cell, an epithelial cell, a mammary cell, a liver cell, a kidney cell, a skin cell, a digestive tract cell, a cumulus cell, a gland cell, or a pancreatic islet cell. More specifically, the primary human cell may be a hematopoietic progenitor cell, such as a CD34⁺ cell. The primary human cell may be obtained from a blood sample, a hair sample, a skin sample, a saliva sample, a solid tissue sample or any sources known to a person of ordinary skill in the art.

In certain aspects, culturing cells under reprogramming conditions comprises culturing the cells in a reprogramming medium. For example, a reprogramming medium may comprise one or more signaling inhibitor(s) (*e.g.,* an inhibitor that has been added to the medium). The signaling inhibitors may be one or more selected from the group consisting of a glycogen synthase kinase 3β (GSK-3β) inhibitor, a mitogen-activated protein kinase kinase (MEK) inhibitor, a transforming growth factor beta (TGF-β) receptor inhibitor, leukemia inhibitory factor (LIF), and a combination thereof. Particularly, the reprogramming medium can comprise a combination of GSK-3 β inhibitor, MEK inhibitor, TGF-β receptor inhibitor, and optionally, LIF. The medium may further comprise externally added ROCK inhibitor or Myosin II inhibitor. The ROCK inhibitor may be HA-100. The medium may further comprise externally added FGF. In certain aspects, the composition may further comprise a chemically defined medium. Non-limiting examples of a chemically defined medium include TeSR medium, human embryonic cell culture medium, N2B27 medium, E8 medium (Chen *et al.,* 2011) commercialized as Essential 8™ medium, and derivatives thereof. Further methods for reprogramming of somatic cells are detailed in U.S. Patent Publn. 20110104125.

Described herein are methods comprising culturing cells in the presence of feeder cells, such as irradiated mouse embryonic fibroblast (MEF) feeder cells. Alternatively, in accordance with the present claims, cells may be cultured in conditions essentially free of feeder cells. For example, a method according to the embodiments may comprise culturing cells in the presence of a matrix component to replace feeder cells to support culture of the cell population. Such a matrix component for cell adhesion can be any material intended to attach stem cells or feeder cells (if used). Non-limiting examples of the matrix component include collagen, gelatin, poly-L-lysine, poly-D-lysine, vitronectin, laminin, and fibronectin and mixtures thereof, for example, Matrigel™ and lysed cell membrane preparations. In a particular example, the matrix composition includes a fibronectin fragment, such as RetroNectin® (see, *e.g.,* U.S. Patent Nos. 5,686,278; 6,033,907, 7,083,979 and 6,670,177). RetroNectin® is a -63 kDa protein of (574 amino acids) that contains a central cell-binding domain (type III repeat, 8,9,10), a high affinity heparin-binding domain II (type III repeat, 12,13,14), and CS1 site within the alternatively spliced IIICS region of human fibronectin.

Culturing of cells under reprogramming conditions may comprise culturing the cells for at least from about one day, one week or one month under reprogramming conditions. For example, the cells can be cultured in a reprogramming medium (*e.g.,* a medium comprising signaling inhibitors as described above) for at least or about 1, 2, 3, 4, 6, 7, 8, 9, 10, 11, 12, 13, 14, 15, 16, 17, 18, 19, 20 days, or any range derivable therein. The reprogramming conditions may last a period including at least from about one day to five days after introduction of the first nucleic acid molecule and/or the extra-chromosomal element into the somatic cells. The starting and ending time points may be selected from the 1, 2, 3, 4, 6, 7, 8, 9, 10, 11, 12, 13, 14, 15, 16, 17, 18, 19, 20 days, or any range derivable therein after the introduction, for example, in accordance with the present claims, from about one day to fifteen days post-transfection of the nucleic acids.

In yet further aspects of the embodiments culturing the cells under reprogramming conditions further comprises selecting or screening the cells for the presence of the first nucleic acid molecule. For example, the cells can be selected or screened by fluorescence activated cell sorting (FACS), magnetic activated cell sorting (MACS) or flow cytometry. Alternatively or additionally, the first nucleic acid may comprise a drug resistance marker and the cells can be selected by addition of an appropriate drug to the cell culture medium (*e.g.,* puromycin). Accordingly, in certain aspects, culturing the cells under reprogramming conditions comprises culturing the cells in a reprogramming medium that comprises drug for selection of cells comprising the first nucleic acid molecule. For example, cells can be cultured in the presence of the selection drug beginning about 1 to 10 days (*e.g.,* about 1 to 2 days, 1 to 3 days or 1 to 5 days) after introduction of the first nucleic acid molecule and/or extra-chromosomal genetic element into the cells. Likewise, in certain aspects, the cells are cultured in the presence of the drug for at least about 1 to 10 days, such as for about 5, 10, 15, 20, 25, 30 or more days. Thus, cells may be cultured in the presence of a selection drug for the entire time that they are in a reprogramming medium (*e.g.,* at least until iPS cells are produced). However, in alternative embodiments, the drug selection only is performed during a portion of the period that the cells are in a reprogramming medium.

In yet a further aspect, the methods of the embodiments may further comprise selecting iPS cells, for example, based on one or more embryonic cell characteristics, such as an ES cell-like morphology. Thus, in still further embodiments, a method comprises selecting pluripotent cells based on the expression of at least a first marker of pluripotency and selecting cells for the presence of the first nucleic acid molecule. Such selection steps can be performed sequentially or essentially concomitantly. For example, a population of cells that express at least a first marker of pluripotency (*e.g.,* Tra160) can be isolated by picking of a clonal cell colony or by FACS. The pluripotent population can then be further separated into cells that comprise the first nucleic acid molecule (engineered iPS cells) and cells that do not comprise the first nucleic acid molecule (iPS cells).

In further aspects of the embodiments, a method of the embodiments comprises the step of (d) culturing the iPS cells and/or engineered iPS cells under expansion conditions. For example, after reprogramming (and/or screening or selection), the cells can be subjected to expansion conditions, such as by culturing in an expansion medium. The expansion medium may, for example, be essentially free of externally added GSK-3 inhibitor, MEK inhibitor, and TGF-β receptor inhibitor. The expansion medium may have one or more of the signaling inhibitors and/or LIF. Examples of expansion media include, but are not limited to, a normal ES cell culture medium, Essential 8 medium or TeSR medium.

In still a further aspect a method of the embodiments comprises (e) characterizing the iPS cells and/or engineered iPS cells. For example, characterizing the iPS cells can comprise detecting one or more pluripotency markers; performing a karoytype analysis; detecting the presence of the first nucleic acid molecule; determining the sequence of the first nucleic acid molecule; detecting the presence of the extra-chromosomal genetic element; teratoma formation analysis; epigenetic analysis; RNA expression analysis; protein expression analysis; or small tandem repeat (STR) detection.

Starting cells for the present methods may comprise at least or about 10⁴, 10⁵, 10⁶, 10⁷, 10⁸, 10⁹, 10¹⁰, 10¹¹, 10¹², 10¹³ cells or any range derivable therein. The starting cell population may have a seeding density of at least or about 10, 10¹, 10², 10³, 10⁴, 10⁵, 10⁶, 10⁷, 10⁸ cells/ml, or any range derivable therein.

Embodiments discussed in the context of methods and/or compositions of the invention may be employed with respect to any other method or composition described herein. Thus, an embodiment pertaining to one method or composition may be applied to other methods and compositions of the invention as well.

As used herein the terms "encode" or "encoding" with reference to a nucleic acid are used to make the invention readily understandable by the skilled artisan; however, these terms may be used interchangeably with "comprise" or "comprising" respectively.

As used herein the specification, "a" or "an" may mean one or more. As used herein in the claim(s), when used in conjunction with the word "comprising", the words "a" or "an" may mean one or more than one.

The use of the term "or" in the claims is used to mean "and/or" unless explicitly indicated to refer to alternatives only or the alternatives are mutually exclusive, although the disclosure supports a definition that refers to only alternatives and "and/or." As used herein "another" may mean at least a second or more.

Throughout this application, the term "about" is used to indicate that a value includes the inherent variation of error for the device, the method being employed to determine the value, or the variation that exists among the study subjects.

Other objects, features and advantages of the present invention will become apparent from the following detailed description. It should be understood, however, that the detailed description and the specific examples, while indicating preferred embodiments of the invention, are given by way of illustration only.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.
**FIG. 1****:** A schematic showing an exemplary method according to the embodiments. In this case, the genome engineering construct includes an expression cassette for zsGreen and pluripotency is assessed by an anti-Tra160 antibody visualized with a red fluorescent dye. The methods result in four possible cells types: 1. A reprogrammed and engineered iPS cell Tra160⁺ (red) and zsGreen⁺ (green); 2. A reprogrammed iPS cell Tra160⁺ and zsGreen⁻; 3. Engineered cells Tra160⁻ and zsGreen⁺; and 4. Cells that are not engineered or reprogrammed, Tra160⁻ and zsGreen⁻.
**FIG. 2****:** A schematic of vector 1024, which can be used in conjunction with a Zinc Finger Nuclease RNA to insert a constitutively expressed puromycin resistance gene at AAVS1 cut site- Chromosome 19: 60,318,931-60,318,961. This vector also inserts a fluorescent (EGFP) under the control of a cardiac specific Tropin T (TNNT2) promoter.
**FIG. 3****:** A schematic of cell reprogramming vector #34, pEP4EO2SEN2K.
**FIG. 4****:** A schematic of cell reprogramming vector #36, pEP4EO2SET2K.
**FIG. 5****:** A schematic of cell reprogramming vector #123, pCEP4-LM2L (also referred to as L-myc ires Lin28).
**FIG. 6****:** A schematic of cell engineering vector #1036, pZD EFx-ZsGreen PGKpuro. This vector can be used in conjunction with a Zinc Finger Nuclease RNA to insert both a constitutively active zsGreen fluorescent protein gene using the pEFx promoter and a constitutively active puromycin resistance gene using the PGK promoter.
**FIG. 7****:** A schematic of piggyBac vector #1038 for constitutive expression of the puromycin resistance gene and ZsGreen fluorescent protein gene. This vector can be used in conjunction with a plasmid encoding the piggyBac transposase or an RNA encoding the piggyBac transposase to insert sequences from plasmid #1038 into the genome.
**FIG. 8****:** A schematic showing an exemplary method according to the embodiments using a piggyBac transposon system for genome engineering.
**FIG. 9****:** A schematic showing an exemplary method according to the embodiments using a Zinc finger nuclease system for genome engineering.
**FIG. 10****:** A schematic comparing the efficiency of using sequential genome engineering and reprogramming to methods of combined genome engineering and reprogramming according to the embodiments of the invention.

### DESCRIPTION OF ILLUSTRATIVE EMBODIMENTS

### I. Introduction

A variety of protocols for procuring iPS cells, both as therapeutics and as research tools, require that the cells be engineered with exogenous nucleic acid molecules incorporated into their genome. However, reprogramming of iPS cells is, itself, a complex, time consuming and expensive process. Further genomic engineering of such cells complicates and lengthens the production process and increases the cost of any resulting cells. The field is therefore in need of improved methods for production of engineered iPS cells.

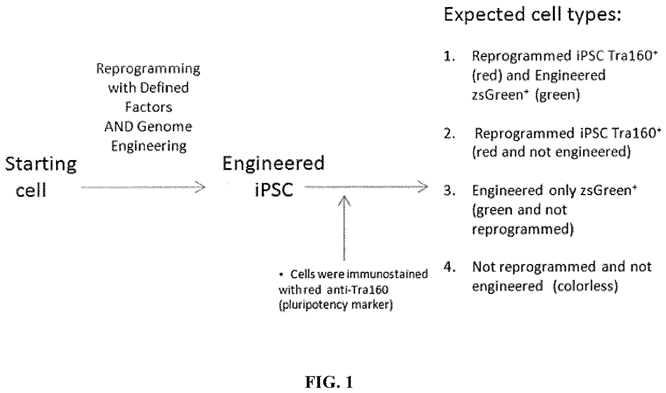

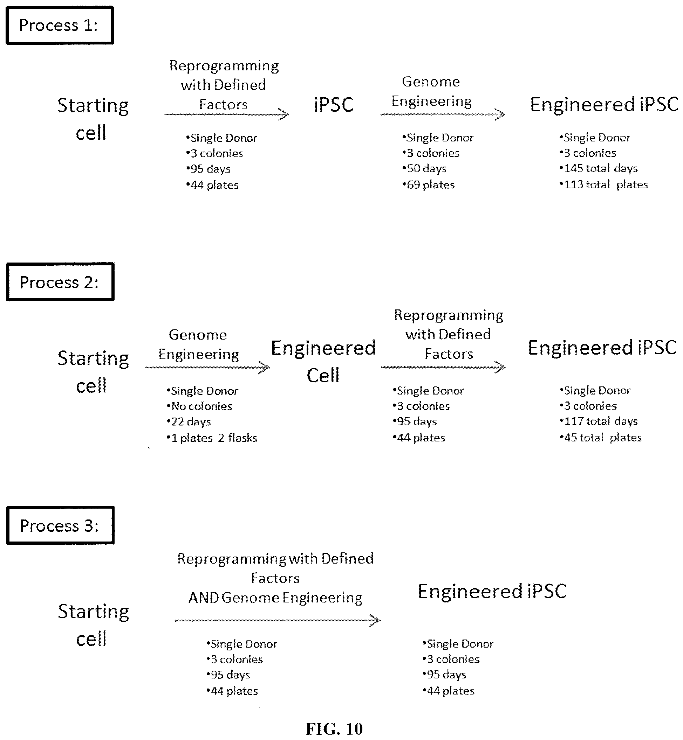

The present invention is based, in part, on the surprising discovery of methods that allow for virtually simultaneous reprogramming and engineering of cells to produce genetically engineered iPS cells. In particular, it has been found that, even after successful reprogramming, iPS cells can be produced with an engineered construct in their genome which is not silenced during the reprogramming process and can be expressed in the iPS cells (or differentiated cells produced therefrom). Moreover, despite the need for stringent control of cell culture conditions during somatic cell reprogramming, cells can be screened or even selected for genomic integration of a selected nucleic acid molecule during the reprogramming process. After such parallel selection (or screening) and reprogramming iPS cell clones representing individual integration events can be isolated and expanded. This combined process, an example of which is depicted in FIG. 1 and FIG. 10, results in significant time and cost savings. Specifically, a method according to the embodiments can take at least 1/3 less time (34% less) and use fewer than half as many culture plates (60% fewer) as methods that involve serial reprogramming followed by genome engineering. Likewise, in addition to isolation of the genetically engineered iPS cells, cells that do not comprise an integration event can be simultaneously isolated thereby producing both iPS cells and engineered iPS cells in the same method. The ability to generated both engineered and non-engineered iPSCs in tandem is particularly useful because the non-engineered iPSCs can serve as control cells for the characterization of the engineered iPSCs.

Further advances in the composition and methods for production of engineered iPS cell populations are also described below.

### II. Definitions

A "primary cell," as used herein, refers to a cell directly obtained from a living organism or a progeny thereof without being established or immobilized into a cell line. A "human primary cell" refers to a primary cell obtained from a living human subject.

"Embryonic stem (ES) cells" are pluripotent stem cells that may be derived from early embryos. An ES cell was first established in 1981, which has also been applied to production of knockout mice since 1989. In 1998, a human ES cell was established, which is currently becoming available for regenerative medicine.

"Induced pluripotent stem cells," commonly abbreviated as iPS cells or iPSCs, refer to a type of pluripotent stem cell artificially prepared from a non-pluripotent cell, typically an adult somatic cell, or terminally differentiated cell, such as fibroblast, a hematopoietic cell, a myocyte, a neuron, an epidermal cell, or the like, by reprogramming.

"Pluripotency" refers to a stem cell that has the potential to differentiate into all cells constituting one or more tissues or organs, or preferably, any of the three germ layers: endoderm (interior stomach lining, gastrointestinal tract, the lungs), mesoderm (muscle, bone, blood, urogenital), or ectoderm (epidermal tissues and nervous system). "Pluripotent stem cells" used herein refer to cells that can differentiate into cells derived from any of the three germ layers, for example, direct descendants of totipotent cells, embryonic stem cell, or induced pluripotent stem cells.

As used herein, the term "somatic cell" refers to any cell other than germ cells, such as an egg, a sperm, or the like, which does not directly transfer its DNA to the next generation. Typically, somatic cells have limited or no pluripotency. Somatic cells used herein may be naturally-occurring or genetically modified.

"Reprogramming" is a process that confers on a cell a measurably increased capacity to form progeny of at least one new cell type, either in culture or *in vivo,* than it would have under the same conditions without reprogramming. More specifically, reprogramming is a process that confers on a somatic cell a pluripotent potential. This means that after sufficient proliferation, a measurable proportion of progeny having phenotypic characteristics of the new cell type if essentially no such progeny could form before reprogramming; otherwise, the proportion having characteristics of the new cell type is measurably more than before reprogramming. Under certain conditions, the proportion of progeny with characteristics of the new cell type may be at least about 0.05%, 0.1%, 0.5%, 1%, 5%, 25% or more in the in order of increasing preference.

As used herein the term "engineered" in reference to cells refers to cells that comprise at least one genetic element exogenous to the cell that is integrated into the cell genome. In some aspects, the exogenous genetic element can be integrated at a random location in the cell genome. In other aspects, the genetic element is integrated at a specific site in the genome. For example, the genetic element may be integrated at a specific position to replace an endogenous nucleic acid sequence, such as to provide a change relative to the endogenous sequence (*e.g.,* a change in single nucleotide position).

The term "exogenous," when used in relation to a protein, gene, nucleic acid, polynucleotide, genetic elements, or vector elements in a cell or organism, refers to a protein, gene, nucleic acid, polynucleotide, genetic element or vector element which has been introduced into the cell or organism by artificial or natural means, or in relation to a cell, refers to a cell which was isolated and subsequently introduced to other cells or to an organism by artificial or natural means. An exogenous nucleic acid may be from a different organism or cell, or it may be one or more additional copies of a nucleic acid which occurs naturally within the organism or cell. An exogenous cell may be from a different organism, or it may be from the same organism. By way of a non-limiting example, an exogenous nucleic acid is in a chromosomal location different from that of natural cells, or is otherwise flanked by a different nucleic acid sequence than that found in nature. Alternatively, an exogenous nucleic acid may be extrachromosomal, such as in an episomal vector.

The term "drug" refers to a molecule including, but not limited to, small molecules, nucleic acids and proteins or combinations thereof that alter or are candidates for altering a phenotype associated with disease.

An "origin of replication" ("ori") or "replication origin" is a DNA sequence, *e.g.,* in a lymphotrophic herpes virus, that when present in a plasmid in a cell is capable of maintaining linked sequences in the plasmid, and/or a site at or near where DNA synthesis initiates. An ori for EBV includes FR sequences (20 imperfect copies of a 30 bp repeat), and preferably DS sequences, however, other sites in EBV bind EBNA-1, *e.g.,* Rep* sequences can substitute for DS as an origin of replication (Kirchmaier and Sugden, 1998). Thus, a replication origin of EBV includes FR, DS or Rep* sequences or any functionally equivalent sequences through nucleic acid modifications or synthetic combination derived therefrom. For example, the present invention may also use genetically engineered replication origin of EBV, such as by insertion or mutation of individual elements, as specifically described in Lindner *et al* (2008).

A "lymphotrophic" herpes virus is a herpes virus that replicates in a lymphoblast (*e.g.,* a human B lymphoblast) or other cell types and replicates extra-chromosomally for at least a part of its natural life-cycle. After infecting a host, these viruses latently infect the host by maintaining the viral genome as a plasmid. Herpes simplex virus (HSV) is not a "lymphotrophic" herpes virus. Exemplary lymphotropic herpes viruses include, but are not limited to EBV, Kaposi's sarcoma herpes virus (KSHV), Herpes virus saimiri (HS) and Marek's disease virus (MDV).

A "vector" or "construct" (sometimes referred to as gene delivery or gene transfer "vehicle") refers to a macromolecule or complex of molecules comprising a polynucleotide to be delivered to a host cell, either *in vitro* or *in vivo.*

A "plasmid", a common type of a vector, is an extra-chromosomal DNA molecule separate from the chromosomal DNA which is capable of replicating independently of the chromosomal DNA. In certain cases, it is circular and double-stranded.

A "template" as used herein is a DNA or RNA molecule which contains a replication origin. An "integrated template" is one which is stably maintained in the genome of the cell, *e.g.,* integrated into a chromosome of that cell. An "extra-chromosomal template" is one which is maintained stably in a cell but which is not integrated into the chromosome.

By "expression construct" or "expression cassette" is meant a nucleic acid molecule that is capable of directing transcription. An expression construct includes, at the least, a promoter or a structure functionally equivalent to a promoter. Additional elements, such as an enhancer, and/or a transcription termination signal, may also be included. A nucleic acid molecule may be DNA or RNA.

The term "corresponds to" is used herein to mean that a polynucleotide sequence is homologous (*i.e.,* is identical, not strictly evolutionarily related) to all or a portion of a reference polynucleotide sequence, or that a polypeptide sequence is identical to a reference polypeptide sequence. In contradistinction, the term "complementary to" is used herein to mean that the complementary sequence is homologous to all or a portion of a reference polynucleotide sequence. For illustration, the nucleotide sequence "TATAC" corresponds to a reference sequence "TATAC" and is complementary to a reference sequence "GTATA".

### III. iPS cells

Induced pluripotent stem cells, commonly abbreviated as iPS cells or iPSCs, are a type of pluripotent stem cell artificially derived from a non-pluripotent cell, typically an adult somatic cell. Induced pluripotent stem cells are believed to be similar if not identical to natural pluripotent stem cells, such as embryonic stem cells in many respects, such as in terms of the expression of certain stem cell genes and proteins, chromatin methylation patterns, doubling time, embryoid body formation, teratoma formation, viable chimera formation, and potency and differentiability, but the full extent of their relation to natural pluripotent stem cells is still being assessed.

Generation of induced pluripotent cells derived from human tissue other than of embryonic origin is desired to alleviate ethical concerns regarding experimental use of embryos and embryonic tissue. The promise of therapeutic applications from induced pluripotent cells has been touted. Medical applications include treatments for Alzheimer's disease, Diabetes and Spinal cord injuries to name a few. Other applications include disease modeling and pharmaceutical drug screening.

iPS cells were first produced in 2006 (Takahashi *et al.,* 2006) from mouse cells and in 2007 from human cells (Takahashi *et al.,* 2007; Yu *et al,* 2007). This has been cited as an important advancement in stem cell research, as it may allow researchers to obtain pluripotent stem cells, which are important in research and potentially have therapeutic uses, without the controversial use of embryos. The first successful demonstration of generating induced pluripotent cells (iPS cells) from mouse or human tissue involved the use of retroviral vectors expressing a specific set of transcription factors. Research in the laboratories of James Thomson and Shinya Yamanaka has demonstrated that introduction of specific transcription factors by retroviral vectors into mouse or human fibroblasts is sufficient to reprogram those cells to undifferentiated pluripotent stems cells. The factors used by Thomson include Oct4, Sox2, Nanog and Lin28. The factors used by Yamanaka include Oct4, Sox2, Klf4 and c-Myc. Reprogramming via either gene set is accomplished by integration into the host cell genome and expression of the transcription factors. Oct4 and Sox2 appear to be essential transcription factors required for reprogramming. The efficiency of reprogramming is low with frequencies in the range of 0.01 - 0.02% of the starting cell population.

Original embryonic stem cells (ES cells) are pluripotent stem cells derived from the inner cell mass of the blastocyst, an early-stage embryo. ES cells are distinguished by two distinctive properties: their pluripotency and their capability to self-renew themselves indefinitely. ES cells are pluripotent, that is, they are able to differentiate into all derivatives of the three primary germ layers: ectoderm, endoderm, and mesoderm. Additionally, under defined conditions, embryonic stem cells are capable of propagating themselves indefinitely. This allows embryonic stem cells to be employed as useful tools for both research and regenerative medicine, because they can produce limitless numbers of themselves for continued research or clinical use.

However, there are notable differences between mouse and human ES cells. Human ES cells, when discovered by James Thomson, were found to be different than mouse ES cells in their potency and in their culture conditions, notable by being totally non-responsive to LIF (a required element in culturing mouse ES cells), which results from an inactive leukemia inhibitory factor pathway in human ES cells. Existing human iPS cells are similar to human ES cells in these regards, therefore they could termed human ES cell-like iPS cells.

### IV. Genome Integration of Nucleic Acids

In certain embodiments, the invention involves genomic integration of nucleic acid molecules via genetic engineering. For example, such nucleic acids may be used to correct a genetic defect in the cells, provide resistance to a pathogen infection, provide resistance to a drug, provide sensitivity to a drug, to alter the immunogencity of the cells or to provide a genetic tag in the cells (*e.g.,* an expressed fluorescent marker). Methods for effecting either site-specific or random genome integration of such nucleic acid molecules are known in the art and can be used for genetic engineering.

### A. Viral Vectors

Viral vectors may be employed to facilitate integration of nucleic acid molecules into the genome of cells. Retroviruses, for example, can be used to randomly integrate nucleic acid molecules into a host cell genome. In order to construct a retroviral vector, a nucleic acid is inserted into the viral genome in the place of certain viral sequences to produce a virus that is replication-defective. In order to produce virions, a packaging cell line containing the gag, pol, and env genes but without the LTR and packaging components is constructed (Mann *et al.,* 1983). When a recombinant plasmid containing a cDNA, together with the retroviral LTR and packaging sequences is introduced into a special cell line *(e.g.,* by calcium phosphate precipitation for example), the packaging sequence allows the RNA transcript of the recombinant plasmid to be packaged into viral particles, which are then secreted into the culture media (Nicolas and Rubenstein, 1988; Temin, 1986; Man*n et al.,* 1983). The media containing the recombinant retroviruses is then collected, optionally concentrated, and used for gene transfer. Retroviral vectors are able to infect a broad variety of cell types. However, integration and stable expression require the division of host cells (Paskind *et al.,* 1975).

Lentiviruses are complex retroviruses, which, in addition to the common retroviral genes *gag, pol,* and *env,* contain other genes with regulatory or structural function. Lentiviral vectors are well known in the art (see, for example, Naldini *et al.,* 1996; Zufferey *et al.,* 1997; Blomer *et al.,* 1997; U.S. Patents 6,013,516 and 5,994,136).

Recombinant lentiviral vectors are capable of infecting non-dividing cells and can be used for both *in vivo* and *ex vivo* gene transfer and expression of nucleic acid sequences. For example, recombinant lentivirus capable of infecting a non-dividing cell wherein a suitable host cell is transfected with two or more vectors carrying the packaging functions, namely gag, pol and env, as well as rev and tat is described in U.S. Patent 5,994,136.

Likewise, adeno-associated viral (AAV) vectors can be used to mediate integration of a nucleic acid molecules into a host cell genome. For example, a gut-less AAV vector can be used such that inverted terminal repeats (ITRs) of the virus flank the nucleic acid molecule for integration. If a cell is transduced with such a vector, essentially random genome integration can be achieved. On the other hand, if cells are transduced in the presence of a functional AAV Rep gene (either in the virus or expressed in trans) then site-specific integration of the sequence at the AAVS1 integration site can be accomplished.

### B. Transposon-based integration systems

The integration of a nucleic acid may use a transposon - transposase system. Such systems can be used to effectively and randomly insert a nucleic acid molecule into a cell genome. For example, the used transposon - transposase system could be the well known Sleeping Beauty, the Frog Prince transposon - transposase system (for the description of the latter see *e.g.,* EP1507865), or the TTAA-specific transposon piggyBac system.

Transposons are sequences of DNA that can move around to different positions within the genome of a single cell, a process called transposition. In the process, they can cause mutations and change the amount of DNA in the genome. Transposons were also once called jumping genes, and are examples of mobile genetic elements.

There are a variety of mobile genetic elements, and they can be grouped based on their mechanism of transposition. Class I mobile genetic elements, or retrotransposons, copy themselves by first being transcribed to RNA, then reverse transcribed back to DNA by reverse transcriptase, and then being inserted at another position in the genome. Class II mobile genetic elements move directly from one position to another using a transposase to "cut and paste" them within the genome. Any such system can be used to mediate genomic integration of nucleic acid molecules according to the embodiments.

### C. Homologous recombination

In certain aspects of the invention, nucleic acid molecules can be introduced into cells in a specific manner for genome engineering, for example, via homologous recombination. As discussed above, some approaches to express genes in cells involve the use of viral vectors or transgenes that integrate randomly in the genome. These approaches, however, have the drawback of integration occurring either at sites that are unable to effectively mediate expression from the integrated nucleic or that result in the disruption of native genes. Problems associated with random integration could be partially overcome by homologous recombination to a specific locus in the target genome, *e.g.,* the AAVS1 or Rosa26 locus.

Homologous recombination (HR), also known as general recombination, is a type of genetic recombination used in all forms of life in which nucleotide sequences are exchanged between two similar or identical strands of DNA. The technique has been the standard method for genome engineering in mammalian cells since the mid 1980s. The process involves several steps of physical breaking and the eventual rejoining of DNA. This process is most widely used to repair potentially lethal double-strand breaks in DNA. In addition, homologous recombination produces new combinations of DNA sequences during meiosis, the process by which eukaryotes make germ cells like sperm and ova. These new combinations of DNA represent genetic variation in offspring which allow populations to evolutionarily adapt to changing environmental conditions over time. Homologous recombination is also used in horizontal gene transfer to exchange genetic material between different strains and species of bacteria and viruses. Homologous recombination is also used as a technique in molecular biology for introducing genetic changes into target organisms.

Homologous recombination can be used as targeted genome modification. The efficiency of standard HR in mammalian cells is only 10⁻⁶ to 10⁻⁹ of cells treated (Capecchi, 1990). The use of meganucleases, or homing endonucleases, such as I-SceI have been used to increase the efficiency of HR. Both natural meganucleases as well as engineered meganucleases with modified targeting specificities have been utilized to increase HR efficiency (Pingoud and Silva, 2007; Chevalier et al., 2002).

On the path toward increasing the efficiency of HR has been to engineer chimeric endonucleases with programmable DNA specificity domains (Silva et al., 2011). Zinc-finger nucleases (ZFN) are one example of such a chimeric molecule in which Zinc-finger DNA binding domains are fused with the catalytic domain of a Type IIS restriction endonuclease such as FokI (as reviewed in Durai *et al.,* 2005; PCT/US2004/030606).

Another class of such specificity molecules includes Transcription Activator Like Effector (TALE) DNA binding domains fused to the catalytic domain of a Type IIS restriction endonuclease such as FokI (Miller et al., 2011: PCT/IB2010/000154). TALENs can be designed for site-specific genome modification at virtually any given site of interest (Cermak *et al.,* 2011; Christian *et al.,* 2010; Li *et al.,* 2011; Miller *et al.,* 2011; Weber *et al.,* 2011; Zhang *et al.,* 2011). The site-specific DNA binding domain is expressed as a fusion protein with a DNA cleavage enzyme such as Fok I. The DNA binding domain is a scaffold of repeating amino acids; linking each of the repeats are two variable amino acids that bind to a single nucleotide in the DNA. For example, Asn-Asn binds guanosine, Asn-Ile binds adenosine, Asn-Gly bind thymidine, and His-Asp binds Cytosine. These two amino acids are known as the Repeat Variable Diresidue or RVD. There are many different RVD's and they can be engineered into the TAL Effector/Fok1 protein construct to create a specific TALEN. The RNA encoding the recombinant TALEN can then be purified and transfected into a cell for site-specific genome modification. Once the TALEN introduces the double strand DNA break, the DNA can be modified by non-homologous end joining (NHEJ) or by homologous directed repair (HDR). This allows DNA mutagenesis, deletions, or additions depending on what additional sequences are present during the DNA repair.

### V. Genetic Elements for Reprogramming

Induction of pluripotent stem cells from human somatic cells has been achieved using retroviruses or lentiviral vectors for ectopic expression of reprogramming genes. Recombinant retroviruses such as the Moloney murine leukemia virus have the ability to integrate into the host genome in a stable fashion. They contain a reverse transcriptase which allows integration into the host genome. Lentiviruses are a subclass of retroviruses. They are widely adapted as vectors thanks to their ability to integrate into the genome of non-dividing as well as dividing cells. The viral genome in the form of RNA is reverse-transcribed when the virus enters the cell to produce DNA, which is then inserted into the genome at a random position by the viral integrase enzyme. Therefore, current technology of successful reprogramming is dependent on integration-based viral approaches.

However, methods of the present invention makes use of extra-chromosomal genetic element for reprogramming. For example, extra-chromosomally replicating vectors, or vectors capable of replicating episomally (see U.S. Patent Publn. 20100003757) can be employed. In further aspects, RNA molecules encoding reprogramming factors or reprogramming factor proteins can be employed. In each case, expression of reprogramming factors can be used in combination with culturing of cells in the presence of cellular signaling inhibitors to achieve optimal reprogramming efficiency and kinetics.

A number of DNA viruses, such as adenoviruses, Simian vacuolating virus 40 (SV40), bovine papilloma virus (BPV), or budding yeast ARS (Autonomously Replicating Sequences)-containing plasmids replicate extra-chromosomally in mammalian cells. These episomal plasmids are intrinsically free from all these disadvantages (Bode *et al.,* 2001) associated with integrating vectors. A lymphotrophic herpes virus-based system including Epstein Barr Virus (EBV) may also replicate extra-chromosomally and help deliver reprogramming genes to somatic cells.

For example, the episomal vector-based approach used in the invention extracts robust elements necessary for the successful replication and maintenance of an EBV element-based system without compromising the system's tractability in a clinical setting as described in detail below. The useful EBV elements are OriP and EBNA-1, or their variants or functional equivalents. An additional advantage of this system is that these exogenous elements will be lost with time after being introduced into cells, leading to self-sustained iPS cells essentially free of these elements.

### A. Epstein-Barr Virus

The Epstein-Barr Virus (EBV), also called Human herpesvirus 4 (HHV-4), is a virus of the herpes family (which includes Herpes simplex virus and Cytomegalovirus), and is one of the most common viruses in humans. EBV maintains its genome extra-chromosomally and works in collaboration with host cell machinery for efficient replication and maintenance (Lindner and Sugden, 2007), relying solely on two essential features for its replication and its retention within cells during cell division (Yates *et al.* 1985; Yates *et al.* 1984). One element, commonly referred to as *oriP,* exists *in cis* and serves as the origin of replication. The other factor, EBNA-1, functions *in trans* by binding to sequences within *oriP* to promote replication and maintenance of the plasmid DNA. As a non-limiting example, certain aspects of the invention extract these two features and use them in the context of a vector to shuttle the genes necessary for reprogramming somatic cells to facilitate the replication and sustained expression of these genes over conventional plasmids.

### B. Replication Origin

In certain aspects, a replication origin of EBV, *OriP,* may be used. *OriP* is the site at or near which DNA replication initiates and is composed of two *cis*-acting sequences approximately 1 kilobase pair apart known as the family of repeats (FR) and the dyad symmetry (DS).

FR is composed of 21 imperfect copies of a 30 bp repeat and contains 20 high affinity EBNA-1-binding sites. When FR is bound by EBNA-1, it both serves as a transcriptional enhancer of promoters in *cis* up to 10 kb away (Reisman and Sugden, 1986; Yates, 1988; Sugden and Warren, 1989; Wysokenski and Yates, 1989; Gahn and Sugden, 1995; Kennedy and Sugden, 2003; Altmann *et al.,* 2006), and contributes to the nuclear retention and faithful maintenance of FR containing plasmids (Langle-Rouault *et al.,* 1998; Kirchmaier and Sugden, 1995; Wang *et al.,* 2006; Nanbo and Sugden, 2007). The efficient partitioning of oriP plasmids is also likely attributable to FR. While the virus has evolved to maintain 20 EBNA-1-binding sites in FR, efficient plasmid maintenance requires only seven of these sites, and can be reconstituted by a polymer of three copies of DS, having a total of 12 EBNA-1-binding sites (Wysokenski and Yates, 1989).

The dyad symmetry element (DS) is sufficient for initiation of DNA synthesis in the presence of EBNA-1 (Aiyar *et al.,* 1998; Yates *et al.,* 2000), and initiation occurs either at or near DS (Gahn and Schildkraut, 1989; Niller *et al.,* 1995). Termination of viral DNA synthesis is thought to occur at FR, because when FR is bound by EBNA-1 it functions as a replication fork barrier as observed by 2D gel electrophoresis (Gahn and Schildkraut, 1989; Ermakova *et al.,* 1996; Wang *et al.,* 2006). Initiation of DNA synthesis from DS is licensed to once-per-cell-cycle (Adams, 1987; Yates and Guan, 1991), and is regulated by the components of the cellular replication system (Chaudhuri *et al.,* 2001; Ritzi *et al.,* 2003; Dhar *et al.,* 2001; Schepers *et al.,* 2001; Zhou *et al.,* 2005; Julien *et al.,* 2004). DS contains four EBNA-1-binding sites, albeit with lower affinity than those found in FR (Reisman *et al.,* 1985). The topology of DS is such that the four binding sites are arranged as two pairs of sites, with 21 bp center-to-center spacing between each pair and 33 bp center-to-center spacing between the two non-paired internal binding sites (Baer *et al.,* 1984; Rawlins *et al.,* 1985).

The functional roles of the elements within DS have been confirmed by studies of another region of EBV's genome, termed Rep*, which was identified as an element that can substitute for DS inefficiently (Kirchmaier and Sugden, 1998). Polymerizing Rep* eight times yielded an element as efficient as DS in its support of replication (Wang *et al.,* 2006). Biochemical dissection of Rep* identified a pair of EBNA-1-binding sites with a 21 bp center-to-center spacing critical for its replicative function (ibid). The minimal replicator of Rep* was found to be the pair of EBNA-1-binding sites, as replicative function was retained even after all flanking sequences in the polymer were replaced with sequences derived from lambda phage. Comparisons of DS and Rep* have revealed a common mechanism: these replicators support the initiation of DNA synthesis by recruiting the cellular replicative machinery via a pair of appropriately spaced sites, bent and bound by EBNA-1.

There are other extra-chromosomal, licensed plasmids that replicate in mammalian cells that are unrelated to EBV and in some ways appear similar to the zone of initiation within the Raji strain of EBV. Hans Lipps and his colleagues have developed and studied plasmids that contain "nuclear scaffold/matrix attachment regions" (S/MARs) and a robust transcriptional unit (Piechaczek *et al.,* 1999; Jenke *et al.,* 2004). Their S/MAR is derived from the human interferon-beta gene, is A/T rich, and operationally defined by its association with the nuclear matrix and its preferential unwinding at low ionic strength or when embedded in supercoiled DNA (Bode *et al.,* 1992). These plasmids replicate semiconservatively, bind ORC proteins, and support the initiation of DNA synthesis effectively randomly throughout their DNA (Schaarschmidt *et al.,* 2004). They are efficiently maintained in proliferating hamster and human cells without drug selection and when introduced into swine embryos can support expression of GFP in most tissues of fetal animals(Manzini *et al.,* 2006).

### C. Trans-acting Factor

A particular example of the trans-acting factor could be Epstein Barr nuclear antigen 1 (EBNA-1), which is a DNA-binding protein that binds to FR and DS of *oriP* or Rep* to facilitate replication and faithful partitioning of the EBV-based vector to daughter cells independent of, but in concert with, cell chromosomes during each cell division.

The 641 amino acids (AA) of EBNA-1 have been categorized into domains associated with its varied functions by mutational and deletional analyses. Two regions, between AA40-89 and AA329-378 are capable of linking two DNA elements in *cis* or in *trans* when bound by EBNA-1, and have thus been termed Linking Region 1 and 2 (LR1, LR2) (Middleton and Sugden, 1992; Frappier and O'Donnell, 1991; Su *et al.,* 1991; Mackey *et al.,* 1995). Fusing these domains of EBNA-1 to GFP homes the GFP to mitotic chromosomes (Marechal *et al.,* 1999; Kanda *et al.,* 2001). LR1 and LR2 are functionally redundant for replication; a deletion of either one yields a derivative of EBNA-1 capable of supporting DNA replication (Mackey and Sugden, 1999; Sears *et al.,* 2004). LR1 and LR2 are rich in arginine and glycine residues, and resemble the AT-hook motifs that bind A/T rich DNA (Aravind and Landsman, 1998), (Sears *et al.,* 2004). An *in vitro* analysis of LR1 and LR2 of EBNA-1 has demonstrated their ability to bind to A/T rich DNA (Sears *et al.,* 2004). When LR1, containing one such AT-hook, was fused to the DNA-binding and dimerization domain of EBNA-1, it was found to be sufficient for DNA replication of oriP plasmids, albeit less efficiently than the wild-type EBNA-1 (ibid).

LR1 and LR2 do differ, though. The C-terminal half of LR1 is composed of amino acids other than the repeated Arg-Gly of the N-terminal half, and is termed unique region 1 (UR1). UR1 is necessary for EBNA-1 to activate transcription efficiently from transfected and integrated reporter DNAs containing FR (Wu *et al.,* 2002; Kennedy and Sugden, 2003; Altmann *et al.,* 2006). UR1 is also essential for the efficient transformation of B-cells infected by EBV. When a derivative of EBNA-1 lacking this domain replaces the wild-type protein in the context of the whole virus, these derivative viruses have 0.1% of the transforming ability of the wild-type virus (Altmann *et al.,* 2006).

LR2 is not required for EBNA-1's support of oriP replication (Shire *et al.,* 1999; Mackey and Sugden, 1999; Sears *et al.,* 2004). Additionally, the N-terminal half of EBNA-1 can be replaced with cellular proteins containing AT-hook motifs, such as HMGA1a, and still retain replicative function (Hung *et al.,* 2001; Sears *et al.,* 2003; Altmann *et al.,* 2006). These findings indicate that it likely is the AT-hook activities of LR1 and LR2 that are required for the maintenance of oriP in human cells.

A third of EBNA-1's residues (AA91-328) consist of glycine-glycine-alanine (GGA) repeats, implicated in EBNA-1's ability to evade the host immune response by inhibiting proteosomal degradation and presentation (Levitskaya *et al.,* 1995; Levitskaya *et al.,* 1997). These repeats have also been found to inhibit translation of EBNA-1 *in vitro* and *in vivo* (Yin *et al.,* 2003). However, the deletion of much of this domain has no apparent effect on functions of EBNA-1 in cell culture, making the role that this domain plays difficult to elucidate.

A nuclear localization signal (NLS) is encoded by AA379-386, which also associates with the cellular nuclear importation machinery (Kim *et al.,* 1997; Fischer *et al.,* 1997). Sequences within the Arg-Gly rich regions of LR1 and LR2 may also function as NLSs due to their highly basic content.

Lastly, the C-terminus (AA458-607) encodes the overlapping DNA-binding and dimerization domains of EBNA-1. The structure of these domains bound to DNA has been solved by X-ray crystallography, and was found to be similar to the DNA-binding domain of the E2 protein of papillomaviruses (Hegde *et al.,* 1992; Kim *et al.,* 2000; Bochkarev *et al.,* 1996).

A reprogramming vector may contain both *oriP* and an abbreviated sequence encoding a version of EBNA-1 competent to support plasmid replication and its proper maintenance during cell division. The highly repetitive sequence within the amino-terminal one-third of wild-type EBNA-1 and removal of a 25 amino-acid region that has demonstrated toxicity in various cells are dispensable for EBNA-1's trans-acting function associated with *oriP* (Yates *et al.* 1985; Kennedy *et al.* 2003). Therefore, the abbreviated form of EBNA-1, known as deltaUR1, could be used alongside *oriP* within this episomal vector-based system in one embodiment.

A derivative of EBNA-1 that may be used in the invention is a polypeptide which, relative to a corresponding wild-type polypeptide, has a modified amino acid sequence. The modifications include the deletion, insertion or substitution of at least one amino acid residue in a region corresponding to the unique region (residues about 65 to about 89) of LR1 (residues about 40 to about 89) in EBNA-1, and may include a deletion, insertion and/or substitution of one or more amino acid residues in regions corresponding to other residues of EBNA-1, *e.g.,* about residue 1 to about residue 40, residues about 90 to about 328 ("Gly-Gly-Ala" repeat region), residues about 329 to about 377 (LR2), residues about 379 to about 386 (NLS), residues about 451 to about 608 (DNA binding and dimerization), or residues about 609 to about 641, so long as the resulting derivative has the desired properties, *e.g.,* dimerizes and binds DNA containing an ori corresponding to oriP, localizes to the nucleus, is not cytotoxic, and activates transcription from an extra-chromosomal but does not substantially active transcription from an integrated template.

### D. Residue-free feature

Importantly, the replication and maintenance of *oriP*-based episomal vector is imperfect and is lost precipitously (25% per cell division) from cells within the first two weeks of its being introduced into cells; however, those cells that retain the plasmid lose it less frequently (3% per cell division) (Leight and Sugden, 2001; Nanbo and Sugden, 2007). Once selection for cells harboring the plasmid is removed, plasmids will be lost during each cell division until all of them have been eliminated over time without leaving a footprint of its former existence within the resulting daughter cells. Certain aspects of the invention make use of this footprint-less feature of the *oriP*-based system as an alternative to the current viral-associated approach to deliver genes to generate iPS cells. Other extra-chromosomal vectors will also be lost during replication and propagation of host cells and could also be employed in the present invention.

### E. Reprogramming Factors

The generation of iPS cells is crucial on the genes used for the induction. The following factors or combination thereof could be used in the vector system disclosed in the present invention. In certain aspects, nucleic acids encoding Sox and Oct (preferably Oct3/4) will be included into the reprogramming vector. For example, a reprogramming vector may comprise expression cassettes encoding Sox2, Oct4, Nanog and optionally Lin-28, or expression cassettes encoding Sox2, Oct4, Klf4 and optionally C-myc, L-myc or Glis-1. Nucleic acids encoding these reprogramming factors may be comprised in the same expression cassette, different expression cassettes, the same reprogramming vector, or different reprogramming vectors.

Oct-3/4 and certain members of the Sox gene family (Sox1, Sox2, Sox3, and Sox15) have been identified as crucial transcriptional regulators involved in the induction process whose absence makes induction impossible. Additional genes, however, including certain members of the Klf family (Klf1, Klf2, Klf4, and Klf5), the Myc family (C-myc, L-myc, and N-myc), Nanog, and LIN28, have been identified to increase the induction efficiency.

Oct-3/4 (Pou5f1) is one of the family of octamer ("Oct") transcription factors, and plays a crucial role in maintaining pluripotency. The absence of Oct-3/4 in Oct-3/4+ cells, such as blastomeres and embryonic stem cells, leads to spontaneous trophoblast differentiation, and presence of Oct-3/4 thus gives rise to the pluripotency and differentiation potential of embryonic stem cells. Various other genes in the "Oct" family, including Oct-3/4's close relatives, Oct1 and Oct6, fail to elicit induction.

The Sox family of genes is associated with maintaining pluripotency similar to Oct-3/4, although it is associated with multipotent and unipotent stem cells in contrast with Oct-3/4, which is exclusively expressed in pluripotent stem cells. While Sox2 was the initial gene used for induction by Takahashi *et al.* (2006), Wernig *et al.* (2007), and Yu *et al.* (2007), other genes in the Sox family have been found to work as well in the induction process. Sox1 yields iPS cells with a similar efficiency as Sox2, and genes Sox3, Sox15, and Sox18 also generate iPS cells, although with decreased efficiency.

Nanog is a transcription factor critically involved with self-renewal of undifferentiated embryonic stem cells. In humans, this protein is encoded by the *NANOG* gene. Nanog is a gene expressed in embryonic stem cells (ESCs) and is thought to be a key factor in maintaining pluripotency. NANOG is thought to function in concert with other factors such as Oct4 (POU5F1) and Sox2 to establish ESC identity.

LIN28 is an mRNA binding protein expressed in embryonic stem cells and embryonic carcinoma cells associated with differentiation and proliferation. Yu *et al.* (2007) demonstrated it is a factor in iPS generation, although it is not essential.

Klf4 of the Klf family of genes was initially identified by Takahashi *et al.* (2006) and confirmed by Wernig *et al.* (2007) as a factor for the generation of mouse iPS cells and was demonstrated by Takahashi *et al.* (2007) as a factor for generation of human iPS cells. However, Yu *et al.* (2007) reported that Klf4 was not essential for generation of human iPS cells. Klf2 and Klf4 were found to be factors capable of generating iPS cells, and related genes Klf1 and Klf5 did as well, although with reduced efficiency.

The Myc family of genes are proto-oncogenes implicated in cancer. Takahashi *et al.* (2006) and Wernig *et al.* (2007) demonstrated that C-myc is a factor implicated in the generation of mouse iPS cells and Yamanaka *et al.* demonstrated it was a factor implicated in the generation of human iPS cells. However, Yu *et al.* (2007) and Takahashi *et al.* (2007) reported that c-myc was unnecessary for generation of human iPS cells. Usage of the "myc" family of genes in induction of iPS cells is troubling for the eventuality of iPS cells as clinical therapies, as 25% of mice transplanted with c-myc-induced iPS cells developed lethal teratomas. N-myc and L-myc have been identified to induce pluripotency instead of C-myc with similar efficiency. In certain aspects, Myc mutants, variants, homologs, or derivatives may be used, such as mutants that have reduced transformation of cells. Examples include LMYC (NM_001033081), MYC with 41 amino acids deleted at the N-terminus (dN2MYC), or MYC with mutation at amino acid position 136 (*e.g.,* W136E).

### VI. Cellular Signaling Inhibitors

In certain aspects of the invention, during at least part of the reprogramming process, the cell may be maintained in the presence of one or more signaling inhibitors which inhibit a signal transducer involved in a signaling cascade, *e.g.,* in the presence of a MEK inhibitor, a GSK3 inhibitor, a TGF-β receptor inhibitor, both a MEK inhibitor and a GSK3 inhibitor, both a GSK3 inhibitor and a TGF-β receptor inhibitor, both a MEK inhibitor and a TGF-β receptor inhibitor, a combination of all three inhibitors, or inhibitor of other signal transducers within these same pathways. In certain aspects, ROCK inhibitors, such as HA-100 and H-1152, or Myosin II inhibitor, such as blebbistatin, may be used to facilitate clonal expansion of reprogrammed cells and resulting iPS cells. High concentration of FGF, in combination with specific reprogramming medium such as conditioned human ES cell culture medium or a chemically defined medium such as serum-free defined N2B27 medium, TeSR medium or Essential 8 medium may also be used to increase reprogramming efficiency.

In certain embodiments, in addition to introducing the cells with one or more reprogramming factors (*e.g.* two, three or more, as described herein) by extra-chromosome genetic elements, the cells are treated with a reprogramming medium comprising: a MEK inhibitor, a TGF-β receptor inhibitor, a GSK3 inhibitor, and optionally LIF, with the advantages such as improving reprogramming efficiency and kinetics and facilitating iPS cell identification in the primary reprogramming culture, thus preserving iPS cell clonality.

It will be understood that in these aspects and embodiments, other signaling inhibitors which inhibit a signaling component of the same signaling pathway (*e.g.* ERK1 or ERK2 cascade) may be substituted where desired for the MEK inhibitor. This may include inhibition of an upstream stimulus of the MAPK pathway, in particular through the FGF receptor (Ying, 2008). Likewise, the GSK3 inhibitor may be substituted where desired for other inhibitors of GSK3-related signaling pathways, such as insulin synthesis and Wnt/β-catenin signaling; the LIF may be substituted where desired for other activators of Stat3 or gp130 signaling.

Such a signaling inhibitor, *e.g.,* a MEK inhibitor, a GSK3 inhibitor, a TGF-β receptor inhibitor, may be used at an effective concentration of at least or about 0.02, 0.05, 0.1, 0.2, 0.5, 1, 2, 3, 4, 5, 10, 15, 20, 25, 30, 35, 40, 45, 50, 100, 150, 200, 500 to about 1000 µM, or any range derivable therein.

Inhibitors may be provided or obtained by those skilled in the art by conventional means or from conventional sources (see also WO2007113505).

### A. Glycogen synthase kinase 3 inhibitor

Glycogen synthase kinase 3 (GSK-3) is a serine/threonine protein kinase that mediates the addition of phosphate molecules on certain serine and threonine amino acids in particular cellular substrates. The phosphorylation of these other proteins by GSK-3 usually inhibits the target protein (also called the "substrate"). As mentioned, GSK-3 is known for phosphorylating and thus inactivating glycogen synthase. It has also been implicated in the control of cellular response to damaged DNA and Wnt signaling. GSK-3 also phosphorylates Ci in the Hedgehog (Hh) pathway, targeting it for proteolysis to an inactive form. In addition to glycogen synthase, GSK-3 has many other substrates. However, GSK-3 is unusual among the kinases in that it usually requires a "priming kinase" to first phosphorylate a substrate.

The consequence of GSK-3 phosphorylation is usually inhibition of the substrate. For example, when GSK-3 phosphorylates another of its substrates, the NFAT family of transcription factors, these transcription factors can not translocate to the nucleus and are therefore inhibited. In addition to its important role in the Wnt signaling pathway, which is required for establishing tissue patterning during development, GSK-3 is also critical for the protein synthesis that is induced in settings such as skeletal muscle hypertrophy. Its roles as an NFAT kinase also places it as a key regulator of both differentiation and cellular proliferation.

GSK3 inhibition may refer to inhibition of one or more GSK3 enzymes. The family of GSK3 enzymes is well-known and a number of variants have been described (see *e.g.* Schaffer *et al.,* 2003). In specific embodiments GSK3-β is inhibited. GSK3-α inhibitors are also suitable, and in certain aspects inhibitors for use in the invention inhibit both GSK3-α and GSK3-β.

Inhibitors of GSK3 can include antibodies that bind, dominant negative variants of, and siRNA and antisense nucleic acids that target GSK3. Examples of GSK3 inhibitors are described in Bennett *et al.* (2002) and in Ring *et al.* (2003).

Specific examples of GSK3 inhibitors include, but are not limited to, Kenpaullone, 1-Azakenpaullone, CHIR99021, CHIR98014, AR-A014418 (see, *e.g.,* Gould *et al.,* 2004), CT 99021 (see, *e.g.,* Wagman, 2004), CT 20026 (see, Wagman, *supra*), SB415286, SB216763 (see, *e.g.,* Martin *et al.,* 2005), AR-A014418 (see, *e.g.,* Noble *et al.,* 2005), lithium (see, *e.g.,* Gould *et al.,* 2003), SB 415286 (see, *e.g.,* Frame *et al.,* 2001) and TDZD-8 (see, *e.g.,* Chin *et al.,* 2005). Further exemplary GSK3 inhibitors available from Calbiochem (see, *e.g.,* Dalton et al., WO2008/094597), include but are not limited to BIO (2'Z,3'£)-6-Bromomdirubm-3'-oxime (GSK3 Inhibitor IX); BIO-Acetoxime (2'Z,3'E)-6-Bromoindirubin-3'-acetoxime (GSK3 Inhibitor X); (5-Methyl-1H-pyrazol-3-yl)-(2-phenylquinazolin-4-yl)amine (GSK3-Inhibitor XIII); Pyridocarbazole-cyclopenadienylruthenium complex (GSK3 Inhibitor XV); TDZD-8 4-Benzyl-2-methyl-1,2,4-thiadiazolidine-3,5-dione (GSK3beta Inhibitor I); 2-Thio(3-iodobenzyl)-5-(1-pyridyl)-[1,3,4]-oxadiazole (GSK3beta Inhibitor II); OTDZT 2,4-Dibenzyl-5-oxothiadiazolidine-3-thione (GSK3beta Inhibitor III); alpha-4-Dibromoacetophenone (GSK3beta Inhibitor VII); AR-AO 14418 N-(4-Methoxybenzyl)-N'-(5-nitro-1,3-thiazol-2-yl)urea (GSK-3beta Inhibitor VIII); 3-(1-(3-Hydroxypropyl)-1H-pyrrolo[2,3-b]pyridin-3-yl]-4-pyrazin-2-yl-pyrrole-2,5-dione (GSK-3beta Inhibitor XI); TWSl 19 pyrrolopyrimidine compound (GSK3beta Inhibitor XII); L803 H-KEAPP APPQSpP-NH2 or its Myristoylated form (GSK3beta Inhibitor XIII); 2-Chloro-1-(4,5-dibromo-thiophen-2-yl)-ethanone (GSK3beta Inhibitor VI); AR-AO144-18; SB216763; and SB415286.

GSK3 inhibitors can activate, for example, the Wnt/β-catenin pathway. Many of β-catenin downstream genes co-regulate pluripotency gene networks. For example, a GSK inhibitor activates cMyc expression as well as enhances its protein stability and transcriptional activity. Thus, in some embodiments, GSK3 inhibitors can be used to stimulate endogenous Myc polypeptide expression in a cell, thereby eliminating the need for Myc expression to induce pluripotency.

In addition, the structure of the active site of GSK3-β has been characterized and key residues that interact with specific and non-specific inhibitors have been identified (Bertrand *et al.,* 2003). This structural characterization allows additional GSK inhibitors to be readily identified.

The inhibitors used herein are preferably specific for the kinase to be targeted. The inhibitors of certain embodiments are specific for GSK3-β and GSK3-α, substantially do not inhibit erk2 and substantially do not inhibit cdc2. Preferably the inhibitors have at least 100 fold, more preferably at least 200 fold, very preferably at least 400 fold selectivity for human GSK3 over mouse erk2 and/or human cdc2, measured as ratio of IC₅₀ values; here, reference to GSK3 IC₅₀ values refers to the mean values for human GSK3-β and GSK3-α. Good results have been obtained with CHIR99021 which is specific for GSK3. Suitable concentrations for use of CHIR99021 are in the range 0.01 to 100, preferably 0.1 to 20, more preferably 0.3 to 10 micromolar.

### B. MEK inhibitor

MEK inhibitors, which include inhibitors of mitogen-activated protein kinase kinase (MAPK/ERK kinase or MEK) or its related signaling pathways like MAPK cascade, may be used in certain aspects of the invention. Mitogen-activated protein kinase kinase (sic) is a kinase enzyme which phosphorylates mitogen-activated protein kinase. It is also known as MAP2K. Extracellular stimuli lead to activation of a MAP kinase via a signaling cascade ("MAPK cascade") composed of MAP kinase, MAP kinase kinase (MEK, MKK, MEKK, or MAP2K), and MAP kinase kinase kinase (MKKK or MAP3K).

A MEK inhibitor herein refers to MEK inhibitors in general. Thus, a MEK inhibitor refers to any inhibitor of a member of the MEK family of protein kinases, including MEK1, MEK2 and MEK5. Reference is also made to MEK1 , MEK2 and MEK5 inhibitors. Examples of suitable MEK inhibitors, already known in the art, include the MEK1 inhibitors PD184352 and PD98059, inhibitors of MEK1 and MEK2 U0126 and SL327, and those discussed in Davies *et al.* (2000).

In particular, PD184352 and PD0325901 have been found to have a high degree of specificity and potency when compared to other known MEK inhibitors (Bain *et al.,* 2007). Other MEK inhibitors and classes of MEK inhibitors are described in Zhang *et al.* (2000).

Inhibitors of MEK can include antibodies to, dominant negative variants of, and siRNA and antisense nucleic acids that suppress expression of MEK. Specific examples of MEK inhibitors include, but are not limited to, PD0325901 (see, *e.g.,* Rinehart *et al.,* 2004), PD98059 (available, *e.g.,* from Cell Signaling Technology), U0126 (available, for example, from Cell Signaling Technology), SL327 (available, *e.g.,* from Sigma-Aldrich), ARRY- 162 (available, *e.g.,* from Array Biopharma), PD184161 (see, *e.g.,* Klein *et al.,* 2006), PD184352 (CI- 1040) (see, *e.g.,* Mattingly *et al.,* 2006), sunitinib (see, *e.g.,* Voss, et al., US2008004287), sorafenib (see, Voss *supra*), Vandetanib (see, Voss *supra*), pazopanib (see, *e.g.,* Voss *supra*), Axitinib (see, Voss *supra*) and PTK787 (see, Voss *supra*).

Currently, several MEK inhibitors are undergoing clinical trial evaluations. CI-1040 has been evaluate in Phase I and II clinical trials for cancer (see, *e.g.,* Rinehart *et al.,* 2004). Other MEK inhibitors being evaluated in clinical trials include PD 184352 (see, *e.g.,* English *et al.,* 2002), BAY 43-9006 (see, *e.g.,* Chow *et al.,* 2001), PD-325901 (also PD0325901), GSK1 120212, ARRY-438162, RDEA1 19, AZD6244 (also ARRY-142886 or ARRY-886), RO5126766, XL518 and AZD8330 (also ARRY-704).

Inhibition of MEKs can also be conveniently achieved using RNA-mediated interference (RNAi). Typically, a double-stranded RNA molecule complementary to all or part of a MEK gene is introduced into pluripotent cells, thus promoting specific degradation of MEK-encoding mRNA molecules. This post-transcriptional mechanism results in reduced or abolished expression of the targeted MEK gene. Suitable techniques and protocols for achieving MEK inhibition using RNAi are known.

A number of assays for identifying kinase inhibitors, including GSK3 inhibitors and MEK inhibitors, are known. For example, Davies *et al.* (2000) describes kinase assays in which a kinase is incubated in the presence of a peptide substrate and radiolabeled ATP. Phosphorylation of the substrate by the kinase results in incorporation of the label into the substrate. Aliquots of each reaction are immobilized on phosphocellulose paper and washed in phosphoric acid to remove free ATP. The activity of the substrate following incubation is then measured and provides an indication of kinase activity. The relative kinase activity in the presence and absence of candidate kinase inhibitors can be readily determined using such an assay. Downey *et al.* (1996) also describes assays for kinase activity which can be used to identify kinase inhibitors.

### C. TGF-β receptor inhibitor

TGF-β receptor inhibitors may include any inhibitors of TGF signaling in general or inhibitors specific for TGF-β receptor (*e.g.,* ALK5) inhibitors, which can include antibodies to, dominant negative variants of, and siRNA and antisense nucleic acids that suppress expression of, TGF beta receptors (*e.g.,* ALK5). Exemplary TGFβ receptor/ALK5 inhibitors include, but are not limited to, SB431542 (see, *e.g.,* Inman *et al.,* 2002), A-83-01, also known as 3-(6-Methyl-2-pyridinyl)-N-phenyl-4-(4-quinolinyl)-1H-pyrazole-1-carbothioamide (see, *e.g.,* Tojo *et al.,* 2005, and commercially available from, *e.g.,* Toicris Bioscience); 2-(3-(6-Methylpyridin-2-yl)-1H-pyrazol-4-yl)-1,5-naphthyridine, Wnt3a/BIO (see, *e.g.,* Dalton, et al., WO2008/094597), BMP4 (see, Dalton, *supra*), GW788388 (- (4-[3-(pyridin-2-yl)-1H-pyrazol-4-yl]pyridm-2-yl}-N-(tetrahydro-2H-pyran-4-yl)benzamide) (see, *e.g.,* Gellibert *et al.,* 2006), SM16 (see, *e.g.,* Suzuki *et al.,* 2007), IN-1130 (3-((5-(6-methylpyridin-2-yl)-4-(quinoxalin-6-yl)-1H-imidazol-2-yl)methyl)benzamide) (see, *e.g.,* Kim *et al.,* 2008), GW6604 (2-phenyl-4-(3-pyridin-2-yl-1H-pyrazol-4-yl)pyridine) (.see, *e.g.,* de Gouville *et al.,* 2006), SB- 505124 (2-(5-benzo[1,3]dioxol-5-yl-2-tert-butyl-3H-imidazol-4-yl)-6-methylpyridine hydrochloride) (see, *e.g.,* DaCosta *et al.,* 2004) and pyrimidine derivatives (see, *e.g.,* those listed in Stiefl et al., WO2008/006583).

Further, while an "ALK5 inhibitor" is not intended to encompass non-specific kinase inhibitors, an "ALK5 inhibitor" should be understood to encompass inhibitors that inhibit ALK4 and/or ALK7 in addition to ALK5, such as, for example, SB-431542 (see, *e.g.,* Inman *et al.,* 2002). Without intending to limit the scope of the invention, it is believed that ALK5 inhibitors affect the mesenchymal to epithelial conversion/transition (MET) process. TGFβ/activin pathway is a driver for epithelial to mesenchymal transition (EMT). The inventors contemplate that inhibiting the TGFβ/activin pathway can facilitate MET (*i.e.,* reprogramming) process.

It is believed that inhibition of the TGFβ/activin pathway will have similar effects. Thus, any inhibitor (*e.g.,* upstream or downstream) of the TGFβ/activin pathway can be used in combination with, or instead of, TGF-β/ALK5 inhibitors as described herein. Exemplary TGFβ/activin pathway inhibitors include but are not limited to: TGF beta receptor inhibitors, inhibitors of SMAD 2/3 phosphorylation, inhibitors of the interaction of SMAD 2/3 and SMAD 4, and activators/agonists of SMAD 6 and SMAD 7. Furthermore, the categorizations described herein are merely for organizational purposes and one of skill in the art would know that compounds can affect one or more points within a pathway, and thus compounds may function in more than one of the defined categories.

TGF beta receptor inhibitors can include antibodies to, dominant negative variants of, and siRNA or antisense nucleic acids that target TGF beta receptors. Specific examples of inhibitors include but are not limited to SU5416; 2-(5-benzo[1,3]dioxol-5-yl-2-tert-butyl-3H-imidazol-4-yl)-6-methylpyridine hydrochloride (SB-505124); lerdelimumb (CAT- 152); metelimumab (CAT-192); GC-1008; IDl 1; AP-12009; AP-11014; LY550410; LY580276; LY364947; LY2109761; SB-505124; SB-431542; SD-208; SM16; NPC-30345; Ki26894; SB-203580; SD-093; Gleevec; 3,5,7,2',4'-pentahydroxyfiavone (Morin); activin-M108A; P144; soluble TBR2-Fc; and antisense transfected tumor cells that target TGF beta receptors (See, *e.g.,* Wrzesinski *et al.,* 2007; Kaminska *et al.,* 2005; and Chang *et al.,* 2007).

### D. ROCK inhibitors and Myosin II ATPase Inhibitors

Pluripotent stem cells, especially human ES cells and iPS cells, are vulnerable to apoptosis upon cellular detachment and dissociation, which are important for clonal isolation or expansion and differentiation induction. Recently, a small class of molecules have been found to increase clonal efficiency and survival of dissociated pluripotent stem cells, such as Rho-associated kinase (ROCK) inhibitors, which are inhibitors for ROCK-related signaling pathways, for example, Rho-specific inhibitors, ROCK-specific inhibitors or myosin II-specific inhibitors. In certain aspects of the invention, ROCK inhibitors may be used for culturing and passaging of pluripotent stem cells and/or differentiation of the stem cells. Therefore, ROCK inhibitors could be present in any cell culture medium in which pluripotent stem cells grow, dissociate, form aggregates, or undergo differentiation, such as an adherent culture or suspension culture. Unless otherwise stated herein, myosin II inhibitors, such as blebbistatin, can substitute for the experimental use of ROCK inhibitors.

ROCK signaling pathways may include Rho family GTPases; ROCK, a major effector kinase downstream of Rho; Myosin II, the predominant effector downstream of ROCK (Harb *et al.,* 2008); and any intermediate, upstream, or downstream signal processors. ROCK may phosphorylate and inactivate myosin phosphatase target subunit 1 (MYPT1), one of the major downstream targets of ROCK that negatively regulates myosin function through dephosphorylation of myosin regulatory light chain (MRLC).

ROCKs are serine/threonine kinases that serve as a target proteins for Rho (of which three isoforms exist--RhoA, RhoB and RhoC). Theses kinases were initially characterized as mediators of the formation of RhoA-induced stress fibers and focal adhesions. The two ROCK isoforms ROCK1 (p160ROCK, also called ROKβ) and ROCK2 (ROKα) are comprised of a N-terminal kinase domain, followed by a coiled-coil domain containing a Rho-binding domain and a pleckstrin-homology domain (PH). Both ROCKs are cytoskeletal regulators, mediating RhoA effects on stress fiber formation, smooth muscle contraction, cell adhesion, membrane ruffling and cell motility. ROCKs may exert their biological activity by targeting downstream molecules, such as myosin II, myosin light chain (MLC), MLC phosphatase (MLCP) and the phosphatase and tensin homolog (PTEN).

Non-limiting examples of ROCK inhibitors include HA-100, Y-27632, H-1152, Fasudil (also referred to as HA1077), Y-30141 (described in U.S. Patent 5,478,838), Wf-536, HA-1077, hydroxyl-HA-1077, GSK269962A, SB-772077-B, and derivatives thereof, and antisense nucleic acid for ROCK, RNA interference inducing nucleic acid (for example, siRNA), competitive peptides, antagonist peptides, inhibitory antibodies, antibody-ScFV fragments, dominant negative variants and expression vectors thereof. Further, since other low molecular compounds are known as ROCK inhibitors, such compounds or derivatives thereof can be also used in embodiments (for example, refer to U.S. Patent Publication Nos. 20050209261, 20050192304, 20040014755, 20040002508, 20040002507, 20030125344 and 20030087919, and International Patent Publication Nos. 2003/062227, 2003/059913, 2003/062225, 2002/076976 and 2004/039796). In certain aspects of the present invention, a combination of one or two or more of the ROCK inhibitors can also be used.

Rho-specific inhibitors, such as Clostridium botulinum C3 exoenzyme, and/or Myosin II-specific inhibitors may also be used as a ROCK inhibitor in certain aspects of the invention.

### VII. Culturing of Reprogrammed Cells

The starting cell and the end, reprogrammed cell generally have differing requirements for culture medium and conditions. Likewise, when simultaneously selecting cells for integration of an engineering construct, a selective drug may be added to the culture medium during specific portions of the reprogramming process. To allow for this while also allowing that reprogramming of the cell is taking place, it is usual to carry out at least an initial stage of culture, after introduction of the reprogramming factors, in the presence of medium and under culture conditions known to be suitable for growth of the starting cell. However, this initial stage may also include a selection drug, such that only cells comprising a resistence marker proliferate during this initial growth phase.

This is followed by a subsequent period of culture in the presence of a reprogramming medium (in the absence or presence of a selection drug) and under conditions known to be suitable for pluripotent cells - on feeders with serum or use chemically-defined medium or feeder-free conditions. Suitable feeders (if used) include primary or immortalized fibroblast lines, typically inactivated so they do not overgrow the growth of the cells being reprogrammed. After a sufficient time for reprogramming, the reprogrammed cells may be further cultured for expansion of iPS cells either before or after selection of iPS cells in an expansion medium. Such an expansion medium may comprise one or more signaling inhibitors as described above or comprise a culture medium essentially free of these inhibitors.

The initial stage of culture is preferably for a period of up to 6 days, more preferably up to 4 days and in particular embodiments, described below for not more than 3 days, and more particularly up to or about one day. The subsequent stage of culture in reprogramming medium comprising one or more signaling inhibitors is suitably for a period of at least or about 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33 days, or any range derivable therein, and can be for a period of up to 120 days, preferably up to 10 days, or until detection of iPS cells and/or engineered iPS cells. In a specific example described below used to generate reprogrammed human cells, the initial stage of culture was for a period of about 1 day and the subsequent stage was for about 9 to 28 days by culture in a reprogramming condition the presence of a reprogramming medium comprising a MEK inhibitor, a TGF-β receptor inhibitor, and a GSK3 inhibitor. The reprogramming condition may be essentially free of feeder cells. In further aspects, the reprogramming medium may be chemically defined. To improve reprogramming, the reprogramming medium may further comprise high concentration of FGF and may be essentially free of TGFβ.

The combination of a MEK inhibitor, a TGF-β receptor inhibitor, and a GSK3 inhibitor may facilitate the reprogramming process, including increasing reprogramming efficiency and shortening reprogramming time. LIF is an example of an activator of gp130 signaling, another being IL-6 in combination with soluble IL-6 receptor, and promotes growth and survival of the cell as it is in the process of being reprogrammed. During reprogramming, cells may be cultured in the presence of LIF; using LIF may help reprogrammed cells in certain aspects of the present invention to improve cell survival and clonogenicity.

### A. Stem Cell Culture Conditions in General

The culturing conditions according to the present invention will be appropriately defined depending on the medium and stem cells used and in accordance with the present claims. The medium can be prepared using a medium used for culturing animal cells as its basal medium, such as any of TeSR, Essential 8 medium, BME, BGJb, CMRL 1066, Glasgow MEM, Improved MEM Zinc Option, IMDM, Medium 199, Eagle MEM, αMEM, DMEM, Ham, RPMI 1640, and Fischer's media, as well as any combinations thereof, but the medium is not particularly limited thereto as far as it can be used for culturing animal cells.

The medium may be a serum-containing or serum-free medium. The serum-free medium refers to media with no unprocessed or unpurified serum, and accordingly can include media with purified blood-derived components or animal tissue-derived components (such as growth factors). From the aspect of preventing contamination with heterogeneous animal-derived components, serum can be derived from the same animal as that of the stem cell(s).

The medium may contain or may not contain any alternatives to serum. The alternatives to serum can include materials which appropriately contain albumin (such as lipid-rich albumin, albumin substitutes such as recombinant albumin, plant starch, dextrans and protein hydrolysates), transferrin (or other iron transporters), fatty acids, insulin, collagen precursors, trace elements, 2-mercaptoethanol, 3'-thiolgiycerol, or equivalents thereto. The alternatives to serum can be prepared by the method disclosed in International Publication No. 98/30679, for example. Alternatively, any commercially available materials can be used for more convenience. The commercially available materials include knockout Serum Replacement (KSR), Chemically-defined Lipid concentrated (Gibco), and Glutamax (Gibco).

The medium may also contain fatty acids or lipids, amino acids (such as non-essential amino acids), vitamin(s), growth factors, cytokines, antioxidant substances, 2-mercaptoethanol, pyruvic acid, buffering agents, and inorganic salts. The concentration of 2-mercaptoethanol can be, for example, about 0.05 to 1.0 mM, and particularly about 0.1 to 0.5 mM, but the concentration is particularly not limited thereto as long as it is appropriate for culturing the stem cell(s).

A culture vessel used for culturing the stem cell(s) can include, but is particularly not limited to: flask, flask for tissue culture, dish, petri dish, dish for tissue culture, multi dish, micro plate, micro-well plate, multi plate, multi-well plate, micro slide, chamber slide, tube, tray, CellSTACK® Chambers, culture bag, and roller bottle, as long as it is capable of culturing the stem cells therein. The stem cells may be cultured in a volume of at least or about 0.2, 0.5, 1, 2, 5, 10, 20, 30, 40, 50 ml, 100 ml, 150 ml, 200 ml, 250 ml, 300 ml, 350 ml, 400 ml, 450 ml, 500 ml, 550 ml, 600 ml, 800 ml, 1000 ml, 1500 ml, or any range derivable therein, depending on the needs of the culture. In a certain embodiment, the culture vessel may be a bioreactor, which may refer to any device or system that supports a biologically active environment. The bioreactor may have a volume of at least or about 2, 4, 5, 6, 8, 10, 15, 20, 25, 50, 75, 100, 150, 200, 500 liters, 1, 2, 4, 6, 8, 10, 15 cubic meters, or any range derivable therein.

The culture vessel can be cellular adhesive or non-adhesive and selected depending on the purpose. The cellular adhesive culture vessel can be coated with any of substrates for cell adhesion such as extracellular matrix (ECM) to improve the adhesiveness of the vessel surface to the cells. The substrate for cell adhesion can be any material intended to attach stem cells or feeder cells (if used). The substrate for cell adhesion includes collagen, gelatin, poly-L-lysine, poly-D-lysine, vitronectin, laminin, fibronectin, and RetroNectin and mixtures thereof for example Matrigel™, and lysed cell membrane preparations (Klimanskaya *et al.,* 2005).

Other culturing conditions can be appropriately defined. For example, the culturing temperature can be about 30 to 40°C, for example, at least or about 31, 32, 33, 34, 35, 36, 37, 38, 39°C but particularly not limited to them. The CO₂ concentration can be about 1 to 10%, for example, about 2 to 5%, or any range derivable therein. The oxygen tension can be at least or about 1, 5, 8, 10, 20%, or any range derivable therein.

The methods described herein can be used for adhesion culture of stem cells, for example. In this case, the cells can be cultured in the presence of feeder cells. In the case where the feeder cells are used in the methods of the present invention, stromal cells such as fetal fibroblasts can be used as feeder cells (for example, refer to; Hogan et al., Manipulating the Mouse Embryo, A Laboratory Manual (1994); Gene Targeting, A Practical Approach (1993); Martin (1981); Evans and Kaufman (1981); Jainchill *et al.,* (1969); Nakano *et al.* (1996); Kodama *et al.* (1982); and International Publication Nos. 01/088100 and 2005/080554).

The methods described herein can also be used for a suspension culture of stem cells, including suspension culture on carriers (Fernandes *et al.,* 2004) or gel/biopolymer encapsulation (United States Publication 2007/0116680). The term suspension culture of the stem cells means that the stem cells are cultured under non-adherent condition with respect to the culture vessel or feeder cells (if used) in a medium. The suspension culture of stem cells includes a dissociation culture of stem cells and an aggregate suspension culture of stem cells. The term dissociation culture of stem cells means that suspended stem cells is cultured, and the dissociation culture of stem cells include those of single stem cell or those of small cell aggregates composed of a plurality of stem cells (for example, about 2 to 400 cells). When the aforementioned dissociation culture is continued, the cultured, dissociated cells form a larger aggregate of stem cells, and thereafter an aggregate suspension culture can be performed. The aggregate suspension culture includes an embryoid culture method (see Keller *et al.,* 1995), and a SFEB method (Watanabe *et al.,* 2005; International Publication No. 2005/123902).

### B. Culturing of pluripotent stem cells

Depending on culture conditions, pluripotent stem cells can produce colonies of differentiated cells or undifferentiated cells. The term "differentiate" means the progression of a cell down a developmental pathway. The term "differentiated" is a relative term describing a cell's progression down a developmental pathway in comparison with another cell. For example, a pluripotent cell can give rise to any cell of the body, while a more differentiated cell such as a hematopoetic cell will give rise to fewer cell types.

Cultures of pluripotent stem cells are described as "undifferentiated" when a substantial proportion of stem cells and their derivatives in the population display morphological characteristics of undifferentiated cells, clearly distinguishing them from differentiated cells of embryo or adult origin. Undifferentiated ES or iPS cells are recognized by those skilled in the art, and typically appear in the two dimensions of a microscopic view in colonies of cells with high nuclear/cytoplasmic ratios and prominent nucleoli. It is understood that colonies of undifferentiated cells can have neighboring cells that are differentiated.

ES cells can be maintained in an undifferentiated state by culturing the cells in the presence of serum and a feeder layer, typically mouse embryonic fibroblasts. Other methods for maintaining stem cells in an undifferentiated state are also known. For example, mouse ES cells can be maintained in an undifferentiated state by culturing in the presence of LIF without a feeder layer. However, unlike mouse ES cells, pre-existing human ES cells do not respond to LIF. Human ES cells can be maintained in an undifferentiated state by culturing ES cells on a feeder layer of fibroblasts in the presence of basic fibroblast growth factor (Amit *et al.,* 2000), or by culturing on a protein matrix, such as Matrigel™ or laminin, without a feeder layer and in the presence of fibroblast-conditioned medium plus basic fibroblast growth factor (Xu et al., 2001; U.S. Patent No. 6,833,269).

Methods for preparing and culturing ES cells can be found in standard textbooks and reviews in cell biology, tissue culture, and embryology, including teratocarcinomas and embryonic stem cells: A practical approach (1987); Guide to Techniques in Mouse Development (1993); Embryonic Stem Cell Differentiation in vitro (1993); Properties and uses of Embryonic Stem Cells: Prospects for Application to Human Biology and Gene Therapy (1998). Standard methods used in tissue culture generally are described in Animal Cell Culture (1987); Gene Transfer Vectors for Mammalian Cells (1987); and Current Protocols in Molecular Biology and Short Protocols in Molecular Biology (1987 & 1995).

After somatic cells are introduced or contacted with reprogramming factors, these cells may be cultured in a medium sufficient to maintain the pluripotency and the undifferentiated state. Culturing of induced pluripotent stem (iPS) cells generated in this invention can use various medium and techniques developed to culture primate pluripotent stem cells, more specially, embryonic stem cells, as described in U.S. Pat. Publication 20070238170 and U.S. Pat. Publication 20030211603, and U.S. Pat. Publication 20080171385. It is appreciated that additional methods for the culture and maintenance of pluripotent stem cells, as would be known to one of skill, may be used with the present invention.

Undefined conditions may be used; for example, pluripotent cells may be cultured on fibroblast feeder cells or a medium that has been exposed to fibroblast feeder cells in order to maintain the stem cells in an undifferentiated state.

Alternately, pluripotent cells may be cultured and maintained in an essentially undifferentiated state using defined, feeder-independent culture system, such as a TeSR medium (Ludwig *et al.,* 2006a; Ludwig *et al.,* 2006b) or Essential 8 medium. Feeder-independent culture systems and media may be used to culture and maintain pluripotent cells. These approaches allow derived human iPS cells as well as human embryonic stem cells to remain in an essentially undifferentiated state without the need for mouse fibroblast "feeder layers." As described herein, various modifications may be made to these methods in order to reduce costs as desired.

Various matrix components may be used in culturing and maintaining human pluripotent stem cells. For example, Matrigel™, collagen IV, fibronectin, laminin, and vitronectin in combination may be used to coat a culturing surface as a means of providing a solid support for pluripotent cell growth, as described in Ludwig *et al.* (2006a; 2006b), which are incorporated by reference in their entirety. Particularly, Matrigel™ may be used to provide a substrate for cell culture and maintenance of human pluripotent stem cells. Matrigel™ is a gelatinous protein mixture secreted by mouse tumor cells and is commercially available from BD Biosciences (New Jersey, USA). This mixture resembles the complex extracellular environment found in many tissues and is used by cell biologists as a substrate for cell culture.

### C. Cell Passaging

Certain aspects described herein can further involve a step of dissociating stem cells. Stem cell dissociation can be performed using any known procedures. These procedures include treatments with a chelating agent (such as EDTA), an enzyme (such as trypsin, collagenase), or the like, and operations such as mechanical dissociation (such as pipetting). The stem cell(s) can be treated with the ROCK inhibitor before and/or after dissociation. For example, the stem cell(s) can be treated only after dissociation.

In some further examples of pluripotent stem cell culturing, once a culture container is full, the colony may be split into aggregated cells or even single cells by any method suitable for dissociation, which cell are then placed into new culture containers for passaging. Cell passaging is a technique that enables to keep cells alive and growing under cultured conditions for extended periods of time. Cells usually would be passed when they are about 70%-100% confluent.

Single-cell dissociation of pluripotent stem cells followed by single cell passaging may be used in the present methods with several advantages, like facilitating cell expansion, cell sorting, and defined seeding for differentiation and enabling automatization of culture procedures and clonal expansion. For example, progeny cell clonally derivable from a single cell may be homogenous in genetic structure and/or synchronized in cell cycle, which may increase targeted differentiation. Exemplary methods for single cell passaging may be as described in U.S. Pat. App. 20080171385.

Pluripotent stem cells may be dissociated into single individual cells, or a combination of single individual cells and small cell clusters comprising 2, 3, 4, 5, 6, 7, 8, 9, 10 cells or more. The dissociation may be achieved by mechanical force, or by a cell dissociation agent, such as NaCitrate, or an enzyme, for example, trypsin, trypsin-EDTA, TrypLE Select, or the like.

Based on the source of pluripotent stem cells and the need for expansion, the dissociated cells may be transferred individually or in small clusters to new culture containers in a splitting ratio such as at least or about 1:2, 1:4, 1:5, 1:6, 1:8, 1:10, 1:20, 1:40, 1:50, 1:100, 1:150, 1:200, or any range derivable therein. Suspension cell line split ratios may be done on volume of culture cell suspension. The passage interval may be at least or about every 1, 2, 3, 4, 5, 6, 7, 8, 9, 10, 11, 12, 13, 14, 15, 16, 17, 18, 19, 20 days or any range derivable therein. For example, the achievable split ratios for the different enzymatic passaging protocols may be 1:2 every 3-7 days, 1:3 every 4-7 days, and 1:5 to 1:10 approximately every 7 days, 1:50 to 1:100 every 7 days. When high split ratios are used, the passage interval may be extended to at least 12-14 days or any time period without cell loss due to excessive spontaneous differentiation or cell death.

In certain aspects, single cell passaging may be in the presence of a small molecule effective for increasing cloning efficiency and cell survival, such as a ROCK inhibitor as described above. Such a ROCK inhibitor, *e.g.,* Y-27632, HA-1077, H-1152, HA-100, or blebbistatin, may be used at an effective concentration, for example, at least or about 0.02, 0.05, 0.1, 0.2, 0.5, 1, 2, 3, 4, 5, 10, 15, 20, 25, 30, 35, 40, 45, 50 to about 100 µM, or any range derivable therein.

### VIII. Selection of iPS and engineered iPS cells

In certain aspects of the invention, after one or more extra-chromosomal genetic element and one or more nucleic acid molecule for integration are introduced into somatic cells, cells may be cultured for expansion (optionally selected for the presence of vector elements like positive selection or screenable marker) and these genetic elements will express reprogramming factors in these cells and replicate and partition along with cell division. These expressed reprogramming factors will reprogram somatic cell genome to establish a self-sustaining pluripotent state, and in the meantime or after removal of positive selection of the presence of vectors, exogenous genetic elements will be lost gradually. These induced pluripotent stem cells could be selected from progeny derived from these somatic cells based on embryonic stem cell characteristics because they are expected to be substantially identical to pluripotent embryonic stem cells. An additional negative selection step could be also employed to accelerate or help selection of iPS cells essentially free of extra-chromosomal genetic elements by testing the absence of reprogramming vector DNA or using selection markers.

### A. Selection for embryonic stem cell characteristics

The successfully generated iPSCs from previous studies were remarkably similar to naturally-isolated pluripotent stem cells (such as mouse and human embryonic stem cells, mESCs and hESCs, respectively) in the following respects, thus confirming the identity, authenticity, and pluripotency of iPSCs to naturally-isolated pluripotent stem cells. Thus, induced pluripotent stem cells generated from the methods disclosed in this invention could be selected based on one or more of following embryonic stem cell characteristics.

### i. Cellular biological properties

**Morphology:** iPSCs are morphologically similar to ESCs. Each cell may have round shape, large nucleolus and scant cytoplasm. Colonies of iPSCs could be also similar to that of ESCs. Human iPSCs form sharp-edged, flat, tightly-packed colonies similar to hESCs and mouse iPSCs form the colonies similar to mESCs, less flatter and more aggregated colonies than that of hESCs. In certain embodiments, the present method may generate large human iPS cells, which may have a diameter of at least or about 1.5, 1.6, 1.7, 1.8, 1.9, 2.0, 2.1, 2.2, 2.3, 2.4, 2.5 mm, or any range derivable therein, and be easily discernable from non-iPS cells.

**Growth properties:** Doubling time and mitotic activity are cornerstones of ESCs, as stem cells must self-renew as part of their definition. iPSCs could be mitotically active, actively self-renewing, proliferating, and dividing at a rate equal to ESCs.

**Stem Cell Markers:** iPSCs may express cell surface antigenic markers expressed on ESCs. Human iPSCs expressed the markers specific to hESC, including, but not limited to, SSEA-3, SSEA-4, TRA-1-60, TRA-1-81, TRA-2-49/6E, and Nanog. Mouse iPSCs expressed SSEA-1 but not SSEA-3 nor SSEA-4, similarly to mESCs.

**Stem Cell Genes:** iPSCs may express genes expressed in undifferentiated ESCs, including Oct-3/4, Sox2, Nanog, GDF3, REX1, FGF4, ESG1, DPPA2, DPPA4, and hTERT.

**Telomerase Activity:** Telomerases are necessary to sustain cell division unrestricted by the Hayflick limit of -50 cell divisions. Human ESCs express high telomerase activity to sustain self-renewal and proliferation, and iPSCs also demonstrate high telomerase activity and express hTERT (human telomerase reverse transcriptase), a necessary component in the telomerase protein complex.

**Pluripotency:** iPSCs will be capable of differentiation in a fashion similar to ESCs into fully differentiated tissues.

**Neural Differentiation:** iPSCs could be differentiated into neurons, expressing βIII-tubulin, tyrosine hydroxylase, AADC, DAT, ChAT, LMX1B, and MAP2. The presence of catecholamine-associated enzymes may indicate that iPSCs, like hESCs, may be differentiable into dopaminergic neurons. Stem cell-associated genes will be downregulated after differentiation.

**Cardiac Differentiation:** iPSCs could be differentiated into cardiomyocytes that spontaneously begin beating. Cardiomyocytes express TnTc, MEF2C, MYL2A, MYHCβ, TNNT2 and NKX2.5. Stem cell-associated genes will be downregulated after differentiation.

**Teratoma Formation:** iPSCs injected into immunodeficient mice may spontaneously formed teratomas after certain time, such as nine weeks. Teratomas are tumors of multiple lineages containing tissue derived from the three germ layers endoderm, mesoderm and ectoderm; this is unlike other tumors, which typically are of only one cell type. Teratoma formation is a landmark test for pluripotency.

**Embryoid Body:** Human ESCs in culture spontaneously form ball-like embryo-like structures termed "embryoid bodies," which consist of a core of mitotically active and differentiating hESCs and a periphery of fully differentiated cells from all three germ layers. iPSCs may also form embryoid bodies and have peripheral differentiated cells.

**Blastocyst Injection:** Human ESCs naturally reside within the inner cell mass (embryoblast) of blastocysts, and in the embryoblast, differentiate into the embryo while the blastocyst's shell (trophoblast) differentiates into extraembryonic tissues. The hollow trophoblast is unable to form a living embryo, and thus it is necessary for the embryonic stem cells within the embryoblast to differentiate and form the embryo. iPSCs injected by micropipette into a trophoblast to generate a blastocyst transferred to recipient females may result in chimeric living mouse pups: mice with iPSC derivatives incorporated all across their bodies with 10%-90 and chimerism.

### ii. Epigenetic reprogramming

**Promoter Demethylation:** Methylation is the transfer of a methyl group to a DNA base, typically the transfer of a methyl group to a cytosine molecule in a CpG site (adjacent cytosine/guanine sequence). Widespread methylation of a gene interferes with expression by preventing the activity of expression proteins or recruiting enzymes that interfere with expression. Thus, methylation of a gene effectively silences it by preventing transcription. Promoters of pluripotency-associated genes, including Oct-3/4, Rex1, and Nanog, may be demethylated in iPSCs, showing their promoter activity and the active promotion and expression of pluripotency-associated genes in iPSCs.

**Histone Demethylation:** Histones are compacting proteins that are structurally localized to DNA sequences that can effect their activity through various chromatin-related modifications. H3 histones associated with Oct-3/4, Sox2, and Nanog may be demethylated to activate the expression of Oct-3/4, Sox2, and Nanog.

### B. Selection for residue free feature

A reprogramming vector such as oriP-based vector could replicate extra-chromosomally and be lost from host cells after generations. However, an additional selection step for progeny cells essentially free of extra-chromosomal vector elements may facilitate this process. For example, a sample of progeny cell may be extracted to test the presence or loss of extra-chromosomal vector elements as known in the art (Leight and Sugden, 2001).

A reprogramming vector may further comprise a selection marker, more specifically, a negative selection marker, such as a gene encoding a thymidine kinase to select for progeny cells essentially free of such a selection marker. The human herpes simplex virus thymidine kinase type 1 gene (HSVtk) acts as a conditional lethal marker in mammalian cells. The HSVtk-encoded enzyme is able to phosphorylate certain nucleoside analogs (*e.g.,* ganciclovir, an antiherpetic drug), thus converting them to toxic DNA replication inhibitors. An alternative or a complementary approach is to test the absence of extra-chromosomal genetic elements in progeny cells, using conventional methods, such as RT-PCR, PCR, FISH (Fluorescent in situ hybridization), gene array, or hybridization (*e.g.,* Southern blot).

### IX. Nucleic Acid Molecule Construction and Delivery

Genetic elements comprising reprogramming factors and/or nucleic acid molecules for genome integration may be constructed to comprise functional genetic elements, such as elements for expression of a coding sequence. Details of components of these vectors and delivery methods are disclosed below.

### A. Regulatory Elements

Eukaryotic expression cassettes included in the vectors preferably contain (in a 5'-to-3' direction) an eukaryotic transcriptional promoter operably linked to a protein-coding sequence, splice signals including intervening sequences, and a transcriptional termination/polyadenylation sequence.

### i. Promoter/Enhancers

A "promoter" is a control sequence that is a region of a nucleic acid sequence at which initiation and rate of transcription are controlled. It may contain genetic elements at which regulatory proteins and molecules may bind, such as RNA polymerase and other transcription factors, to initiate the specific transcription a nucleic acid sequence. The phrases "operatively positioned," "operatively linked," "under control," and "under transcriptional control" mean that a promoter is in a correct functional location and/or orientation in relation to a nucleic acid sequence to control transcriptional initiation and/or expression of that sequence.

Promoters suitable for use in EBNA-1-encoding vector of the invention are those that direct the expression of the expression cassettes encoding the EBNA-1 protein to result in sufficient steady-state levels of EBNA-1 protein to stably maintain EBV oriP-containing vectors. Promoters may be also used for efficient expression of expression cassettes encoding reprogramming factors.

A promoter generally comprises a sequence that functions to position the start site for RNA synthesis. The best known example of this is the TATA box, but in some promoters lacking a TATA box, such as, for example, the promoter for the mammalian terminal deoxynucleotidyl transferase gene and the promoter for the SV40 late genes, a discrete element overlying the start site itself to help fix the place of initiation. Additional promoter elements regulate the frequency of transcriptional initiation. Typically, these are located in the region 30-110 bp upstream of the start site, although a number of promoters have been shown to contain functional elements downstream of the start site as well. To bring a coding sequence "under the control of" a promoter, one positions the 5' end of the transcription initiation site of the transcriptional reading frame "downstream" of (*i.e.,* 3' of) the chosen promoter. The "upstream" promoter stimulates transcription of the DNA and promotes expression of the encoded RNA.

The spacing between promoter elements frequently is flexible, so that promoter function is preserved when elements are inverted or moved relative to one another. In the *tk* promoter, the spacing between promoter elements can be increased to 50 bp apart before activity begins to decline. Depending on the promoter, it appears that individual elements can function either cooperatively or independently to activate transcription. A promoter may or may not be used in conjunction with an "enhancer," which refers to a cis-acting regulatory sequence involved in the transcriptional activation of a nucleic acid sequence.

A promoter may be one naturally associated with a nucleic acid sequence, as may be obtained by isolating the 5' non-coding sequences located upstream of the coding segment and/or exon. Such a promoter can be referred to as "endogenous." Similarly, an enhancer may be one naturally associated with a nucleic acid sequence, located either downstream or upstream of that sequence. Alternatively, certain advantages will be gained by positioning the coding nucleic acid segment under the control of a recombinant or heterologous promoter, which refers to a promoter that is not normally associated with a nucleic acid sequence in its natural environment. A recombinant or heterologous enhancer refers also to an enhancer not normally associated with a nucleic acid sequence in its natural environment. Such promoters or enhancers may include promoters or enhancers of other genes, and promoters or enhancers isolated from any other virus, or prokaryotic or eukaryotic cell, and promoters or enhancers not "naturally occurring," *i.e.,* containing different elements of different transcriptional regulatory regions, and/or mutations that alter expression. For example, promoters that are most commonly used in recombinant DNA construction include the β-lactamase (penicillinase), lactose and tryptophan (trp) promoter systems. In addition to producing nucleic acid sequences of promoters and enhancers synthetically, sequences may be produced using recombinant cloning and/or nucleic acid amplification technology, including PCR™, in connection with the compositions disclosed herein (see U.S. Patent Nos. 4,683,202 and 5,928,906). Furthermore, it is contemplated the control sequences that direct transcription and/or expression of sequences within non-nuclear organelles such as mitochondria, chloroplasts, and the like, can be employed as well.

Naturally, it will be important to employ a promoter and/or enhancer that effectively directs the expression of the DNA segment in the organelle, cell type, tissue, organ, or organism chosen for expression. Those of skill in the art of molecular biology generally know the use of promoters, enhancers, and cell type combinations for protein expression (see, for example Sambrook *et al.* 1989). The promoters employed may be constitutive, tissue-specific, inducible, and/or useful under the appropriate conditions to direct high level expression of the introduced DNA segment, such as is advantageous in the large-scale production of recombinant proteins and/or peptides. The promoter may be heterologous or endogenous.

Additionally any promoter/enhancer combination (as per, for example, the Eukaryotic Promoter Data Base EPDB, the World Wide Web at epd.isb-sib.ch/) could also be used to drive expression. Use of a T3, T7 or SP6 cytoplasmic expression system is another possible embodiment. Eukaryotic cells can support cytoplasmic transcription from certain bacterial promoters if the appropriate bacterial polymerase is provided, either as part of the delivery complex or as an additional genetic expression construct.

Non-limiting examples of promoters include early or late viral promoters, such as, SV40 early or late promoters, cytomegalovirus (CMV) immediate early promoters, Rous Sarcoma Virus (RSV) early promoters; eukaryotic cell promoters, such as, *e. g.,* beta actin promoter (Ng, 1989, Quitsche *et al.,* 1989), GADPH promoter (Alexander *et al.,* 1988, Ercolani *et al.,* 1988), metallothionein promoter (Karin *et al.,* 1989; Richards *et al.,* 1984); and concatenated response element promoters, such as cyclic AMP response element promoters (cre), serum response element promoter (sre), phorbol ester promoter (TPA) and response element promoters (tre) near a minimal TATA box. It is also possible to use human growth hormone promoter sequences (*e.g.,* the human growth hormone minimal promoter described at Genbank, accession no. X05244, nucleotide 283-341) or a mouse mammary tumor promoter (available from the ATCC, Cat. No. ATCC 45007). A specific example could be a phosphoglycerate kinase (PGK) promoter.

### ii. Initiation Signals and Internal Ribosome Binding Sites and Protease Cleavage Sites/Self-Cleaving Peptides

A specific initiation signal also may be required for efficient translation of coding sequences. These signals include the ATG initiation codon or adjacent sequences. Exogenous translational control signals, including the ATG initiation codon, may need to be provided. One of ordinary skill in the art would readily be capable of determining this and providing the necessary signals. It is well known that the initiation codon must be "in-frame" with the reading frame of the desired coding sequence to ensure translation of the entire insert. The exogenous translational control signals and initiation codons can be either natural or synthetic. The efficiency of expression may be enhanced by the inclusion of appropriate transcription enhancer elements.

The use of internal ribosome entry sites (IRES) elements may be used to create multigene, or polycistronic, messages. IRES elements are able to bypass the ribosome scanning model of 5' methylated Cap dependent translation and begin translation at internal sites (Pelletier and Sonenberg, 1988). IRES elements from two members of the picornavirus family (polio and encephalomyocarditis) have been described (Pelletier and Sonenberg, 1988), as well an IRES from a mammalian message (Macejak and Sarnow, 1991). IRES elements can be linked to heterologous open reading frames. Multiple open reading frames can be transcribed together, each separated by an IRES, creating polycistronic messages. By virtue of the IRES element, each open reading frame is accessible to ribosomes for efficient translation. Multiple genes can be efficiently expressed using a single promoter/enhancer to transcribe a single message (see U.S. Patent Nos. 5,925,565 and 5,935,819).

The genes encoding markers or other proteins may be connected to one another by a sequence (there may be more than one) coding for a protease cleavage site (*i.e.,* a sequence comprising the recognition site of a protease) or at least one self-cleaving peptide.

Suitable protease cleavages sites and self-cleaving peptides are known to the skilled person (see, *e.g.,* in Ryan *et al.,* 1997; Scymczak *et al.,* 2004). Preferred examples of protease cleavage sites are the cleavage sites of potyvirus NIa proteases (*e.g.,* tobacco *etc*h virus protease), potyvirus HC proteases, potyvirus PI (P35) prôteases, byovirus Nla proteases, byovirus RNA-2- encoded proteases, aphthovirus L proteases, enterovirus 2A proteases, rhinovirus 2A proteases, picorna 3C proteases, comovirus 24K proteases, nepovirus 24K proteases, RTSV (rice tungro spherical virus) 3Ciike protease, PY\IF (parsnip yellow fleck virus) 3C-like protease, thrombin, factor Xa and enterokinase.

Preferred self-cleaving peptides (also called "cis-acting hydrolytic elements", CHYSEL; see deFelipe (2002)) are derived from potyvirus and cardiovirus 2A peptides. Especially preferred self-cleaving peptides are selected from 2A peptides derived from FMDV (foot-and-mouth disease virus), equine rhinitis A virus, Thoseà asigna virus and porcine teschovirus.

### iii. Multiple Cloning Sites

Vectors can include a multiple cloning site (MCS), which is a nucleic acid region that contains multiple restriction enzyme sites, any of which can be used in conjunction with standard recombinant technology to digest the vector (see, for example, Carbonelli *et al.,* 1999, Levenson *et al.,* 1998, and Cocea, 1997.) "Restriction enzyme digestion" refers to catalytic cleavage of a nucleic acid molecule with an enzyme that functions only at specific locations in a nucleic acid molecule. Many of these restriction enzymes are commercially available. Use of such enzymes is widely understood by those of skill in the art. Frequently, a vector is linearized or fragmented using a restriction enzyme that cuts within the MCS to enable exogenous sequences to be ligated to the vector. "Ligation" refers to the process of forming phosphodiester bonds between two nucleic acid fragments, which may or may not be contiguous with each other. Techniques involving restriction enzymes and ligation reactions are well known to those of skill in the art of recombinant technology.

### iv. Splicing Sites

Most transcribed eukaryotic RNA molecules will undergo RNA splicing to remove introns from the primary transcripts. Vectors containing genomic eukaryotic sequences may require donor and/or acceptor splicing sites to ensure proper processing of the transcript for protein expression (see, for example, Chandler *et al.,* 1997.)

### v. Termination Signals

The vectors or constructs of the present invention will generally comprise at least one termination signal. A "termination signal" or "terminator" is comprised of the DNA sequences involved in specific termination of an RNA transcript by an RNA polymerase. Thus, in certain embodiments a termination signal that ends the production of an RNA transcript is contemplated. A terminator may be necessary *in vivo* to achieve desirable message levels.

In eukaryotic systems, the terminator region may also comprise specific DNA sequences that permit site-specific cleavage of the new transcript so as to expose a polyadenylation site. This signals a specialized endogenous polymerase to add a stretch of about 200 A residues (polyA) to the 3' end of the transcript. RNA molecules modified with this polyA tail appear to more stable and are translated more efficiently. Thus, in other embodiments involving eukaryotes, it is preferred that that terminator comprises a signal for the cleavage of the RNA, and it is more preferred that the terminator signal promotes polyadenylation of the message. The terminator and/or polyadenylation site elements can serve to enhance message levels and to minimize read through from the cassette into other sequences.

Terminators contemplated for use in the invention include any known terminator of transcription described herein or known to one of ordinary skill in the art, including but not limited to, for example, the termination sequences of genes, such as for example the bovine growth hormone terminator or viral termination sequences, such as for example the SV40 terminator. In certain embodiments, the termination signal may be a lack of transcribable or translatable sequence, such as due to a sequence truncation.

### vi. Polyadenylation Signals

In expression, particularly eukaryotic expression, one will typically include a polyadenylation signal to effect proper polyadenylation of the transcript. The nature of the polyadenylation signal is not believed to be crucial to the successful practice of the invention, and any such sequence may be employed. Preferred embodiments include the SV40 polyadenylation signal or the bovine growth hormone polyadenylation signal, convenient and known to function well in various target cells. Polyadenylation may increase the stability of the transcript or may facilitate cytoplasmic transport.

### vii. Origins of Replication

In order to propagate a vector in a host cell, it may contain one or more origins of replication sites (often termed "ori"), for example, a nucleic acid sequence corresponding to oriP of EBV as described above, which is a specific nucleic acid sequence at which replication is initiated. Alternatively a replication origin of other extra-chromosomally replicating virus as described above or an autonomously replicating sequence (ARS) can be employed.

### viii. Selection and Screenable Markers

In certain embodiments of the invention, cells containing a nucleic acid construct of the present invention may be identified or selected *in vitro* or *in vivo* by including a marker in the expression cassette. Such markers would confer an identifiable change to the cell permitting easy identification of cells containing the expression cassette. Generally, a selection marker is one that confers a property that allows for selection. A positive selection marker is one in which the presence of the marker allows for its selection, while a negative selection marker is one in which its presence prevents its selection. An example of a positive selection marker is a drug resistance marker.

Usually the inclusion of a drug selection marker aids in the cloning and identification of transformants, for example, genes that confer resistance to neomycin, puromycin, blastocidin, geneticin, hygromycin, DHFR, GPT, zeocin and histidinol are useful selection markers. In addition to markers conferring a phenotype that allows for the discrimination of transformants based on the implementation of conditions, other types of markers including screenable markers such as GFP, whose basis is colorimetric analysis, are also contemplated. Alternatively, screenable enzymes as negative selection markers such as herpes simplex virus thymidine kinase (*tk*) or chloramphenicol acetyltransferase (CAT) may be utilized. One of skill in the art would also know how to employ immunologic markers, possibly in conjunction with FACS analysis. The marker used is not believed to be important, so long as it is capable of being expressed simultaneously with the nucleic acid encoding a gene product. Further examples of selection and screenable markers are well known to one of skill in the art.

Certain embodiments of the present invention utilize screenable reporter genes to indicate specific property of cells, for example, differentiation along a defined cell lineage by activating a condition-responsive regulatory element which controls the reporter marker gene expression.

Examples of such reporters include genes encoding cell surface proteins (*e.g.,* CD4, HA epitope), fluorescent proteins, antigenic determinants and enzymes (*e.g.,* β-galactosidase or a nitroreductase). The vector containing cells may be isolated, *e.g.,* by FACS using fluorescently-tagged antibodies to the cell surface protein or substrates that can be converted to fluorescent products by a vector encoded enzyme. In certain aspects cell-permeable dyes can be used to identify cells expressing a reporter. For example, expression of a NFAT nitroreductase gene can be detected by using a cell permeable pro-fluorogenic substrate such as CytoCy5S (see, *e.g.,* U.S. Patent Nos. 5,633,158, 5,780,585, 5,977,065 and EP Patent No. EP 1252520).

The reporter gene may be a fluorescent protein. A broad range of fluorescent protein genetic variants have been developed that feature fluorescence emission spectral profiles spanning almost the entire visible light spectrum (see Table 1 for non-limiting examples). Mutagenesis efforts in the original Aequorea victoria jellyfish green fluorescent protein have resulted in new fluorescent probes that range in color from blue to yellow, and are some of the most widely used *in vivo* reporter molecules in biological research. Longer wavelength fluorescent proteins, emitting in the orange and red spectral regions, have been developed from the marine anemone, Discosoma striata, and reef corals belonging to the class Anthozoa. Still other species have been mined to produce similar proteins having cyan, green, yellow, orange, and deep red fluorescence emission. Developmental research efforts are ongoing to improve the brightness and stability of fluorescent proteins, thus improving their overall usefulness.

**Table 1: Fluorescent Protein Properties**

| Protein (Acronym) | Excitation Maximum (nm) | Emission Maximum (nm) | Molar Extinction Coefficient | Quantum Yield | *in vivo* Structure | Relative Brightness (% of EGFP) |
|---|---|---|---|---|---|---|
| GFP (wt) | 395/475 | 509 | 21,000 | 0.77 | Monomer* | 48 |

| Green Fluorescent Proteins | | | | | | |
|---|---|---|---|---|---|---|
| EGFP | 484 | 507 | 56,000 | 0.60 | Monomer* | 100 |
| AcGFP | 480 | 505 | 50,000 | 0.55 | Monomer* | 82 |
| TurboGFP | 482 | 502 | 70,000 | 0.53 | Monomer* | 110 |
| Emerald | 487 | 509 | 57,500 | 0.68 | Monomer* | 116 |
| Azami Green | 492 | 505 | 55,000 | 0.74 | Monomer | 121 |
| ZsGreen | 493 | 505 | 43,000 | 0.91 | Tetramer | 117 |

| Blue Fluorescent Proteins | | | | | | |
|---|---|---|---|---|---|---|
| EBFP | 383 | 445 | 29,000 | 0.31 | Monomer* | 27 |
| Sapphire | 399 | 511 | 29,000 | 0.64 | Monomer* | 55 |
| T-Sapphire | 399 | 511 | 44,000 | 0.60 | Monomer* | 79 |

| Cyan Fluorescent Proteins | | | | | | |
|---|---|---|---|---|---|---|
| ECFP | 439 | 476 | 32,500 | 0.40 | Monomer* | 39 |
| mCFP | 433 | 475 | 32,500 | 0.40 | Monomer | 39 |
| Cerulean | 433 | 475 | 43,000 | 0.62 | Monomer* | 79 |
| CyPet | 435 | 477 | 35,000 | 0.51 | Monomer* | 53 |
| AmCyan1 | 458 | 489 | 44,000 | 0.24 | Tetramer | 31 |
| Midon-Ishi Cyan | 472 | 495 | 27,300 | 0.90 | Dimer | 73 |
| mTFP1 (Teal) | 462 | 492 | 64,000 | 0.85 | Monomer | 162 |

| Yellow Fluorescent Proteins | | | | | | |
|---|---|---|---|---|---|---|
| EYFP | 514 | 527 | 83,400 | 0.61 | Monomer* | 151 |
| Topaz | 514 | 527 | 94,500 | 0.60 | Monomer* | 169 |
| Venus | 515 | 528 | 92,200 | 0.57 | Monomer* | 156 |
| mCitrine | 516 | 529 | 77,000 | 0.76 | Monomer | 174 |
| YPet | 517 | 530 | 104,000 | 0.77 | Monomer* | 238 |
| PhiYFP | 525 | 537 | 124,000 | 0.39 | Monomer* | 144 |
| ZsYellowl | 529 | 539 | 20,200 | 0.42 | Tetramer | 25 |
| mBanana | 540 | 553 | 6,000 | 0.7 | Monomer | 13 |

| Orange and Red Fluorescent Proteins | | | | | | |
|---|---|---|---|---|---|---|
| Kusabira Orange | 548 | 559 | 51,600 | 0.60 | Monomer | 92 |
| mOrange | 548 | 562 | 71,000 | 0.69 | Monomer | 146 |
| dTomato | 554 | 581 | 69,000 | 0.69 | Dimer | 142 |
| dTomato-Tandem | 554 | 581 | 138,000 | 0.69 | Monomer | 283 |
| DsRed | 558 | 583 | 75,000 | 0.79 | Tetramer | 176 |
| DsRed2 | 563 | 582 | 43,800 | 0.55 | Tetramer | 72 |
| DsRed-Express (T1) | 555 | 584 | 38,000 | 0.51 | Tetramer | 58 |
| DsRed-Monomer | 556 | 586 | 35,000 | 0.10 | Monomer | 10 |
| mTangerine | 568 | 585 | 38,000 | 0.30 | Monomer | 34 |
| mStrawberry | 574 | 596 | 90,000 | 0.29 | Monomer | 78 |
| AsRed2 | 576 | 592 | 56,200 | 0.05 | Tetramer | 8 |
| mRFP1 | 584 | 607 | 50,000 | 0.25 | Monomer | 37 |
| JRed | 584 | 610 | 44,000 | 0.20 | Dimer | 26 |
| mCherry | 587 | 610 | 72,000 | 0.22 | Monomer | 47 |
| HcRed1 | 588 | 618 | 20,000 | 0.015 | Dimer | 1 |
| mRaspberry | 598 | 625 | 86,000 | 0.15 | Monomer | 38 |
| HcRed-Tandem | 590 | 637 | 160,000 | 0.04 | Monomer | 19 |
| mPlum | 590 | 649 | 41,000 | 0.10 | Monomer | 12 |
| AQ143 | 595 | 655 | 90,000 | 0.04 | Tetramer | 11 |
| * Weak Dimer | | | | | | |

### B. Nucleic Acid Delivery

Introduction of a nucleic acid, be it a reprogramming vector or other molecule for genome integration, into somatic cells according to the current invention may use any suitable methods for nucleic acid delivery for transformation of a cell., as described herein or as would be known to one of ordinary skill in the art. Such methods include, but are not limited to, direct delivery of DNA such as by *ex vivo* transfection (Wilson *et al*., 1989, Nabel *et al.*, 1989); by injection (U.S. Patent Nos. 5,994,624, 5,981,274, 5,945,100, 5,780,448, 5,736,524, 5,702,932, 5,656,610, 5,589,466 and 5,580,859), including microinjection (Harland and Weintraub, 1985; U.S. Patent No. 5,789,215); by electroporation (U.S. Patent No. 5,384,253; Tur-Kaspa *et al.*, 1986; Potter *et al.*, 1984); by calcium phosphate precipitation (Graham and Van Der Eb, 1973; Chen and Okayama, 1987; Rippe *et al.*, 1990); by using DEAE-dextran followed by polyethylene glycol (Gopal, 1985); by direct sonic loading (Fechheimer *et al.*, 1987); by liposome mediated transfection (Nicolau and Sene, 1982; Fraley *et al.*, 1979; Nicolau *et al.*, 1987; Wong *et al.*, 1980; Kaneda *et al.*, 1989; Kato *et al.*, 1991) and receptor-mediated transfection (Wu and Wu, 1987; Wu and Wu, 1988); by microprojectile bombardment (PCT Application Nos. WO 94/09699 and 95/06128; U.S. Patent Nos. 5,610,042; 5,322,783 5,563,055, 5,550,318, 5,538,877 and 5,538,880); by agitation with silicon carbide fibers (Kaeppler et al., 1990; U.S. Patent Nos. 5,302,523 and 5,464,765); by desiccation/inhibition-mediated DNA uptake (Potrykus *et al.*, 1985), and any combination of such methods. Through the application of techniques such as these, organelle(s), cell(s), tissue(s) or organism(s) may be stably or transiently transformed.

### i. Liposome-Mediated Transfection

In a certain embodiment of the invention, a nucleic acid may be entrapped in a lipid complex such as, for example, a liposome. Liposomes are vesicular structures characterized by a phospholipid bilayer membrane and an inner aqueous medium. Multilamellar liposomes have multiple lipid layers separated by aqueous medium. They form spontaneously when phospholipids are suspended in an excess of aqueous solution. The lipid components undergo self-rearrangement before the formation of closed structures and entrap water and dissolved solutes between the lipid bilayers (Ghosh and Bachhawat, 1991). Also contemplated is a nucleic acid complexed with Lipofectamine (Gibco BRL) or Superfect (Qiagen). The amount of liposomes used may vary upon the nature of the liposome as well as the cell used, for example, about 5 to about 20 µg vector DNA per 1 to 10 million of cells may be contemplated.

Liposome-mediated nucleic acid delivery and expression of foreign DNA *in vitro* has been very successful (Nicolau and Sene, 1982; Fraley *et al.*, 1979; Nicolau *et al.*, 1987). The feasibility of liposome-mediated delivery and expression of foreign DNA in cultured chick embryo, HeLa and hepatoma cells has also been demonstrated (Wong *et al.*, 1980).

In certain embodiments of the invention, a liposome may be complexed with a hemagglutinating virus (HVJ). This has been shown to facilitate fusion with the cell membrane and promote cell entry of liposome-encapsulated DNA (Kaneda *et al.*, 1989). In other embodiments, a liposome may be complexed or employed in conjunction with nuclear non-histone chromosomal proteins (HMG-1) (Kato *et al.*, 1991). In yet further embodiments, a liposome may be complexed or employed in conjunction with both HVJ and HMG-1. In other embodiments, a delivery vehicle may comprise a ligand and a liposome.

### ii. Electroporation

In certain embodiments of the present invention, a nucleic acid is introduced into an organelle, a cell, a tissue or an organism *via* electroporation. One type of electroporation is nucleofection, in which nucleic acid is transferred to a cell through the use of a device called a Nucleofector and in combination with cell specific reagents (such as the Amaxa system; Lonza Cologne AG). Electroporation involves the exposure of a suspension of cells and DNA to a high-voltage electric discharge. Recipient cells can be made more susceptible to transformation by mechanical wounding. Also the amount of vectors used may vary upon the nature of the cells used, for example, about 5 to about 20 µg vector DNA per 1 to 10 million of cells may be contemplated.

Transfection of eukaryotic cells using electroporation has been quite successful. Mouse pre-B lymphocytes have been transfected with human kappa-immunoglobulin genes (Potter *et al.*, 1984), and rat hepatocytes have been transfected with the chloramphenicol acetyltransferase gene (Tur-Kaspa *et al.*, 1986) in this manner.

### iii. Calcium Phosphate

In other embodiments of the present invention, a nucleic acid is introduced to the cells using calcium phosphate precipitation. Human KB cells have been transfected with adenovirus 5 DNA (Graham and Van Der Eb, 1973) using this technique. Also in this manner, mouse L(A9), mouse C127, CHO, CV-1, BHK, NIH3T3 and HeLa cells were transfected with a neomycin marker gene (Chen and Okayama, 1987), and rat hepatocytes were transfected with a variety of marker genes (Rippe *et al.*, 1990).

### iv. DEAE-Dextran

In another embodiment, a nucleic acid is delivered into a cell using DEAE-dextran followed by polyethylene glycol. In this manner, reporter plasmids were introduced into mouse myeloma and erythroleukemia cells (Gopal, 1985).

### v. Sonication Loading

Additional embodiments of the present invention include the introduction of a nucleic acid by direct sonic loading. LTK⁻ fibroblasts have been transfected with the thymidine kinase gene by sonication loading (Fechheimer *et al.*, 1987).

### vi. Receptor Mediated Transfection

Still further, a nucleic acid may be delivered to a target cell via receptor-mediated delivery vehicles. These take advantage of the selective uptake of macromolecules by receptor-mediated endocytosis that will be occurring in a target cell. In view of the cell type-specific distribution of various receptors, this delivery method adds another degree of specificity to the present invention.

Certain receptor-mediated gene targeting vehicles comprise a cell receptor-specific ligand and a nucleic acid-binding agent. Others comprise a cell receptor-specific ligand to which the nucleic acid to be delivered has been operatively attached. Several ligands have been used for receptor-mediated gene transfer (Wu and Wu, 1987; Wagner *et al.*, 1990; Perales *et al.*, 1994; Myers, EPO 0273085), which establishes the operability of the technique. Specific delivery in the context of another mammalian cell type has been described (Wu and Wu, 1993). In certain aspects of the present invention, a ligand will be chosen to correspond to a receptor specifically expressed on the target cell population.

In other embodiments, a nucleic acid delivery vehicle component of a cell-specific nucleic acid targeting vehicle may comprise a specific binding ligand in combination with a liposome. The nucleic acid(s) to be delivered are housed within the liposome and the specific binding ligand is functionally incorporated into the liposome membrane. The liposome will thus specifically bind to the receptor(s) of a target cell and deliver the contents to a cell. Such systems have been shown to be functional using systems in which, for example, epidermal growth factor (EGF) is used in the receptor-mediated delivery of a nucleic acid to cells that exhibit upregulation of the EGF receptor.

In still further embodiments, the nucleic acid delivery vehicle component of a targeted delivery vehicle may be a liposome itself, which will preferably comprise one or more lipids or glycoproteins that direct cell-specific binding. For example, lactosyl-ceramide, a galactose-terminal asialganglioside, have been incorporated into liposomes and observed an increase in the uptake of the insulin gene by hepatocytes (Nicolau *et al.*, 1987). It is contemplated that the tissue-specific transforming constructs of the present invention can be specifically delivered into a target cell in a similar manner.

### vii Microprojectile Bombardment

Microprojectile bombardment techniques can be used to introduce a nucleic acid into at least one, organelle, cell, tissue or organism (U.S. Patent No. 5,550,318; U.S.

Patent No. 5,538,880; U.S. Patent No. 5,610,042; and PCT Application WO 94/09699). This method depends on the ability to accelerate DNA-coated microprojectiles to a high velocity allowing them to pierce cell membranes and enter cells without killing them (Klein *et al.*, 1987). There are a wide variety of microprojectile bombardment techniques known in the art, many of which are applicable to the invention.

In this microprojectile bombardment, one or more particles may be coated with at least one nucleic acid and delivered into cells by a propelling force. Several devices for accelerating small particles have been developed. One such device relies on a high voltage discharge to generate an electrical current, which in turn provides the motive force (Yang *et al.*, 1990). The microprojectiles used have consisted of biologically inert substances such as tungsten or gold particles or beads. Exemplary particles include those comprised of tungsten, platinum, and preferably, gold. It is contemplated that in some instances DNA precipitation onto metal particles would not be necessary for DNA delivery to a recipient cell using microprojectile bombardment. However, it is contemplated that particles may contain DNA rather than be coated with DNA. DNA-coated particles may increase the level of DNA delivery via particle bombardment but are not, in and of themselves, necessary.

For the bombardment, cells in suspension are concentrated on filters or solid culture medium. Alternatively, immature embryos or other target cells may be arranged on solid culture medium. The cells to be bombarded are positioned at an appropriate distance below the macroprojectile stopping plate.

### X. Examples

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice..

### Example 1 - Genome engineering and episomal reprogramming of human foreskin fibroblast cells using a Zinc Finger Nuclease

### Initial Cell Preparation

Live normal human neonatal dermal fibroblast (HNDF) cells were purchased from AllCells, LLC (Emeryville, CA., ID# NF090119, Cat# HN006002). Fibroblasts were cultured in Neonatal Human Dermal Fibroblast Medium with supplement (NHNDF basal medium + supplement) from AllCells, LLC. (Emeryville, CA., Cat# HN006006 and HN006007). Cells from a T75 flask were split 1:10 using a standard trypsin/EDTA method and some of the cells were plated in a 12 well plate to be used to generate a kill curve using puromycin. Six wells were seeded with a 1:5 dilution of cells and 6 wells were seeded with a 1:10 dilution of cells. Six vials of cells were frozen with approximately 1M cells per vial. Frozen cells were at passage 2.

### Engineering Cells

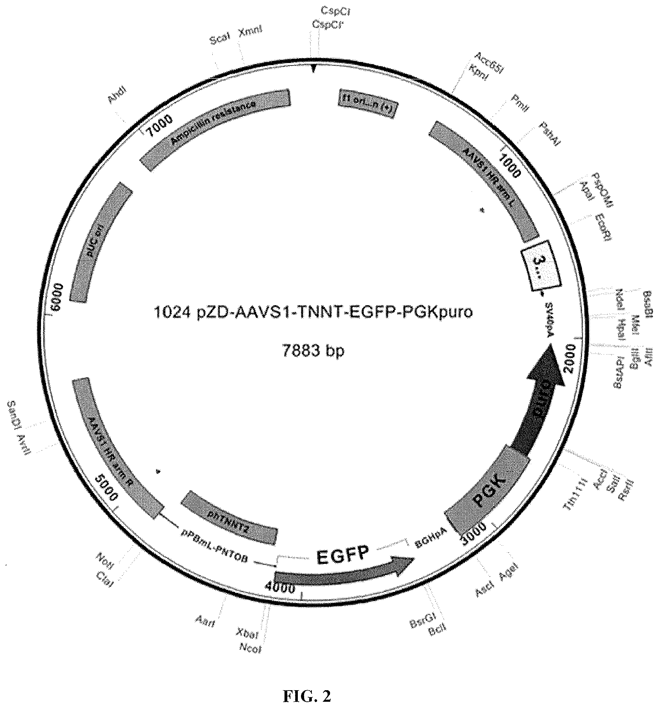

Six days post-split, confluent fibroblasts were harvested by Trypsin/EDTA dissociation. Cells were counted using a hemocytometer and four million cells were pelleted, washed twice with phosphate buffered saline, resuspended in 200 µl of Amaxa NHDF nucleofection solution from Lonza (Walkersville, MD., Cat# VPD-1001), and split into two 100µl tubes. 15 µg of plasmid 1024 (FIG. 2; which encodes a Troponin 2 promoter driving a green fluorescent protein and a constitutive PGK-Puromycin cassette cloned between homology arms for the AAVS1 gene locus; vector 1024) was added to 100 µl of the fibroblast solution. 5 µl of messenger RNA encoding a zinc finger nuclease that targets the AAVS1 locus (Sigma, St Louis, MO., Cat # CTI1-KT) was added and the mixture was transferred to an electroporation cuvette and nucleofected using program U-20 on an Amaxa Nucleofector II device from Lonza (Walkersville, MD). Cells were promptly resuspended in 14 ml of Neonatal Human Dermal Fibroblast Medium, 12 ml of which was plated in one 6-well plate and the remaining 2 ml was diluted into 10 ml of additional media and plated in a second 6-well plate. A second nucleofection was performed with the remaining 100 µl of fibroblasts by adding 5.5 µg of a plasmid constitutively expressing the green fluorescent protein gene, in the absence of zinc finger nuclease, to monitor nucleofection efficiency. After Zinc Finger Nuclease transfection, cells were maintained for 3 days before beginning drug selection (Day 0).

One well of the GFP transfected NHDF was exposed to 0.5 µg/ml puromycin in order to monitor the rate of cell death in puromycin negative NHDF. Four of five wells on the high density plate of the transfected AAVS1 HDNFs were exposed to 0.5 µg/ml puromycin. Medium was changed on Days 1, 3, and 4. On Day 5, medium was changed to non-selective medium. Puromycin selected wells were triturated to remove dead, adherent cells. All of the transfected GFP cells were removed, and all but approximately 15% of the transfected AAVS1 cells were removed. On Day 6, the NHDF transfected AAVS1 cells had begun to recover from selection and proliferate. The unselected NHDF transfected AAVS1 well was expanded to a 6-well plate. On Day 8, five wells of the unselected NHDF transfected AAVS1 cells were harvested and the genomic DNA was purified. One well was used for passage to a new 6-well plate. On Day 10, PCR was performed on the unselected NHDF transfected AAVS1 cells with primers to detected the integrated and wild-type AAVS1 locus. Cells constitutively expressing GFP were used as a positive control, and iPSC derived cardiomyocytes were used as a negative control. The following parameters and reagents were used for the PCR reaction: PCR: 98° 30 sec, [98° 10s, 68° 20s, 72° 60s] x35 cycles, 72° 2min. reaction components are shown in Table 2, below. PCR primers were:

Detects the targeted AAVS1 locus:
"MJM32" AAVS1-F1 - ACC ACT TTG AGC TCT ACT GGC TTC TG (SEQ ID NO: 1)
"MJM15" SV40pA-For - TGGACAAACCACAACTAGAATGCAG (SEQ ID NO: 2)

Detects the unintegrated AAVS1 locus
"MJM32" AAVS1-F1 - ACC ACT TTG AGC TCT ACT GGC TTC TG (SEQ ID NO: 3)
"MJM44" AAVS1-R1 - ACC CAA AAG GCA GCC TGG TAG AC (SEQ ID NO: 4)

**Table 2: PCR reaction components**

| PCR Reaction Component | Volume |
|---|---|
| 5x Phusion Buffer GC | 5ul |
| dNTPs | 0.5 |
| MJM32 primer | 1.25 |
| MJM15 primer | 1.25 |
| DNA (100ng - genomic DNA) | 1 |
| DMSO | 1 |
| Phu DNA polymerase | 0.125 |
| Sterile Water | 14.875 |

Agarose gel electrophoresis of PCR products showed the correct insertion of the recombinant plasmid at the AAVS1 site in the fibroblasts.

Media were also prepared for use after nucleofection. Components for N2B27 medium are shown in Table 3. In each case the components were combined and passed through a 0.22 µm filter.

**Table 3: N2B27 medium (500 ml).**

| **Components** | **Amount** | **Manufacturer** | **Part #** | **Lot #** | **Notes** |
|---|---|---|---|---|---|
| DMEM/F12 | 477.5 ml | Invitrogen | 11330-057 | 829387 | |
| N-2 supplement (100x) | 5.0 ml | Invitrogen | 17502-048 | 903988 | |
| B-27 supplement (50x) | 10 ml | Invitrogen | 0080085-SA | 907983 | |
| NEAA (100x) | 5 ml | Invitrogen | 11140-050 | 738001 | |
| Glutamax (100x) | 2.5 ml | Invitrogen | 35050-061 | 794979 | |
| β-ME | 3.4 µl | Sigma | M7522 | 105K01041 | |
| PD0325901 | 25 µl | Stemgent | 04-0006-2mg | 1768 | 10mM stock was made by diluting 2mg into 415µl DMSO |
| CHIR99021 | 150 µl | Stemgent | 04-0004-2mg | 2172 | 10mM stock was made by diluting 2mg into 430µl DMSO |
| A-83-01 | 25 µl | Stemgent | 04-0014-2mg | 2010 | 10mM stock was made by diluting 2mg into 475µl DMSO |
| hLIF | 500µl | Millipore | LIF1010 | DAM1770440 | |
| HA-100 | 500µl | Santa Cruz | Sc-203072 | J1410 | Diluted to 2.78mg/ml with water |
| zbFGF | 50µl | Promega | X608X | 28608702 | |

### Nucleofection of cells, reprogramming of engineered cells, and simultaneous engineering and reprogramming of wild type cells

The zinc finger nuclease modified NHDF (Normal Human Fetal Fibroblasts) from above were nucleofected with reprogramming DNA vectors.

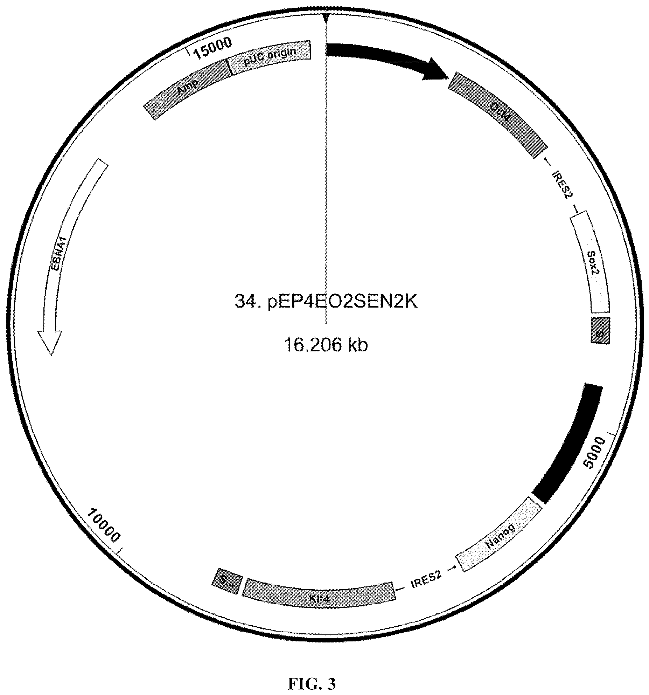

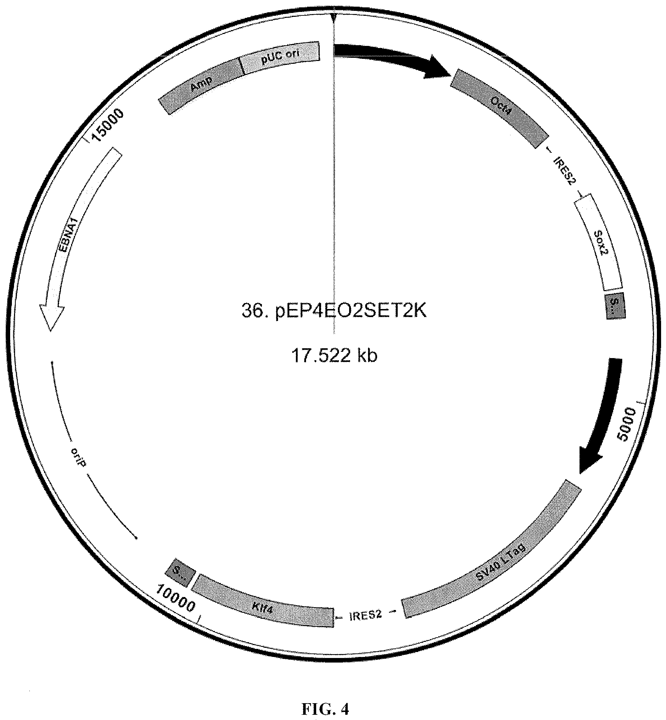

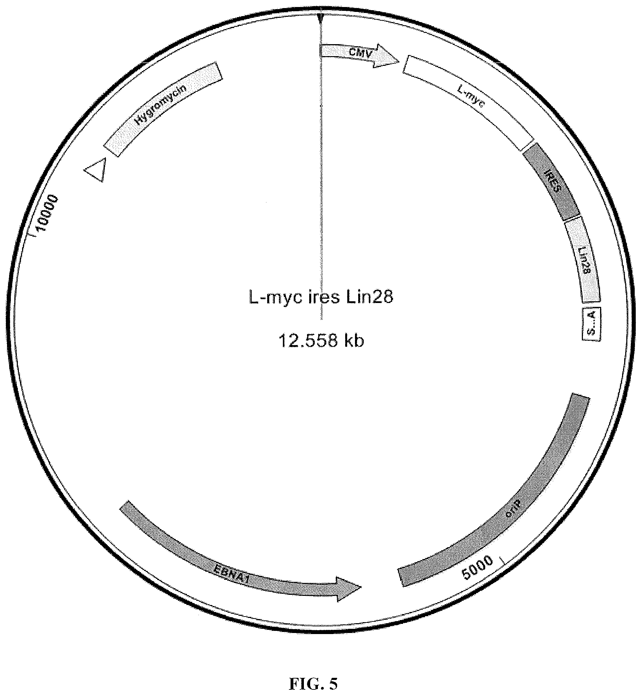

Briefly, three reprogramming vectors (#34 - FIG. 3, #36 -FIG. 4 & #123 - FIG. 5) were combined in two 1.5 mL tubes. Matrigel from four, 6-well plates was aspirated and replaced with 2 mL Recovery Medium (NHNDF basal medium plus supplement from AllCells with 4 ng/mL zbFGF and 10 µM HA-100). The fibroblasts were dissociated using trypsin, counted, and 1x10⁶ cells were resuspended in 100µl nucleofection buffer from the Amaxa NHDF Nucleofection kit (Lonza: VPD-001). The resuspended HNDF cells containing the AAVS1 insert were added to the reprogramming vectors and then transferred to a cuvette. The cells were electroporated using Amaxa program U-20. Warm recovery medium was added to the cuvette, and the contents of the cuvette were transferred to a 15 mL conical tube containing 6 mL Recovery Medium. The cells were distributed amongst two, 6-well plates.

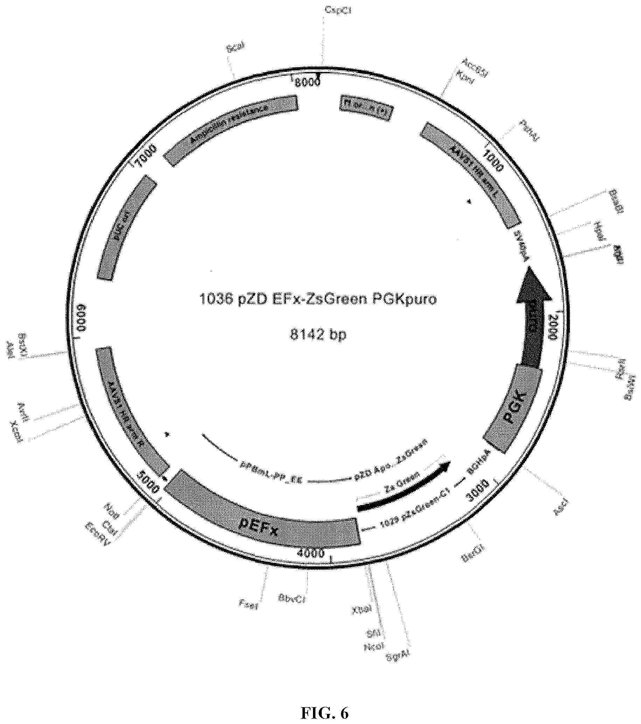

To the second 1.5 mL tube containing the reprogramming plasmids, 8.1 µg of plasmid 1036 was added (1036, see FIG. 6, comprises a constitutive EF1α promoter driving expression of ZsGreen fluorescent protein, a constitutive PGK-Puromycin cassette, both of which were cloned between homology arms for the AAVS1 gene locus). Wildtype HNDF cells (not previously targeted by the ZFN) were collected by trypsin dissociation, resuspended in 100 µL nucleofection solution, and added to the reprogramming plasmids and plasmid 1036. Messenger RNA encoding a zinc finger nuclease (5 µl) that targets the AAVS1 locus (Sigma, St Louis, MO., Cat# CTI1-KT) was added and the cells were nucleofected with Amaxa program U-20 and plated. Following plating, cells from sequential and simultaneous reprogramming/engineering experiments were maintained as follows:
Day 2 - cells were switched to reprogramming medium (see N2B27 - Table 5). Some were fluorescing.
Day 4 - cells were fed with reprogramming medium
Day 6 - cells were fed with reprogramming medium
Day 8 - cells were fed with reprogramming medium
Day 10 - cells were fed with reprogramming medium. Images were acquired.
Day 12 - cells were fed with reprogramming medium
Day 13 - cells were fed with TeSR
Day 15 - cells were fed with TeSR
Day 17 - cells were fed with TeSR
Day 20 - cells were fed with TeSR
Day 21 - performed live stain and pick colonies
Day 27 - picked P2 clones

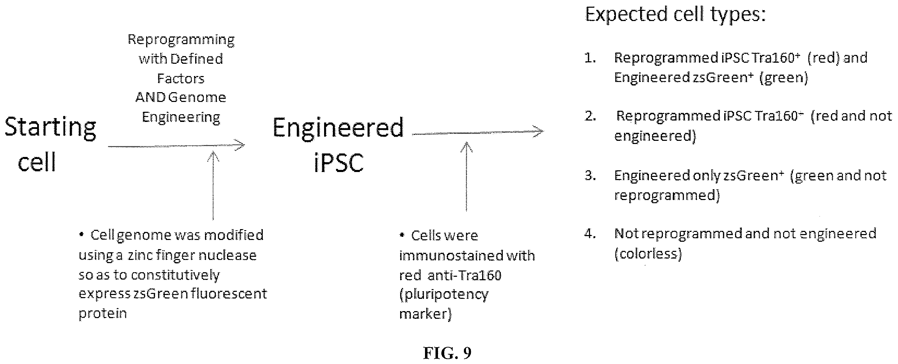

Results from the experiments with zinc finger nuclease mediated integration followed by cell reprogramming in fibroblasts are summarized below and all cellular phenotypes are described in FIG. 1 and FIG. 9:
Starting cells for sequential engineering then reprogramming: 4 million expanded primary human fibroblasts
   - Percentage of cells successfully engineered with drug resistance and selected:
Approximately 15%
   - Starting drug resistant expanded cells for Reprogramming: 2 million
   - Number of reprogrammed colonies (pluripotent) immunostaining positive (red) for Tra-160: >100
   - Number of unstained colonies (likely partially reprogrammed): Approximately 20

Results from the experiments with simultaneous genome engineering with zinc finger nuclease mediated integration and vectors containing reprogramming factors in fibroblasts are summarized below:
- Starting cells for simultaneous engineering and reprogramming: 2 million expanded primary human fibroblasts
- Number of early cell colonies positive for anti-Tra160 (red) and expressing green zsGFP: 14 (Note: After passage 1, there were 6 red and green colonies, 3 red only colonies and 5 colonies that did not survive picking.)
- Number of early cell colonies positive for anti-Tra160 (red only): approximately 40
- Number of green only cells: too numerous to count (These were likely engineered fibroblasts.)
- Number of unstained colonies: approximately 20 (These were likely partially reprogrammed colonies.)
- Number of colorless fibroblasts: too numerous to count (These were starting fibroblasts with no modifications.)

A method for episomal reprogramming and genome engineering as exemplified here is provided as FIG. 1, FIG 8, and FIG. 10.

### Example 2 - Episomal reprogramming and genome engineering of PBMCs using PiggyBac

Reagents were initially prepared for the experiments. A list of such reagents is provided in Table 4.

**Table 4: Regent list**

| **Material/reagent** | **Specification/concentration** | **Manufacturer** | **Part #** | **Lot #/ Serial #** | **Other** |
|---|---|---|---|---|---|
| EB expansion medium | See Table 5, below. | | | | |
| PBMCs | See below | | | | |
| Gentamycin | | Gibco | 15750-060 | 780801 | |
| StemSpan SFEM | | StemCell Technologies | 09650 | | |
| Matrigel | | BD | 354230 | 82895 | |
| DMEM/F12 | | Gibco | 11330 | 891768 | |
| Reprogramming Medium | | | AD1-4-1;1 | | |
| RetroNectin | 1mg/ml | Takara | T100A | AA601 | Diluted to 5µg/ml in PBS. Plates coated with 1ml |
| PBS +/+ | | Gibco | 14040 | 764950 | |
| PBS -/- | | Gibco | 14190-144 | 872284 | |
| Trypsin 0.5% | | Gibco | 15400 | 860083 | Diluted to 0.05% with PBS -/- |
| Amaxa Human CD34 Cell Nucleofector Kit | | Lonza | VPA-1003 | F07990 | |
| Reprogramming vector #34 pEP4EO2SEN2K | 1mg/ml | | | | 2.96µg used |
| Reprogramming vector #36 pEP4EO2SET2K | 1mg/ml | | | | 3.2µg used |
| Reprogramming vector #123 pCEP4-LM2L aka (L-myc ires Lin28) | 1mg/ml | | | | 2.28µg used |
| 1038 pPBml-PP-pEFxZsGreen (FIG. 3) | 1.29mg/ml | | 1038 pPBml-PP-pEFxZsGreen | | 4µg used |
| PBacase | 1mg/ml | | | | 4µg used |
| TeSR | | Stemcell | 05850 (supplement) | 10H35844D | |
| | | | 05857 (basal) | 10K36588D | |
| TeSR2 | | Stemcell | 05861 (basal) | 10J36713 | |
| | | | 05862 (5x supplement) | 10L37035 | |
| | | | 05863 (25x supplement) | 10H36234 | |
| Anti-h TRA-1-61 | | R&D Systems | MAB4770 | | |
| Alexa Fluor594 | | Invitrogen | A21044 | | |
| goat anti-mouse IgM | | | | | |

### Initial Cell Culture

Adult PBMCs were cultured for 6 days as follows prior to engineering/reprogramming.

EB (erythroblast) expansion medium was prepared as shown in Table 5. Each cytokine is dissolved according to recommendations from manufacturer. Stock solutions are prepared in sterile PBS + 2 mg/ml BSA. The thawed cytokine aliquots can be stored at 4°C up to 2 weeks. It is advisable to use antibiotics at initiation of primary cell culture. Gentamycin (10 µg/ml final concentration) was used (Invitrogen).

**Table 5: EB expansion medium**

| ***Name*** | ***Vendor*** | ***Cat#*** | ***Stock*** | ***Storage*** | ***Final conc.*** | ***DF*** | ***100 ml*** |
|---|---|---|---|---|---|---|---|
| StemSpan SFEM | StemCell Technologies | 09650 | 500 ml | 4°C | - | - | 100 ml |
| ExCyte medium supplement | Millipore | 81-129-N | 10 ml | 4°C | - | 1/1000 | 100 µl |
| GlutaMax | Invitrogen | 35050-061 | 100 ml | -20°C | - | 1/100 | 1 ml |
| SCF | Peprotech | 300-07 | 500 µg/ml | -80°C | 250 ng/ml | 1/2000 | 50 µl |
| IL-3 | Peprotech | 200-03 | 100 µg/ml | -80°C | 20 ng/ml | 1/5000 | 20 µl |
| EPO | | | | | 2 U/ml | | |
| IGF-1 | | | | | 40 ng/ml | | |
| Dexamethasone | | | | | 1 µM | | |

One vial of 10 x 10⁷ PBMCs was removed from liquid nitrogen and thawed in a 37°C water bath. The cells were transferred dropwise to a 50 ml conical tube containing 30 ml StemSpan® SFEM. The tube was centrifuged at 1200 RPM for 5 minutes. The cells were resuspended in 10 mL StemSpan® SFEM and then counted using a hemocytometer. 9.4 x 10⁷ cells were recovered. The cells were centrifuged again at 1200 RPM and resuspended in 94 ml EB expansion medium + 10 µg/ml gentamycin for a final cell density of 1 x 10⁶ cells/ml. To 6-well plates, 2 ml of cells were added per well for a total of 44 wells. The cells were incubated at 37°C, 5% CO₂ over the weekend and then 2 ml of EB expansion medium was added to each well.

### Nucleofection

Nucleofection (Day 0), suspended cells were collected and counted using a hemocytometer. 1.8 x 10⁷ cells were recovered. Flow cytometry was conducted on a small sample of cells.

RetroNectin-coated plates were used for this experiment. RetroNectin stock (1 mg/mL) was diluted to 5 µg/mL with standard 1xPBS and gently mixed by inversion. Diluted RetroNectin (1 mL) was added to each well of a non-tissue treated 6 well plate. To ensure even dispersal, the plate was tapped. Plates were incubated for 2 hours at room temperature. Alternatively, plates can be wrapped with parafilm and placed on an even surface at 4°C overnight for use the following day. The treated wells were washed with 1x PBS, 2 mL of 2% BSA solution were added and plates were incubated for 30 minutes at room temperature. Wells were washed with 1x PBS and used immediately.

A DNA master mix for each of the experimental conditions was created as follows:

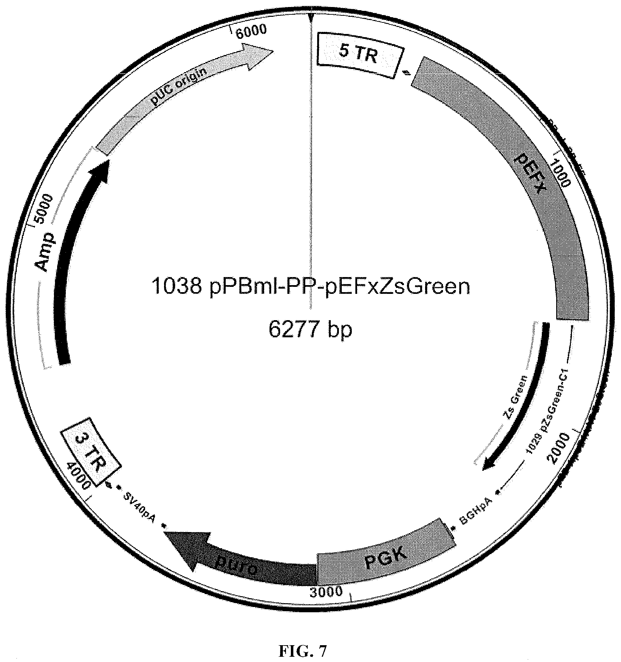

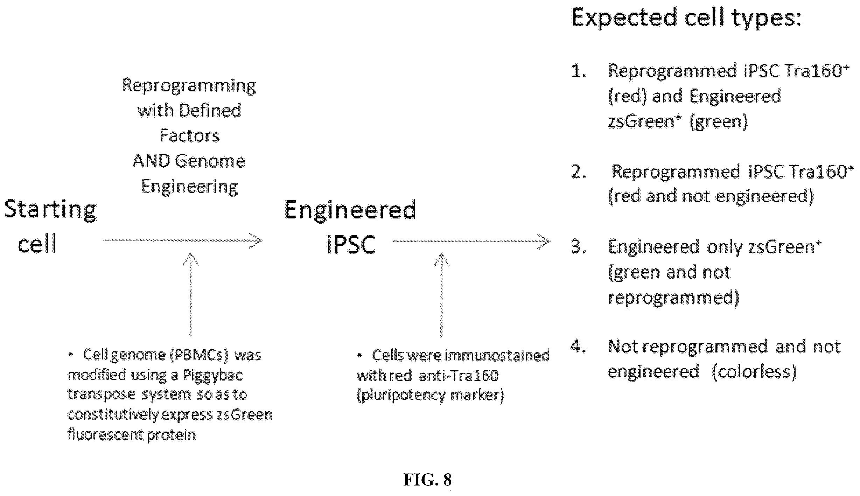

Wells 1.1-1.6: Piggybac transposase DNA vector + Reprogramming DNA vectors (#34, #36 & #123; FIGs. 3, 4 and 5, respectively) + vector DNA #1038 (shown in FIG. 7) for zsGreen expression to be inserted by transposase. Master Mix for wells 1.1-1.6 (all wells in a six well plate). Use 15.5 µl Master Mix + 1 million cells/nucleofection/well, shown in Table 6 below. The general scheme is described in FIG. 8.

**Table 6: Nucleofection plate 1.**

| **Vector** | **µl/RxN** | **µl for 7 RxNs** |
|---|---|---|
| #34 | 2.96 | 20.72 |
| #36 | 3.2 | 22.4 |
| #123 | 2.28 | 15.96 |
| #1038 | 3.1 | 21.7 |
| Pbacase | 4 | 28 |

Control Wells 3.1-3.2: Reprogramming DNA vectors (#34, #36 & #123; FIGs. 3, 4 and 5, respectively) + PB transpose DNA vector #1038 (shown in FIG. 7), no PB transposase. 11.5 µl Master Mix + 1 million cells/nucleofection/well, as shown in Table 7, below.

**Table 7: Nucleofection plate 3.**

| Vector | µl/RxN | µl for 3.5 RxNs |
|---|---|---|
| #34 | 2.96 | 20.72 |
| #36 | 3.2 | 22.4 |
| #123 | 2.28 | 15.96 |
| #1038 | 3.1 | 21.7 |

The appropriate volume of the master mix was placed on the side of each cuvette. Cells were combined with nucleofection solution for a density of 1 x 10⁷ cells/100 µl. 100 µl of cells were added to the electroporation cuvette and flicked several times to mix in the master mix. The cells were electroporated (well 1.5 had an error of Arc.c.1). Immediately following electroporation, 500 µl of reprogramming medium was added to each cuvette following electroporation. The contents of the cuvette were transferred to 1 well of a 6-well plate containing 2 ml reprogramming medium. Plates were incubated at 37°C, 5% CO₂.
Day 2 - Changed medium to reprogramming medium. Spun down each well of cells and resuspended cells in 2 ml reprogramming medium
Day 4 - Added 2 ml reprogramming medium
Day 6 - Removed 1 ml medium, replaced with 2 ml fresh reprogramming medium.
Day 8 - Removed 2 ml medium, replaced with 2 ml fresh reprogramming medium.
Day 11 - Removed 1.5 ml medium, replaced with 2 ml TeSR2. Performed a cursory count of fluorescent green colonies in each well.
Day 13 - 75% of medium removed. Fed with 2 ml TeSR2.
Day 15 - Fed with TeSR2
Day 18 - Fed with TeSR2
Day 19 - Live cell staining, pick colonies (AS)
Day 20 - Live cell staining and picking of colonies (SD)

Results from the experiments with the piggyBac vector integration are summarized below:
- Starting cells for simultaneous engineering and reprogramming: 6 million expanded human PBMCs (6 wells total)
- Number of early cell colonies positive for anti-Tra160 (red) and expressing green zsGFP: 100's
- Number of early cell colonies positive for anti-Tra160 (red only): approximately 50
- Number of green only cells: 2
- Control wells for piggyBac (no transposase added): 2 million human PBMCs (2 wells total)
- Number of early cell colonies positive for anti-Tra160 (red) and expressing green zsGFP: 0
- Number of early cell colonies positive for anti-Tra160 (red only): 100's
- Number of green only cells: 0

### Example 3 - Episomal reprogramming and genome engineering of PBMCs using Zinc Finger Nuclease

The same process was used as described in Example 2 except the experimental conditions were as follows:

Wells 2.1-2.6: Zinc finger nuclease encoding RNA + Reprogramming vectors + vector 1036 for ZsGreen gene expression to be inserted by zinc-finger nuclease. DNA to be inserted at the AAVS1 zinc finger cut site. Used 12.0 µL master mix + 5 µl ZFN RNA + 1 million cells/nucleofection/well, as shown in Table 8 below. The general scheme is described in FIG. 9.

**Table 8: Zinc Finger nucleofection plate**

| Vector | µl/RxN | µl for 7 RxNs |
|---|---|---|
| #34 | 2.96 | 20.72 |
| #36 | 3.2 | 22.4 |
| #123 | 2.28 | 15.96 |
| #1036 | 3.6 | 25.2 |

Control Wells 4.2-4.3: These wells became contaminated and did not produce results. No zinc finger nuclease encoding RNA + reprogramming DNA vectors + vector #1036. Used 12.0 µL master mix + 1 million cells/nucleofection/well, as shown in Table 9 below.

**Table 9: Control Nucleofection plate**

| Vector | µl/RxN | µl for 3.5 RxNs |
|---|---|---|
| #34 | 2.96 | 20.72 |
| #36 | 3.2 | 22.4 |
| #123 | 2.28 | 15.96 |
| #1036 | 3.6 | 25.2 |

The appropriate volume of the master mix was placed on the side of each cuvette (zinc finger mRNA was added to the master mix just before use). The remainder of the procedure was the same as described in Example 2.

Results from the experiments with the zinc finger nuclease are summarized below:
- Starting cells for simultaneous engineering and reprogramming: 6 million expanded human PBMCs (6 wells)
- Number of early cell colonies positive for anti-Tra160 (red) and expressing green zsGFP: 15
- Number of early cell colonies positive for anti-Tra160 (red only): approximately 200
- Number of green only cells: 1
- Control wells for zinc finger experiment (no zinc finger nuclease added): both wells were contaminated and did not give data.
- Number of early cell colonies positive for anti-Tra160 (red) and expressing green zsGFP: 0
- Number of early cell colonies positive for anti-Tra160 (red only): 100's
- Number of green only cells: 0

### Example 4 - Efficiencies evident when genome engineering and cell reprogramming are performed simultaneously compared to sequential approaches

As is presented in FIG. 10, the simultaneous cell reprogramming and genome engineering process of the present invention (Process 3, in FIG. 10) takes significantly less time compared to the time required to perform genome engineering followed by cell reprogramming (Process 2 in FIG. 10) and even greater time savings than is evident for cell reprogramming followed by genome engineering (Process 1 in FIG. 10). In addition to time savings, significantly less materials (such as culture plates) are utilized when cell reprogramming and engineering are performed simultaneously. As such, Process 3 is a significantly more efficient process in terms of both time and materials compared to current best practices. Using Process 2 over Process 1 results in 19% less time spent and uses 60% fewer plates. Using Process 3 over Process 1 results in 34% less time spent and uses 61% fewer plates. Using Process 3 over Process 2 results in 19% less time spent and uses approximately the same number of plates.

Below is a detailed example analysis of microplate and flask usage during cell reprogramming and genome engineering steps:

Colony picking and expansion after reprogramming for 1 donor sample: 44 total plates used to obtain 3 colonies.

Colony picking and expansion after genome engineering iPSCs for 1 donor sample: 69 total plates used to obtain 3 colonies.

Genome engineering of primary cells followed by drug selection and expansion for 1 donor: 1 plate and 2 T-flasks used, no picking of colonies performed.

Simultaneous genome engineering and reprogramming of primary cells for 1 donor sample: 44 total plates used to obtain 3 colonies.

To summarize, Process 1 uses 113 plates (69 + 44) per donor for 3 colonies selected and characterized and 3 additional colonies frozen). Processes 2 and 3 use 44 or 45 plates, respectively, and save roughly 68 plates per donor. If high throughput donor sample preparation is employed, performing either of Processes 2 or 3 compared to Process 1 can result in significant materials savings with Process 3 further providing the greatest time savings (19 % savings compared to Process 2). In summary, employing Process 3, in which cell reprogramming and genome engineering are performed simultaneously, provides a significant improvement in the current best practices for the industrialization of genome engineered iPSC production.

### Example 5: Design of TALE Nuclease Targeting the MYH6 Gene

The last intron of the human MYH6 gene is targeted for homologous recombination in order to produce a fusion protein under the control of the native MYH6 promoter. The targeted human sequence Chromosome 14 (minus strand), intron 38-39, position 23,851,273-23,851,636, is 364 bp long. Twenty seven possible TAL Effector binding sites (plus strand) were identified and range from recognizing 16 to 30 bp DNA using the software program provided at the Iowa State University website (boglabx.plp.iastate.edu/TALENT/). Sixteen of the sequences are exemplified below in Table 10. To select the optimal DNA binding domain, the uniqueness of each sequence is examined using BLAST homology analysis against the human genome and transcribed sequences. The optimal sequences will be as long as possible but still unique and have the appropriate length (16-25 bp) for the best binding affinity. By example, the TALEN at position 294, the HD binds C, NG binds, T, and NN binds G.

Typically, several TALENs are constructed and tested for efficiency and specificity when introducing the double strand breaks. For each TALEN, a pair of flanking homologous arms are identified which span the DNA break site. These arms are typically 500-1500 bp long, although a range of sizes may be used.

Simultaneous genome engineering and reprogramming of somatic cells, using the designed TALENs, can be accomplished as outlined in Examples 1-3; however, the vector for integration in this example comprises MYH6 sequences that flank the TALEN site in the MYH6 gene.

**Table 10: TAL Effector binding domains and sites in the MYH6 gene**

| TAL Start Position | TAL Length | RVD Sequence (SEQ ID NOs: 5-20) | Target_sequence (SEQ ID NOs: 21-36) |
|---|---|---|---|
| 294 | 17 | HD NG NN NI NI NN NN NN HD NI HD HD HD NI NG NI NG | CTGAAGGGCACCCATAT |
| 134 | 18 | HD HD HD NI HD NN NG NG NI NN NI NN NN HD NI HD NG NG | CCCACGTTAGAGGCACTT |
| 179 | 18 | HD NG HD NG NN HD NI NN NI NI NN NG NG HD HD NI NN NG | CTCTGCAGAAGTTCCAGT |
| 231 | 19 | HD NG HD NI NN NN NG NG NI NG NN NG NI NI NN HD NG NI NG | CTCAGGTTATGTAAGCTAT |
| 134 | 20 | HD HD HD NI HD NN NG NG NI NN NI NN NN HD NI HD NG NG NN NG | CCCACGTTAGAGGCACTTGT |
| 181 | 20 | HD NG NN HD NI NN NI NI NN NG NG HD HD NI NN NG HD NI NN NG | CTGCAGAAGTTCCAGTCAGT |
| 248 | 21 | | ATGGGACCCTCAGAACTGCCT |
| 179 | 22 | | CTCTGCAGAAGTTCCAGTCAGT |
| 287 | 22 | | GCCCACTCTGAAGGGCACCCAT |
| 250 | 23 | | GGGACCCTCAGAACTGCCTACAT |
| 226 | 24 | | GGATTCTCAGGTTATGTAAGCTAT |
| 241 | 24 | | GTAAGCTATGGGACCCTCAGAACT |
| 287 | 24 | | GCCCACTCTGAAGGGCACCCATAT |
| 248 | 25 | | ATGGGACCCTCAGAACTGCCTACAT |
| 250 | 25 | | GGGACCCTCAGAACTGCCTACATAT |
| 269 | 25 | | ACATATAGGGCAAGCAGTGCCCACT |

All of the methods disclosed and claimed herein can be made and executed without undue experimentation in light of the present disclosure. While the methods of this invention have been described in terms of preferred embodiments, it will be apparent to those of skill in the art that variations may be applied to the methods described herein. More specifically, it will be apparent that certain agents which are both chemically and physiologically related may be substituted for the agents described herein while the same or similar results would be achieved.

### REFERENCES

The following references provide exemplary procedural or other details supplementary to those set forth herein,.
U.S. Patent 4,683,202
U.S. Patent 5,302,523
U.S. Patent 5,322,783
U.S. Patent 5,384,253
U.S. Patent 5,464,765
U.S. Patent 5,478,838
U.S. Patent 5,538,877
U.S. Patent 5,538,880
U.S. Patent 5,550,318
U.S. Patent 5,563,055
U.S. Patent 5,580,859
U.S. Patent 5,589,466
U.S. Patent 5,610,042
U.S. Patent 5,656,610
U.S. Patent 5,702,932
U.S. Patent 5,736,524
U.S. Patent 5,780,448
U.S. Patent 5,789,215
U.S. Patent 5,925,565
U.S. Patent 5,928,906
U.S. Patent 5,935,819
U.S. Patent 5,945,100
U.S. Patent 5,981,274
U.S. Patent 5,994,624
U.S. Patent 6,833,269
U.S. Appln. 12/478,154
U.S. Patent Publn. 2002/0076976
U.S. Patent Publn. 2003/0059913
U.S. Patent Publn. 2003/0062225
U.S. Patent Publn. 2003/0062227
U.S. Patent Publn. 2003/0087919
U.S. Patent Publn. 2003/0125344
U.S. Patent Publn. 2003/0211603
U.S. Patent Publn. 2004/0002507
U.S. Patent Publn. 2004/0002508
U.S. Patent Publn. 2004/0014755
U.S. Patent Publn. 2004/0039796
U.S. Patent Publn. 2005/0192304
U.S. Patent Publn. 2005/0209261
U.S. Patent Publn. 2005/123902
U.S. Patent Publn. 2007/0116680
U.S. Patent Publn. 2007/0238170
U.S. Patent Publn. 2008/004287
U.S. Patent Publn. 2008/0171385
A practical approach, 1987.
Adams, J. Virol., 61(5):1743-1746, 1987.
Aiyar et al., EMBO J., 17(21):6394-6403, 1998.
Alexander et al., Proc. Nat. Acad. Sci. USA, 85:5092-5096,1988.
Altmann et al., Proc. Natl. Acad. Sci. USA, 103(38):14188-14193, 2006.
Amit et al., Dev Biol., 227(2):271-8, 2000.
Animal Cell Culture, 1987.
Aravind and Landsman, Nucleic Acids Res., 26(19):4413-4421, 1998.
Ausubel et al., Current Protocols in Molecular Biology, Greene Publishing Associates and Wiley Interscience, N.Y., 1994.
Baer et al., Biochemistry, 39:7041-7049, 2000.
Baer et al., Nature, 310(5974):207-211, 1984.
Bain et al., Biochem. J., 408(3):297-315, 2007.
Bennett et al, J. Biol. Chem., 277:34, 2002.
Bertrand et al., J. MoI Biol., 333(2):393-407, 2003.
Bingham, Cell, 90(3):385-387, 1997.
Bochkarev et al., Cell, 84(5):791-800, 1996.
Bode et al., Biol. Chem., 381:801-813, 2000.
Bode et al., Gene Ther. Mol. Biol., 6:33-46, 2001.
Bode et al., Science, 255(5041):195-197,1992.
Buecker et al., Cell Stem Cell, 6:535-546, 2010.
Buehr et al., Cell, 135:1287, 2008.
Carbonelli et al., FEMS Microbiol. Lett., 177(1):75-82, 1999.
Cermak et al., Nucleic Acids Res., 39(12):e82, 2011.
Chandler et al., Proc. Natl. Acad. Sci. USA, 94(8):3596-601, 1997.
Chang, et al., Frontiers in Bioscience, 12:4393-4401, 2007.
Chaudhuri et al., Proc. Natl. Acad. Sci. USA, 98(18):10085-10089, 2001.
Chen and Okayama, Mol. Cell Biol., 7(8):2745-2752, 1987.
Chen et al., Nature Methods, 8:424-429, 2011.
Chen et al., Cell Stem Cell, 7:240-248, 2010.
Chen et al., Gene Ther., 11:856-864, 2004.
Chin et al., Molecular Brain Res., 137(1-2): 193-201, 2005.
Chow et al., Cytometry Commun. Clinical Cytometry, 46:72-78, 2001.
Christian et al., Genetics, 186(2):757-761, 2010.
Cocea, Biotechniques, 23(5):814-816, 1997.
Current Protocols in Molecular Biology and Short Protocols in Molecular Biology, 1987; 1995.
DaCosta et al., Molec. Pharmacol., 65(3):744-752, 2004.
Davies et al., Biochem J., 351:95-105, 2000.
deFelipe, Curr. Gene Ther., 2:355-378, 2002.
de Gouville et al., Drug News Perspective, 19(2):85-90, 2006.
Dhar et al., Cell, 106(3):287-296, 2001.
Downey et al., J. Biol. Chem., 271(35):21005- 21011, 1996.
Embryonic Stem Cell Differentiation in vitro, 1993.
English et al., Trends in Pharmac. Sci., 23(1):40-45, 2002.
Ercolani et al., J. Biol. Chem., 263:15335-15341,1988.
Ermakova et al., J. Biol. Chem., 271(51):33009-33017, 1996.
Esteban et al., J. Biol. Chem., 284:17634-17640, 2009.
European Appln. EPO 0273085
Evans and Kaufman, Nature, 292:154-156, 1981.
Evans, et al., In: Cancer Principles and Practice of Oncology, Devita et al. (Eds.), Lippincot-Raven, NY, 1054-1087, 1997.
Fechheimer et al., Proc Natl. Acad. Sci. USA, 84:8463-8467, 1987.
Fernandes et al., Nature Cell Biology, 6:1082-1093, 2004.
Fischer et al., J. Virol., 71:5148-5146, 1997.
Fraley et al., Proc. Natl. Acad. Sci. USA, 76:3348-3352, 1979.
Frame et al, Biochemical J., 359:1-16, 2001.
Frappier and O'Donnell, Proc. Natl. Acad. Sci. USA, 88(23):10875-10879, 1991.
Fusaki et al., Proc. Jpn. Acad. Ser. B Phys. Biol. Sci., 85:348-362, 2009.
Gahn and Schildkraut, Cell, 58(3):527-535, 1989.
Gahn and Sugden, J. Virol., 69(4):2633-2636, 1995.
Garrick et al., Nat. Genet., 18:56-59, 1998.
Gellibert, et al., J. Med. Chem., 49(7):2210-2221, 2006.
Gene Targeting, A Practical Approach, 1993.
Gene Transfer Vectors for Mammalian Cells, 1987.
Ghosh and Bachhawat, In: Liver Diseases, Targeted Diagnosis and Therapy Using Specific Receptors and Ligands, Wu et al. (Eds.), Marcel Dekker, NY, 87-104, 1991.
Gopal, Mol. Cell Biol., 5:1188-1190, 1985.
Gould et al, Intl. J. Neuropsychopharmacology, 7:387-390, 2004.
Gould et al, Pharmacological Res., 48:49-53, 2003.
Graham and Van Der Eb, Virology, 52:456-467, 1973.
Guide to Techniques in Mouse Development (1993)
Hanna et al., Proc. Natl. Acad. Sci. USA, 107:9222-9227, 2010.
Harb et al., PLoS One, 3(8):e3001, 2008.
Harland and Weintraub, J. Cell Biol., 101(3):1094-1099, 1985.
Hegde et al., Nature, 359(6395):505-512, 1992.
Hogan et al., In: Manipulating the Mouse Embryo: A Laboratory Manual, 2nd Ed.,Cold Spring Harbor Laboratory Press, 1994.
Hung et al., Proc. Natl. Acad. Sci. USA, 98(4):1865-1870, 2001.
Inman et al., Molec. Pharmacol., 62(1):65-74, 2002.
Jainchill et al., J. Virol., 4(5):549-53, 1969.
Jenke et al., Proc. Natl. Acad. Sci. USA, 101 (31), 11322-11327, 2004.
Jia et al., Nat. Methods, 7:197-199, 2010.
Julien et al., Virology, 326(2):317-328, .2004.
Kaeppler et al., Plant Cell Reports, 9:415-418, 1990.
Kahn et al., Molecular Therapy, 18(6): 1192-1199, 2010.
Kaji et al., Nature, 458:771-775, 2009.
Kameda et al., Biochem. Biophys. Res. Commun., 349:1269-1277, 2006.
Kaminska et al., Acta Biochimica Polonica, 52(2):329-337, 2005.
Kanda et al., Mol. Cell. Biol., 21(10):3576-3588, .2001.
Kaneda et al., Science, 243:375-378, 1989.
Karin et al. Cell, 36:371-379,1989.
Kato et al, J. Biol. Chem., 266:3361-3364, 1991.
Keller et al., Curr. Opin. Cell Biol., 7(6):862-9, 1995.
Kennedy and Sugden, Mol. Cell. Biol., 23(19):6901-6908, 2003.
Kennedy et al., Proc. Natl. Acad. Sci. USA, 100:14269-14274, 2003.
Kim et al., Cell Stem Cell, 4:472, 2009.
Kim et al., J. Biol. Chem., 275(40):31245-31254, 2000.
Kim et al., Virology, 239(2):340-351, 1997.
Kim et al., Xenobiotica, 38(3):325-339, 2008.
Kirchmaier and Sugden, J. Virol., 69(2):1280-1283, 1995.
Kirchmaier and Sugden, J. Virol., 72(6):4657-4666, 1998.
Klein et al, Neoplasia, 8:1-8, 2006.
Klein et al., Nature, 327:70-73, 1987.
Klimanskaya et al., Lancet., 365(9471):1636-41, 2005.
Kodama et al. J. Cell Physiol., 112(1):89-95, 1982.
Langle-Rouault et al., J. Virol., 72(7):6181-6185, 1998.
Leight and Sugden, Mol. Cell Bio., 21:4149-61, 2001.
Levenson et al., Hum. Gene Ther., 9(8):1233-1236, 1998.
Levitskaya et al., Nature, 375(6533):685-688, 1995.
Levitskaya et al., Proc. Natl. Acad. Sci. USA, 94(23):12616-12621, 1997.
Li et al., Cell Stem Cell, 4:16, 2009.
Li et al., Cell Stem Cell, 4:16-19, 2009.
Li et al., Cell, 135:1299, 2008.
Li et al., Nucleic Acids Res., 39(1):359-372, 2011.
Lin et al., Nat. Methods, 6:805-808, 2009.
Lindner and Sugden, Plasmid, 58:1-12, 2007.
Lindner et.al. J. Virol., 82(12):5693-702, 2008.
Liu et al., Biochem. Biophys. Res. Commun., 346:131-139, 2006.
Liu et al., Cell Stem Cell 3, 587-590, 2008.
Loh et al., Blood, 113:5476-5479, 2009.
Lowry et al., Proc. Natl. Acad. Sci. USA, 105:2883, 2008.
Ludwig et al., Nat. Biotechnol., 24(2):185-187, 2006b.
Ludwig et al., Nat. Methods, 3(8):637-46, 2006a.
Ludwig et al., Nat. Methods, 3:637-646, 2006c.
Macejak and Sarnow, Nature, 353:90-94, 1991.
Mackey and Sugden, Mol. Cell. Biol., 19(5):3349-3359, 1999.
Mackey et al., J. Virol., 69(10):6199-6208, 1995.
Maniatis, et al., Molecular Cloning, A Laboratory Manual, Cold Spring Harbor Press, Cold Spring Harbor, N.Y., 1988.
Manzini et al., Proc. Natl. Acad. Sci. USA, 103(47): 17672-17677, 2006.
Marechal et al., J. Virol., 73(5):4385-4392, 1999.
Martin, et al., Nature Immunology, 6:111-184, 2005.
Martin, Proc. Natl. Acad. Sci. USA, 78(12):7634-8, 1981.
Mattingly et al, J. Pharmacol. Experimen. Therap., 316:456-465, 2006.
Middleton and Sugden, J. Virol., 66(1):489-495, 1992.
Middleton and Sugden, J. Virol., 68:4067-4071, 1994.
Miller et al., Nat. Biotechnol., 29(2):143-148, 2011.
Nabel et al., Science, 244(4910):1342-1344, 1989.
Nakagawa et al. Proc. Natl. Acad. Sci. USA, 107(32):14152-7, 2010.Nakano et al., Science, 272(5262):722-4, 1996.
Nanbo et al., EMBO J., 26:4252-62, 2007.
Ng, Nuc. Acid Res., 17:601-615,1989.
Nicolau and Sene, Biochim. Biophys. Acta, 721:185-190, 1982.
Nicolau et al., Methods Enzymol., 149:157-176, 1987.
Niller et al., J. Biol. Chem., 270(21):12864-12868, 1995.
Noble et al, Proc. Natl. Acad. Science, USA, 102:6990-6995, 2005.
Okita et al., Nature, 448:313, 2007.
Okita et al., Nature, 448:313-317, 2007.
Okita et al., Science, 322:949, 2008.
Okita et al., Science, 322:949-953, 2008.
Park et al., Nature, 451:141, 2008.
PCT Appln. WO 2007/113505
PCT Appln. WO 2008/006583
PCT Appln. WO 2008/094597
PCT Appln. WO 94/09699
PCT Appln. WO 95/06128
PCT Publn. PCT 2005/080554
PCT Publn. WO 01/088100
PCT Publn. WO 98/30679
Pelletier and Sonenberg, Nature, 334(6180):320-325, 1988.
Perales et al., Proc. Natl. Acad. Sci. USA, 91:4086-4090, 1994.
Piechaczek et al., Nucleic Acids Res., 27(2):426-428, 1999.
Potrykus et al., Mol. Gen. Genet., 199(2):169-177, 1985.
Potter et al., Proc. Natl. Acad. Sci. USA, 81:7161-7165, 1984.
Properties and uses of Embryonic Stem Cells: Prospects for Application to Human Biology and Gene Therapy (1998)
Quitsche et al., J. Biol. Chem., 264:9539-9545,1989.
Rawlins et al., Cell, 42((3):859-868, 1985.
Reisman and Sugden, Mol. Cell. Biol., 6(11):3838-3846, 1986.
Reisman et al., Mol. Cell. Biol., 5(8):1822-1832, 1985.
Richards et al., Cell, 37:263-272, 1984.
Rinehart et al., J. Clinical Oncol., 22:4456-4462, 2004.
Ring et al., Diabetes, 52:588-595, 2003.
Rippe, et al., Mol. Cell Biol., 10:689-695, 1990.
Ritzi et al., J. Cell Sci., 116(Pt 19):3971-3984, 2003.
Ryan et al., J. Gener. Virol., 78:699-722, 1997.
Sambrook et al., In: Molecular cloning: a laboratory manual, 2nd Ed., Cold Spring Harbor Laboratory Press, Cold Spring Harbor, NY, 1989.
Schaarschmidt et al., EMBO J., 23(1):191-201, .2004.
Schaffer et al., Gene, 302(1-2):73-81, 2003.
Schepers et al., EMBO J., 20(16):4588-4602, 2001.
Scymczak et al., Nature Biotech., 5:589-594, 2004.
Sears et al., J. Virol., 77(21):11767-11780, 2003.
Sears et al., J. Virol., 78(21):11487-11505, 2004.
Shi et al., Cell Stem Cell, 3:568, 2008.
Shimada et al., Mol. Reprod. Dev, 77:2, 2010.
Shire et al., J. Virol., 73(4):2587-2595, 1999.
Silva et al., PLoS Biol., 6:e253, 2008.
Stadtfeld et al., Cell Stem Cell, 2:230-240, 2008.
Stadtfeld et al., Science, 322:945, 2008.
Stadtfeld et al., Science, 322:945-949, 2008.
Su et al., Proc. Natl. Acad. Sci. USA, 88(23):10870-19874, 1991.
Sugden and Warren, J. Virol., 63(6):2644-2649, 1989.
Sun et al., Proc. Natl. Acad. Sci. USA, 106:15720-15725, 2009.
Suzuki et al., Cancer Res., 67(5):2351-2359, 2007.
Takahashi et al., Cell, 126(4):663-676, 2006.
Takahashi et al., Cell, 131:861, 2007.
Thomson et al., Science, 282:1145, 1998.
Tojo, et al., Cancer Science, 96(11):791-800, 2005,
Tur-Kaspa et al., Mol. Cell Biol., 6:716-718, 1986.
Wagman, Current Pharmaceutical Design, 10:1105-1137, 2004.
Wagner et al., Proc. Natl. Acad. Sci. USA 87(9):3410-3414, 1990.
Wang et al., Mol. Cell. Biol., 26(3):1124-1134, 2006.
Watanabe et al., Nat. Biotechnol., 25:681-686, 2007.
Watanabe et al., Nat. Neurosci., 8(3):288-96, 2005.
Weber et al., PLoS One, 6(5):e19722, 2011.
Wernig et al., Nature, 448(7151):318-24, 2007.
Wilson et al., Science, 244:1344-1346, 1989.
Woltjen et al., Nature, 458:766, 2009.
Woltjen et al., Nature, 458:766-770, 2009.
Wong et al., Gene, 10:87-94, 1980.
Wrzesinski et al., Clinical Cancer Res., 13(18):5262-5270, 2007.
Wu and Wu, Adv. Drug Delivery Rev., 12:159-167, 1993.
Wu and Wu, Biochemistry, 27: 887-892, 1988.
Wu and Wu, J. Biol. Chem., 262:4429-4432, 1987.
Wu et al., J. Virol., 76(5):2480-2490, 2002.
Wysokenski and Yates, J. Virol., 63(6):2657-2666, 1989.
Xu et al., Nat. Biotechnol., 19:971, 2001.
Xu et al., Nat. Biotechnol., 19:971-974, 2001.
Yamanaka et al., Cell, 131(5):861-72, 2007.
Yang and Russell, Proc. Natl. Acad. Sci. USA, 87:4144-4148, 1990.
Yates and Guan, J. Virol., 65(1):483-488, 1991.
Yates and Guan, J. Virol., 65:483-488, 1991.
Yates et al., J. Virol., 74(10):4512-4522, 2000.
Yates et al., Nature, 313:812-815, 1985.
Yates et al., Proc. Natl. Acad. Sci. USA, 81:3806-3810, 1984.
Yates, Cancer Cells, (6)197-205, 1988.
Yin et al., Science, 301(5638):1371-1374, 2003.
Ying, Nature, 453:519-23, 2008.
Yu et al., Science, 318:1917, 2007.
Yu et al., Science, 324:797, 2009.
Yu et al., Science, 324:797-801, 2009.
Zhang et al., Bioorganic Med. Chem. Letters; 10:2825-2828, 2000.
Zhang et al., Nat. Biotechnol., 29(2):149-153, 2011.
Zhou and Freed, Stem Cells, 2009 (Ahead of Epub Print).
Zhou et al., Cell Stem Cell, 4:381-384, 2009.
Zhou et al., EMBO J., 24(7):1406-1417, 2005.

## Claims

1. An *in vitro* method for producing a population of induced pluripotent stem (iPS) cells, comprising:
a) introducing into somatic cells a first nucleic acid molecule for integration into the genome of the cells and at least a second nucleic acid molecule comprising an extra-chromosomal genetic element that expresses one or more reprogramming factors sufficient when expressed in the somatic cell to convert the somatic cell to a pluripotent stem cell;
b) culturing said cells under reprogramming conditions; and
c) obtaining a population of iPS cells comprising said first nucleic acid integrated in their genome, wherein said first nucleic acid is expressible and wherein the second nucleic acid molecule is not present in the iPS cells.

2. The method of claim 1, further comprising:
d) obtaining a population of iPS cells comprising cells that (i) comprise said first nucleic acid integrated in their genome and cells which (ii) do not comprise the first nucleic acid integrated in their genome;
e) obtaining a first population of iPS cells which comprise said first nucleic acid integrated in their genome and a second population of iPS cells which do not comprise the first nucleic acid integrated in their genome; or
f) screening or selecting iPS cells that comprise a first nucleic acid integrated in their genome thereby obtaining the population of iPS cells wherein said first nucleic acid is expressible.

3. The method of claim 1, wherein the first nucleic acid is expressed upon differentiation of the iPS cells.

4. The method of claim 1, wherein the first nucleic acid molecule is randomly integrated into the genome of the cells or is integrated into a selected genomic site of the cells, preferably the AAVS1 integration site.

5. The method of claim 4, wherein the the first nucleic acid molecule is integrated into a selected genomic site using meganuclease, or a transcription activator-like effector endonuclease (TALEN) that cleaves genomic DNA at the selected site or is integrated into a selected genomic site using homologous recombination.

6. The method of claim 1, wherein step (a) comprises transducing the cells with a viral vector, such as an adeno-associated virus (AAV), a simple retrovirus or a lentivirus vector, comprising the first or second nucleic acid molecule or transfecting with a piggyBac vector comprising the first or second nucleic acid molecule.

7. The method of claim 1, wherein the first nucleic acid molecule comprises a coding sequence of a screenable or selectable marker or a nucleic acid sequence selected from the group consisting of a sequence that corrects a genetic defect in the cells; a sequence that provides resistance to a pathogen infection; a sequence that provides resistance to a drug; a sequence that provides sensitivity to a drug; a sequence that alters immunogenicity of the cells; and a sequence that provides a genetic tag in the cells.

8. The method of claim 1, wherein the somatic cell is a human fibroblast, keratinocyte, hematopoietic cell, mesenchymal cell, adipose cell, endothelial cell, epithelial cell, neural cell, muscle cell, mammary cell, liver cell, kidney cell, skin cell, digestive tract cell, cumulus cell, gland cell, or pancreatic islet cell.

9. The method of claim 1, wherein the second nucleic acid molecule is an RNA or an episomal vector, preferably wherein the episomal vector comprises a replication origin and one or more expression cassettes for expression of reprogramming factors, wherein one or more of said expression cassettes further comprise a nucleotide sequence encoding a trans-acting factor that binds to the replication origin to replicate an extra-chromosomal template, and/or wherein the somatic cell expresses such a trans-acting factor.

10. The method of claim 1, wherein the reprogramming factor comprises one or more selected from the group consisting of Sox, Oct, Nanog, Lin-28, Klf4, C-myc, L-myc, a myc mutant or homolog that is deficient in transformation, and SV40LT.

11. The method of claim 1, wherein step (b) culturing said cells under reprogramming conditions comprises:
(i) culturing the cells essentially free of feeder cells;
(ii) culturing the cells in the presence of a matrix component;
(iii) selecting or screening said cells for the presence of the first nucleic acid molecule,
(iv) culturing the cells for at least from about one day to fifteen days under reprogramming conditions; and/or
(v) culturing the cells in a reprogramming medium.

12. The method of claim 11, wherein the selecting or screening is by fluorescence activated cell sorting (FACS), magnetic activated cell sorting (MACS), flow cytometry or comprises addition of a drug to the cell culture.

13. The method of claim 12, wherein:
(i) the cells are cultured in the presence of the drug beginning about 1 to 3 days after introduction of the first nucleic acid molecule and extra-chromosomal genetic element into the cells and/or
(ii) the cells are cultured in the presence of the drug for about 1 to 10 days.

14. The method of claim 11, wherein:
(i) the reprogramming medium comprises a GSK-3β inhibitor, a MEK inhibitor, a TGF-β receptor inhibitor or a combination thereof;
(ii) the reprogramming medium comprises externally added fibroblast growth factor (FGF), leukemia inhibitory factor (LIF), Rho-associated kinase (ROCK) inhibitor or myosin II inhibitor; and/or
(iii) the reprogramming medium is chemically defined, preferably wherein the chemically defined medium is N2B27 medium.

15. The method of claim 1, further comprising:
(d) culturing the iPS cells under expansion conditions; and/or
(e) characterizing the iPS cells.

16. The method of claim 15, wherein step (d) culturing the iPS cells under expansion conditions comprises
(i) culturing the iPS cells in a medium essentially free of externally added GSK-3β inhibitors, MEK inhibitors, and TGF-13 receptor inhibitors;
(ii) culturing the iPS cells in an expansion medium that is chemically defined; and/or
(iii) culturing the iPS cells in a TeSR medium, mTeSR medium or Essential 8 medium.

17. The method of claim 15, wherein step (e) characterizing the iPS cells comprises detecting one or more pluripotency markers; performing a karyotype analysis; detecting the presence of the first nucleic acid molecule; determining the sequence of the first nucleic acid molecule; detecting the presence of the extra-chromosomal genetic element; teratoma formation analysis; epigenetic analysis; RNA expression analysis; protein expression analysis; or small tandem repeat (STR) detection.

## Patentansprüche

1. *in vitro* Verfahren zur Herstellung einer Population von induzierten pluripotenten Stamm- (iPS) Zellen, umfassend:
a) Einführen eines ersten Nukleinsäuremoleküls in somatische Zellen zur Integration in das Genom der Zellen und mindestens eines zweiten Nukleinsäuremoleküls, umfassend ein extrachromosomales genetisches Element, das einen oder mehrere Umprogrammierungsfaktoren exprimiert, die, wenn sie in der somatischen Zelle exprimiert werden, ausreichend sind, um die somatische Zelle zu einer pluripotenten Stammzelle umzuwandeln;
b) Kultivieren der Zellen unter Umprogrammierungsbedingungen; und
c) Erhalten einer Population von iPS Zellen, umfassend die erste Nukleinsäure, integriert in ihr Genom, wobei die erste Nukleinsäure exprimierbar ist und wobei das zweite Nukleinsäuremolekül nicht in den iPS Zellen vorhanden ist.

2. Verfahren nach Anspruch 1, ferner umfassend:
d) Erhalten einer Population von iPS Zellen, umfassend Zellen, die (i) die erste Nukleinsäure, integriert in ihr Genom, umfassen und Zellen, die (ii) die erste Nukleinsäure, integriert in ihr Genom, nicht umfassen;
e) Erhalten einer ersten Population von iPS Zellen, welche die erste Nukleinsäure, integriert in ihr Genom, umfassen, und eine zweiten Population von iPS Zellen, welche nicht die erste Nukleinsäure, integriert in ihr Genom, umfassen; oder
f) Screenen oder Selektieren von iPS Zellen, die eine erste Nukleinsäure, integriert in ihr Genom, umfassen, wodurch die Population von iPS Zellen erhalten wird, worin die erste Nukleinsäure exprimierbar ist.

3. Verfahren nach Anspruch 1, wobei die erste Nukleinsäure bei Differenzierung der iPS Zellen exprimiert wird.

4. Verfahren nach Anspruch 1, wobei das erste Nukleinsäuremolekül statistisch in das Genom der Zellen integriert ist oder in eine ausgewählte Genomstelle der Zellen, bevorzugt die AAVS1 Integrationsstelle, integriert ist.

5. Verfahren nach Anspruch 4, wobei das erste Nukleinsäuremolekül in eine ausgewählte genomische Stelle integriert wird unter Verwendung einer Meganuklease oder einer Transkriptionsaktivator-ähnlichen Effektorendonuklease (TALEN), die genomische DNA an der ausgewählten Stelle spaltet, oder unter Verwendung homologer Rekombination in eine ausgewählte genomische Stelle integriert wird.

6. Verfahren nach Anspruch 1, wobei Schritt (a) Transduzieren der Zellen mit einem viralen Vektor, wie zum Beispiel einem Adeno-assoziierten Virus (AAV), einem einfachen Retrovirus oder einem Lentivirus-Vektor, umfassend das erste oder zweite Nukleinsäuremolekül, oder Transfizieren mit einem PiggyBac-Vektor, umfassend das erste oder zweite Nukleinsäuremolekül, umfasst.

7. Verfahren nach Anspruch 1, wobei das erste Nukleinsäuremolekül eine kodierende Sequenz eines screenbaren oder selektierbaren Markers oder eine Nukleinsäuresequenz umfasst, die ausgewählt ist aus der Gruppe bestehend aus einer Sequenz, die einen genetischen Defekt in den Zellen korrigiert; einer Sequenz, die Resistenz gegenüber einer Pathogeninfektion bietet; einer Sequenz, die Resistenz gegenüber einem Arzneimittel bietet; einer Sequenz, die Empfindlichkeit gegenüber einem Arzneimittel bietet; einer Sequenz, die die Immunogenität der Zellen verändert; und einer Sequenz, die einen genetischen Tag in den Zellen bietet.

8. Verfahren nach Anspruch 1, wobei die somatische Zelle ein humaner Fibroblast, ein humaner Keratinocyt, eine humane hämatopoetische Zelle, eine humane mesenchymale Zelle, eine humane Fettzelle, eine humane Endothelzelle, eine humane Epithelzelle, eine humane Nervenzelle, eine humane Muskelzelle, eine humane Brustzelle, eine humane Leberzelle, eine humane Nierenzelle, eine humane Hautzelle, eine humane Zelle des Verdauungstrakts, eine humane Kumuluszelle, eine humane Drüsenzelle oder eine humane Pankreasinselzelle ist.

9. Verfahren nach Anspruch 1, wobei das zweite Nukleinsäuremolekül eine RNA oder ein episomaler Vektor ist, bevorzugt wobei der episomale Vektor einen Replikationsursprung und eine oder mehrere Expressionskassetten zur Expression von Umprogrammierungsfaktoren umfasst, wobei eine oder mehrere der Expressionskassetten ferner eine Nukleotidsequenz umfassen, die für einen trans-wirkenden Faktor kodiert, der an den Replikationsursprung bindet um ein extrachromosomales Templat zu replizieren und / oder wobei die somatische Zelle einen solchen trans-wirkenden Faktor exprimiert.

10. Verfahren nach Anspruch 1, wobei der Umprogrammierungsfaktor einen oder mehrere, ausgewählt aus der Gruppe, bestehend aus Sox, Oct, Nanog, Lin-28, Klf4, C-myc, L-myc, einer Myc-Mutante oder einem transformationsdefizienten Homolog und SV40LT umfasst.

11. Verfahren nach Anspruch 1, wobei Schritt (b) Kultivieren der Zellen unter Umprogrammierungsbedingungen umfasst:
(i) Kultivieren der Zellen im Wesentlichen frei von Fütterzellen;
(ii) Kultivieren der Zellen in der Anwesenheit einer Matrixkomponente;
(iii) Selektieren oder Screenen der Zellen auf die Anwesenheit des ersten Nukleinsäuremoleküls,
(iv) Kultivieren der Zellen für mindestens etwa einen Tag bis fünfzehn Tage unter Umprogrammierungsbedingungen; und / oder
(v) Kultivieren der Zellen in einem Umprogrammierungsmedium.

12. Verfahren nach Anspruch 11, wobei das Selektieren oder Screenen durch fluoreszenzaktivierte Zellsortierung (FACS), magnetisch aktivierte Zellsortierung (MACS), Durchflusszytometrie erfolgt oder ein Hinzufügen eines Arzneimittels zu der Zellkultur umfasst.

13. Verfahren nach Anspruch 12, wobei:
(i) die Zellen in der Anwesenheit des Arzneimittels beginnend etwa 1 bis 3 Tage nach Einführung des ersten Nukleinsäuremoleküls und des extrachromosomalen genetischen Elements in die Zellen kultiviert werden und / oder
(ii) die Zellen in der Anwesenheit des Arzneimittels für etwa 1 bis 10 Tage kultiviert werden.

14. Verfahren nach Anspruch 11, wobei:
(i) das Umprogrammierungsmedium einen GSK-3β-Inhibitor, einen MEK-Inhibitor, einen TGF-β-Rezeptor-Inhibitor oder eine Kombination davon umfasst;
(ii) das Umprogrammierungsmedium extern hinzugefügten Fibroblasten-Wachstumsfaktor (FGF), Leukämie-hemmenden Faktor (LIF), Rho-assoziierten Kinase (ROCK)-Inhibitor oder Myosin II-Inhibitor umfasst; und / oder
(iii) das Umprogrammierungsmedium chemisch definiert ist, wobei das chemisch definierte Medium bevorzugt N2B27-Medium ist.

15. Verfahren nach Anspruch 1, ferner umfassend:
(d) Kultivieren der iPS Zellen unter Expansionsbedingungen; und / oder
(e) Charakterisieren der iPS Zellen.

16. Verfahren nach Anspruch 15, wobei Schritt (d) Kultivieren der iPS Zellen unter Expansionsbedingungen umfasst:
(i) Kultivieren der iPS Zellen in einem Medium, das im Wesentlichen frei von extern hinzugefügten GSK-3β-Inhibitoren, MEK-Inhibitoren und TGF-β-Rezeptor-Inhibitoren ist;
(ii) Kultivieren der iPS Zellen in einem Expansionsmedium, das chemisch definiert ist; und / oder
(iii) Kultivieren der iPS Zellen in einem TeSR Medium, einem mTeSR Medium oder einem Essential 8 Medium.

17. Verfahren nach Anspruch 15, wobei Schritt (e) Charakterisieren der iPS Zellen Ermitteln eines oder mehrerer Pluripotenzmarker; Durchführen einer Karyotyp-Analyse; Ermitteln der Anwesenheit des ersten Nukleinsäuremoleküls; Bestimmen der Sequenz des ersten Nukleinsäuremoleküls; Ermitteln der Anwesenheit des extrachromosomalen genetischen Elements; Teratombildungsanalyse; epigenetische Analyse; RNA-Expressionsanalyse; Proteinexpressionsanalyse; oder Small-Tandem-Repeat (STR) Ermittlung umfasst.

## Revendications

1. Procédé *in vitro* pour la production d'une population de cellules souches pluripotentes induites (SPi), comprenant :
a) l'introduction dans des cellules somatiques d'une première molécule d'acide nucléique pour une intégration dans le génome des cellules et d'au moins une seconde molécule d'acide nucléique comprenant un élément génétique extrachromosomique qui exprime un ou plusieurs facteurs de reprogrammation suffisants lorsqu'ils sont exprimés dans la cellule somatique pour convertir la cellule somatique en une cellule souche pluripotente ;
b) la culture desdites cellules dans des conditions de reprogrammation ; et
c) l'obtention d'une population de cellules SPi comprenant ledit premier acide nucléique intégré dans leur génome, ledit premier acide nucléique étant exprimable et la seconde molécule d'acide nucléique n'étant pas présente dans les cellules SPi.

2. Procédé selon la revendication 1, comprenant en outre :
d) l'obtention d'une population de cellules SPi comprenant des cellules qui (i) comprennent ledit premier acide nucléique intégré dans leur génome et de cellules qui (ii) ne comprennent pas le premier acide nucléique intégré dans leur génome ;
e) l'obtention d'une première population de cellules SPi qui comprennent ledit premier acide nucléique intégré dans leur génome et d'une seconde population de cellules SPi qui ne comprennent pas le premier acide nucléique intégré dans leur génome ; ou
f) le criblage ou la sélection des cellules SPi qui comprennent un premier acide nucléique intégré dans leur génome, obtenant de cette façon la population de cellules SPi dans lesquelles ledit premier acide nucléique est exprimable.

3. Procédé selon la revendication 1, dans lequel le premier acide nucléique est exprimé lors de la différenciation des cellules SPi.

4. Procédé selon la revendication 1, dans lequel la première molécule d'acide nucléique est intégrée de façon aléatoire dans le génome des cellules ou est intégrée dans un site génomique choisi des cellules, de préférence le site d'intégration AAVS1.

5. Procédé selon la revendication 4, dans lequel la première molécule d'acide nucléique est intégrée dans un site génomique choisi en utilisant une méganucléase, ou une endonucléase effectrice de type activateur de transcription (TALEN) qui clive l'ADN génomique au niveau du site choisi ou est intégrée dans un site génomique choisi en utilisant une recombinaison homologue.

6. Procédé selon la revendication 1, dans lequel l'étape (a) comprend la transduction des cellules avec un vecteur viral, tel qu'un virus adéno-associé (AAV), un rétrovirus simple ou un vecteur lentiviral, comprenant la première ou la seconde molécule d'acide nucléique ou la transfection avec un vecteur piggyBac comprenant la première ou la seconde molécule d'acide nucléique.

7. Procédé selon la revendication 1, dans lequel la première molécule d'acide nucléique comprend une séquence codante d'un marqueur qui peut être criblé ou sélectionné ou une séquence d'acide nucléique choisie dans le groupe constitué d'une séquence qui corrige une anomalie génétique dans les cellules ; une séquence qui fournit une résistance à une infection par un pathogène ; une séquence qui fournit une résistance à un médicament ; une séquence qui fournit une sensibilité à un médicament ; une séquence qui modifie l'immunogénicité des cellules ; et une séquence qui fournit un marqueur génétique dans les cellules.

8. Procédé selon la revendication 1, dans lequel la cellule somatique est un fibroblaste humain, un kératinocyte, une cellule hématopoïétique, une cellule mésenchymateuse, une cellule adipeuse, une cellule endothéliale, une cellule épithéliale, une cellule neurale, une cellule musculaire, une cellule mammaire, une cellule hépatique, une cellule rénale, une cellule cutanée, une cellule du tube digestif, une cellule du cumulus, une cellule glandulaire, ou une cellule d'îlot pancréatique.

9. Procédé selon la revendication 1, dans lequel la seconde molécule d'acide nucléique est un ARN ou un vecteur épisomique, de préférence dans lequel le vecteur épisomique comprend une origine de réplication et une ou plusieurs cassettes d'expression pour l'expression de facteurs de reprogrammation, dans lequel une ou plusieurs desdites cassettes d'expression comprennent en outre une séquence nucléotidique codant pour un facteur agissant en trans qui se lie à l'origine de réplication pour répliquer une matrice extrachromosomique, et/ou dans lequel la cellule somatique exprime un tel facteur agissant en trans.

10. Procédé selon la revendication 1, dans lequel le facteur de reprogrammation comprend un ou plusieurs du groupe constitué de Sox, Oct, Nanog, Lin-28, Klf4, C-myc, L-myc, un mutant ou homologue de myc qui est déficient en transformation, et SV40LT.

11. Procédé selon la revendication 1, dans lequel l'étape (b) de culture desdites cellules dans des conditions de reprogrammation comprend :
(i) la culture des cellules essentiellement dépourvues de cellules nourricières ;
(ii) la culture des cellules en présence d'un composant matriciel ;
(iii) la sélection ou le criblage desdites cellules pour la présence de la première molécule d'acide nucléique ;
(iv) la culture des cellules pendant au moins environ un jour à quinze jours dans des conditions de reprogrammation ; et/ou
(v) la culture des cellules dans un milieu de reprogrammation.

12. Procédé selon la revendication 11, dans lequel la sélection ou le criblage est réalisé par tri des cellules activées par fluorescence (FACS), tri des cellules activées par des moyens magnétiques (MACS), cytométrie en flux ou comprend l'ajout d'un médicament à la culture cellulaire.

13. Procédé selon la revendication 12, dans lequel :
(i) les cellules sont cultivées en présence du médicament en débutant environ 1 à 3 jours après l'introduction de la première molécule d'acide nucléique et de l'élément génétique extrachromosomique dans les cellules et/ou
(ii) les cellules sont cultivées en présence du médicament pendant environ 1 à 10 jours.

14. Procédé selon la revendication 11, dans lequel :
(i) le milieu de reprogrammation comprend un inhibiteur de GSK-3β, un inhibiteur de MEK, un inhibiteur du récepteur du TGF-β ou l'une de leurs combinaisons ;
(ii) le milieu de reprogrammation comprend, ajoutés en externe, le facteur de croissance des fibroblastes (FGF), le facteur inhibiteur de leucémie (LIF), un inhibiteur de la kinase associée à Rho (ROCK) ou un inhibiteur de la myosine II ; et/ou
(iii) le milieu de reprogrammation est défini chimiquement, de préférence le milieu défini chimiquement étant le milieu N2B27.

15. Procédé selon la revendication 1, comprenant en outre :
(d) la culture des cellules SPi dans des conditions d'expansion ; et/ou
(e) la caractérisation des cellules SPi.

16. Procédé selon la revendication 15, dans lequel l'étape (d) de culture des cellules SPi dans des conditions d'expansion comprend :
(i) la culture des cellules Spi dans un milieu essentiellement dépourvu d'inhibiteurs de GSK-3β, d'inhibiteurs de MEK, et d'inhibiteurs du récepteur du TGF-13, ajoutés en externe ;
(ii) la culture des cellules SPi dans un milieu d'expansion qui est défini chimiquement ; et/ou
(iii) la culture des cellules SPi dans un milieu TeSR, un milieu mTeSR ou un milieu essentiel 8.

17. Procédé selon la revendication 15, dans lequel l'étape (e) de caractérisation des cellules SPi comprend la détection d'un ou de plusieurs marqueurs de pluripotence ; la réalisation d'une analyse du caryotype ; la détection de la présence de la première molécule d'acide nucléique ; la détermination de la séquence de la première molécule d'acide nucléique ; la détection de la présence de l'élément génétique extrachromosomique ; l'analyse de la formation de tératome ; l'analyse épigénétique ; l'analyse de l'expression des ARN ; l'analyse de l'expression des protéines ; ou la détection de petites répétitions en tandem (STR).


### SEQUENCE LISTING

<110> BURKE, THOMAS MILLER, MICHAEL MCLACHLAN, MICHAEL DICKERSON, SARAH STROUSE, ANNE
<120> METHODS FOR CELL REPROGRAMMING AND GENOME ENGINEERING
<130> CDIN.P0038WO
<140> UNKNOWN
   <141> 2012-07-11
<150> 61/506,314
   <151> 2011-07-11
<160> 36
<170> PatentIn version 3.5
<210> 1
   <211> 26
   <212> DNA
   <213> Artificial Sequence
<220>
   <223> Synthetic primer
<400> 1
   accactttga gctctactgg cttctg 26
<210> 2
   <211> 25
   <212> DNA
   <213> Artificial Sequence
<220>
   <223> Synthetic primer
<400> 2
   tggacaaacc acaactagaa tgcag 25
<210> 3
   <211> 26
   <212> DNA
   <213> Artificial Sequence
<220>
   <223> Synthetic primer
<400> 3
   accactttga gctctactgg cttctg 26
<210> 4
   <211> 23
   <212> DNA
   <213> Artificial Sequence
<220>
   <223> Synthetic primer
<400> 4
   acccaaaagg cagcctggta gac 23
<210> 5
   <211> 34
   <212> PRT
   <213> Artificial Sequence
<220>
   <223> Synthetic peptide
<400> 5
   His Asp Asn Gly Asn Asn Asn Ile Asn Ile Asn Asn Asn Asn Asn Asn
   1               5                   10                  15
   His Asp Asn Ile His Asp His Asp His Asp Asn Ile Asn Gly Asn Ile
               20                  25                  30
   Asn Gly
<210> 6
   <211> 36
   <212> PRT
   <213> Artificial Sequence
<220>
   <223> Synthetic peptide
<400> 6
   His Asp His Asp His Asp Asn Ile His Asp Asn Asn Asn Gly Asn Gly
   1               5                   10                  15
   Asn Ile Asn Asn Asn Ile Asn Asn Asn Asn His Asp Asn Ile His Asp
               20                  25                  30
   Asn Gly Asn Gly
           35
<210> 7
   <211> 36
   <212> PRT
   <213> Artificial Sequence
<220>
   <223> Synthetic peptide
<400> 7
   His Asp Asn Gly His Asp Asn Gly Asn Asn His Asp Asn Ile Asn Asn
   1 5 10 15
   Asn Ile Asn Ile Asn Asn Asn Gly Asn Gly His Asp His Asp Asn Ile
   20 25 30
   Asn Asn Asn Gly
   35
<210> 8
   <211> 38
   <212> PRT
   <213> Artificial Sequence
<220>
   <223> Synthetic peptide
<400> 8
   His Asp Asn Gly His Asp Asn Ile Asn Asn Asn Asn Asn Gly Asn Gly
   1 5 10 15
   Asn Ile Asn Gly Asn Asn Asn Gly Asn Ile Asn Ile Asn Asn His Asp
   20 25 30
   Asn Gly Asn Ile Asn Gly
   35
<210> 9
   <211> 40
   <212> PRT
   <213> Artificial Sequence
<220>
   <223> Synthetic peptide
<400> 9
   His Asp His Asp His Asp Asn Ile His Asp Asn Asn Asn Gly Asn Gly
   1 5 10 15
   Asn Ile Asn Asn Asn Ile Asn Asn Asn Asn His Asp Asn Ile His Asp
   20 25 30
   Asn Gly Asn Gly Asn Asn Asn Gly
   35 40
<210> 10
   <211> 40
   <212> PRT
   <213> Artificial Sequence
<220>
   <223> Synthetic peptide
<400> 10
   His Asp Asn Gly Asn Asn His Asp Asn Ile Asn Asn Asn Ile Asn Ile
   1 5 10 15
   Asn Asn Asn Gly Asn Gly His Asp His Asp Asn Ile Asn Asn Asn Gly
   20 25 30
   His Asp Asn Ile Asn Asn Asn Gly
   35 40
<210> 11
   <211> 42
   <212> PRT
   <213> Artificial Sequence
<220>
   <223> Synthetic peptide
<400> 11
   Asn Ile Asn Gly Asn Asn Asn Asn Asn Asn Asn Ile His Asp His Asp
   1 5 10 15
   His Asp Asn Gly His Asp Asn Ile Asn Asn Asn Ile Asn Ile His Asp
   20 25 30
   Asn Gly Asn Asn His Asp His Asp Asn Gly
   35 40
<210> 12
   <211> 44
   <212> PRT
   <213> Artificial Sequence
<220>
   <223> Synthetic peptide
<400> 12
   His Asp Asn Gly His Asp Asn Gly Asn Asn His Asp Asn Ile Asn Asn
   1 5 10 15
   Asn Ile Asn Ile Asn Asn Asn Gly Asn Gly His Asp His Asp Asn Ile
   20 25 30
   Asn Asn Asn Gly His Asp Asn Ile Asn Asn Asn Gly
   35 40
<210> 13
   <211> 44
   <212> PRT
   <213> Artificial Sequence
<220>
   <223> Synthetic peptide
<400> 13
   Asn Asn His Asp His Asp His Asp Asn Ile His Asp Asn Gly His Asp
   1               5                   10                  15
   Asn Gly Asn Asn Asn Ile Asn Ile Asn Asn Asn Asn Asn Asn His Asp
               20                  25                  30
   Asn Ile His Asp His Asp His Asp Asn Ile Asn Gly
           35                  40
<210> 14
   <211> 46
   <212> PRT
   <213> Artificial Sequence
<220>
   <223> Synthetic peptide
<400> 14
   Asn Asn Asn Asn Asn Asn Asn Ile His Asp His Asp His Asp Asn Gly
   1               5                   10                  15
   His Asp Asn Ile Asn Asn Asn Ile Asn Ile His Asp Asn Gly Asn Asn
               20                  25                  30
   His Asp His Asp Asn Gly Asn Ile His Asp Asn Ile Asn Gly
           35                  40                  45
<210> 15
   <211> 48
   <212> PRT
   <213> Artificial Sequence
<220>
   <223> Synthetic peptide
<400> 15
   Asn Asn Asn Asn Asn Ile Asn Gly Asn Gly His Asp Asn Gly His Asp
   1               5                   10                  15
   Asn Ile Asn Asn Asn Asn Asn Gly Asn Gly Asn Ile Asn Gly Asn Asn
               20                  25                  30
   Asn Gly Asn Ile Asn Ile Asn Asn His Asp Asn Gly Asn Ile Asn Gly
           35                  40                  45
<210> 16
   <211> 48
   <212> PRT
   <213> Artificial Sequence
<220>
   <223> Synthetic peptide
<400> 16
   Asn Asn Asn Gly Asn Ile Asn Ile Asn Asn His Asp Asn Gly Asn Ile
   1               5                   10                  15
   Asn Gly Asn Asn Asn Asn Asn Asn Asn Ile His Asp His Asp His Asp
               20                  25                  30
   Asn Gly His Asp Asn Ile Asn Asn Asn Ile Asn Ile His Asp Asn Gly
           35                  40                  45
<210> 17
   <211> 48
   <212> PRT
   <213> Artificial Sequence
<220>
   <223> Synthetic peptide
<400> 17
   Asn Asn His Asp His Asp His Asp Asn Ile His Asp Asn Gly His Asp
   1               5                   10                  15
   Asn Gly Asn Asn Asn Ile Asn Ile Asn Asn Asn Asn Asn Asn His Asp
               20                  25                  30
   Asn Ile His Asp His Asp His Asp Asn Ile Asn Gly Asn Ile Asn Gly
           35                  40                  45
<210> 18
   <211> 50
   <212> PRT
   <213> Artificial Sequence
<220>
   <223> Synthetic peptide
<400> 18
   Asn Ile Asn Gly Asn Asn Asn Asn Asn Asn Asn Ile His Asp His Asp
   1               5                   10                  15
   His Asp Asn Gly His Asp Asn Ile Asn Asn Asn Ile Asn Ile His Asp
               20                  25                  30
   Asn Gly Asn Asn His Asp His Asp Asn Gly Asn Ile His Asp Asn Ile
           35                  40                  45
   Asn Gly
       50
<210> 19
   <211> 50
   <212> PRT
   <213> Artificial Sequence
<220>
   <223> Synthetic peptide
<400> 19
   Asn Asn Asn Asn Asn Asn Asn Ile His Asp His Asp His Asp Asn Gly
   1               5                   10                  15
   His Asp Asn Ile Asn Asn Asn Ile Asn Ile His Asp Asn Gly Asn Asn
               20                  25                  30
   His Asp His Asp Asn Gly Asn Ile His Asp Asn Ile Asn Gly Asn Ile
           35                  40                  45
   Asn Gly
       50
<210> 20
   <211> 50
   <212> PRT
   <213> Artificial Sequence
<220>
   <223> Synthetic peptide
<400> 20
   Asn Ile His Asp Asn Ile Asn Gly Asn Ile Asn Gly Asn Ile Asn Asn
   1               5                   10                  15
   Asn Asn Asn Asn His Asp Asn Ile Asn Ile Asn Asn His Asp Asn Ile
               20                  25                  30
   Asn Asn Asn Gly Asn Asn His Asp His Asp His Asp Asn Ile His Asp
           35                  40                  45
   Asn Gly
       50
<210> 21
   <211> 17
   <212> DNA
   <213> Artificial Sequence
<220>
   <223> Synthetic primer
<400> 21
   ctgaagggca cccatat 17
<210> 22
   <211> 18
   <212> DNA
   <213> Artificial Sequence
<220>
   <223> Synthetic primer
<400> 22
   cccacgttag aggcactt 18
<210> 23
   <211> 18
   <212> DNA
   <213> Artificial Sequence
<220>
   <223> Synthetic primer
<400> 23
   ctctgcagaa gttccagt 18
<210> 24
   <211> 19
   <212> DNA
   <213> Artificial Sequence
<220>
   <223> Synthetic primer
<400> 24
   ctcaggttat gtaagctat 19
<210> 25
   <211> 20
   <212> DNA
   <213> Artificial Sequence
<220>
   <223> Synthetic primer
<400> 25
   cccacgttag aggcacttgt 20
<210> 26
   <211> 20
   <212> DNA
   <213> Artificial Sequence
<220>
   <223> Synthetic primer
<400> 26
   ctgcagaagt tccagtcagt 20
<210> 27
   <211> 21
   <212> DNA
   <213> Artificial Sequence
<220>
   <223> Synthetic primer
<400> 27
   atgggaccct cagaactgcc t 21
<210> 28
   <211> 22
   <212> DNA
   <213> Artificial Sequence
<220>
   <223> Synthetic primer
<400> 28
   ctctgcagaa gttccagtca gt 22
<210> 29
   <211> 22
   <212> DNA
   <213> Artificial Sequence
<220>
   <223> Synthetic primer
<400> 29
   gcccactctg aagggcaccc at 22
<210> 30
   <211> 23
   <212> DNA
   <213> Artificial Sequence
<220>
   <223> Synthetic primer
<400> 30
   gggaccctca gaactgccta cat 23
<210> 31
   <211> 24
   <212> DNA
   <213> Artificial Sequence
<220>
   <223> Synthetic primer
<400> 31
   ggattctcag gttatgtaag ctat 24
<210> 32
   <211> 24
   <212> DNA
   <213> Artificial Sequence
<220>
   <223> Synthetic primer
<400> 32
   gtaagctatg ggaccctcag aact 24
<210> 33
   <211> 24
   <212> DNA
   <213> Artificial Sequence
<220>
   <223> Synthetic primer
<400> 33
   gcccactctg aagggcaccc atat 24
<210> 34
   <211> 25
   <212> DNA
   <213> Artificial Sequence
<220>
   <223> Synthetic primer
<400> 34
   atgggaccct cagaactgcc tacat 25
<210> 35
   <211> 25
   <212> DNA
   <213> Artificial Sequence
<220>
   <223> Synthetic primer
<400> 35
   gggaccctca gaactgccta catat 25
<210> 36
   <211> 25
   <212> DNA
   <213> Artificial Sequence
<220>
   <223> Synthetic primer
<400> 36
   acatataggg caagcagtgc ccact 25